(19) Europäisches Patentamt
European Patent Office
Office européen des brevets

(11) EP 2 164 975 B1

(12) **EUROPÄISCHE PATENTSCHRIFT**

(45) Veröffentlichungstag und Bekanntmachung des Hinweises auf die Patenterteilung:
**01.02.2012 Patentblatt 2012/05**

(21) Anmeldenummer: **08774787.9**

(22) Anmeldetag: **04.07.2008**

(51) Int Cl.:
***C12P 19/02*** *(2006.01)* ***C13K 1/06*** *(2006.01)*
***C07K 14/415*** *(2006.01)* ***A23K 1/14*** *(2006.01)*

(86) Internationale Anmeldenummer:
**PCT/EP2008/058709**

(87) Internationale Veröffentlichungsnummer:
**WO 2009/007326 (15.01.2009 Gazette 2009/03)**

(54) **Verfahren zur Herstellung einer konzentrierten wässrigen Glukoselösung aus Mais**

Process for preparing a concentrated aqueous glucose solution from corn

Procédé de préparation d'une solution de glucose aqueuse concentrée à partir de maïs

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.07.2007 EP 07111976**

(43) Veröffentlichungstag der Anmeldung:
**24.03.2010 Patentblatt 2010/12**

(60) Teilanmeldung:
**11196072.0**

(73) Patentinhaber: **BASF SE**
**67056 Ludwigshafen (DE)**

(72) Erfinder:
- **BOY, Matthias**
  **64625 Bensheim (DE)**
- **CHOI, Jong-Kyu**
  **Jungwhasan**
  **Jeoju (KR)**
- **CHUNG, Jin Won**
  **Gunsan (KR)**
- **LOHSCHEIDT, Markus**
  **69121 Heidelberg (DE)**
- **CHOI, Jong In**
  **Gunsan (KR)**
- **SEO, Jae Yeol**
  **Jeonju (KR)**
- **BRAUN, Jörg**
  **76879 Essingen (DE)**
- **KIM, Mo Se**
  **Jeonju (KR)**
- **KIM, Sung Hyun**
  **Jeonbuk (KR)**
- **KOCHNER, Arno**
  **67165 Waldsee (DE)**

(74) Vertreter: **Reitstötter - Kinzebach**
**Patentanwälte**
**Ludwigsplatz 4**
**67059 Ludwigshafen (DE)**

(56) Entgegenhaltungen:
**WO-A-2005/116228** **WO-A-2006/004748**
**GB-A- 1 153 291** **US-A- 4 361 651**
**US-A- 4 448 881**

EP 2 164 975 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Verfahren zur Herstellung einer konzeutrierten wässrigen Glukoselösung aus Mais bzw. Maiskörnern.

Glukose, insbesondere wässrige Glukoselösungen, ist eine grundlegende Kohlenstoffquelle für viele chemische und fermentative Verfahren zur Herstellung organischer Produkte. Beispielsweise bei der Fermentation werden die Glukosemoleküle von den eingesetzten Mikroorganismen verstoffwechselt und auf diese Weise in das gewünschte organische Wertprodukt umgewandelt. Die Palette derart hergestellter organischer Produkte umfasst zum Beispiel niedermolekulare flüchtige Verbindungen wie Ethanol, aliphatische Carbonsäuren, Aminosäuren, Vitamine, Karotinoide, Zuckeralkohole, Zuckersäuren und Polyole, aber auch Enzyme und organische Polymere.

Für solche allgemein bekannten Fermentationsverfahren werden in Abhängigkeit von den Verfahrensbedingungen und den herzustellenden Produkten unterschiedliche Kohlenstoffquellen genutzt. Diese reichen von reiner Saccharose über Rüben- und Zuckerrohrmelasse, Glukose aus Stärkehydrolysaten bis hin zu Glycerin.

Bei der konventionellen Herstellung von Glukose aus Stärke wird zunächst die Stärke aus einer natürlichen Stärkequelle wie Kartoffeln, Cassava, Getreide, zum Beispiel Weizen, Mais, Gerste, Roggen, Triticale oder Reis, gewonnen und anschließend hydrolysiert, in der Regel durch eine enzymatische Verflüssigung, gefolgt von einer enzymatischen Verzuckerung.

Bei der Herstellung von Glukose durch Verflüssigen und Verzuckern von Stärke geht man in der Regel von einer vorgereinigten Stärke aus, d. h. die natürlichen Stärkequellen wie Kartoffeln, Cassava und Getreide, zum Beispiel Weizen, Mais, Gerste, Roggen, Triticale oder Reis, werden in die Stärkebestandteile und die Nicht-Stärkebestandteile aufgetrennt.

Bei Getreide, insbesondere bei Mais, erfolgt die Gewinnung der vorgereinigten Stärke durch eine mehrstufige Nassvermahlung. Hierzu werden in einem ersten Schritt die Getreidekörner in Wasser gequollen. Die gequollenen Körner werden in einem zweiten Schritt unter Zusatz von Wasser zerkleinert, wonach der Keimling abgetrennt wird. Nach der Abtrennung des Keimlings erfolgt eine Feinvermahlung der verbleibenden Bestandteile, d. h. Stärke, Gluten und Kleie (Faserbestandteile). In weiteren Schritten erfolgt die Abtrennung der Kleie und des Glutens, so dass man am Ende eine wässrige Stärkesuspension erhält, die zur Herstellung von Glukose anschließend einer Verflüssigung/Verzuckerung zugeführt wird. Auf diese Weise erhält man eine sehr reine Glukose.

Die Nassvermahlung von Getreidekörnern ist jedoch vergleichsweise aufwendig. Da die Getreidekörner zunächst mit Wasser eingeweicht werden, müssen die bei der Stärkegewinnung anfallenden Neben- und Abfallprodukte wie Proteine (Gluten), Keimling- und Faserbestandteile vor der Weiterverarbeitung bzw. Entsorgung getrocknet werden, was mit einem erheblichen Energieaufwand verbunden ist. Zudem ist der apparative Aufwand hoch und entsprechende Anlagen sind daher sehr kapitalintensiv. Da andererseits Getreide und insbesondere Mais wichtige Stärkequellen sind, hat es nicht an Versuchen gefehlt, günstigere Alternativen zur Glukosegewinnung aus diesen Stärkequellen bereitzustellen.

Eine kostengünstigere Methode zur Nutzbarmachung der Stärkebestandteile von Getreide, insbesondere Mais, stellt die Trockenvermahlung der Getreidekörner dar. Hierzu werden die Getreidekörner, gegebenenfalls nach Anfeuchten mit geringen Mengen Wasser zur Verbesserung der Geschmeidigkeit des Keimlings, vermahlen und das erhaltene Mahlgut wird als Ganzes einer enzymatischen Verflüssigung/Verzuckerung zugeführt. Auf diese Weise erhält man eine wässrige Glukose, welche große Mengen an nicht-löslichen Feststoffen, die aus den Nicht-Stärkebestandteilen des Getreides resultieren, enthält, nämlich Fasern aus den Schalen, Öl aus den Keimlingen und Proteine, d. h. Gluten. Verfahren zur Herstellung von Glukose durch Trockenvermahlung von Getreide mit anschließender Verflüssigung/Verzuckerung sind bekannt und beispielsweise beschrieben in "The Alcohol Textbook - A reference for the beverage, fuel and industrial alcohol industries", Jaques et al. (Hg.), Nottingham Univ. Press 1995, ISBN 1-8977676-735, Kapitel 2, S. 7 bis 23, und in McAloon et al., "Determining the cost of producing ethanol from corn starch and lignocellulosic feedstocks", NREL/TP-580-28893, National Renewable Energy Laboratory, October 2000.

Die durch Trockenvermahlungsprozesse erhaltene Glukose wird bislang in technischem Umfang nur zur Herstellung von Bioethanol genutzt. Grund hierfür sind mehrere diesem Verfahren inhärente Nachteile: Zum einen hat der hohe Anteil an nicht-löslichen Bestandteilen in der so hergestellten wässrigen Glukose zur Folge, dass die Viskosität der wässrigen Glukoselösung bereits bei geringen Glukosekonzentrationen hoch ist und die wässrige Glukoselösung überdies strukturviskos ist. Daher bleibt die maximale Glukosekonzentration in einer so hergestellten wässrigen Glukose in der Regel auf 30 bis 33 Gew.-% beschränkt. Während hohe Glukosekonzentrationen für die fermentative Bioethanol-Herstellung nicht notwendig oder aufgrund der Toxizität des bei der Fermentation gebildeten Ethanols sogar problematisch sind, führt eine geringe Glukosekonzentration bei der Herstellung anderer Chemikalien zu einer unerwünschten Erhöhung des Volumenstroms. Zudem können sich die nicht-löslichen Bestandteile negativ auf eine Fermentation auswirken, zum Beispiel im Hinblick auf die Sauerstofftransferrate bzw. den Sauerstoffbedarf der zur Fermentation eingesetzten Mikroorganismen. Außerdem können diese Feststoffe die anschließende Aufarbeitung und Isolierung des durch Fermentation hergestellten Produkts nicht unerheblich erschweren. Diese Probleme spielen bei der Herstellung von

Bioethanol durch anaerobe Fermentation gefolgt von destillativer Abtrennung nur eine untergeordnete Rolle.

In jüngerer Zeit wurde verschiedentlich über den Einsatz einer durch ein Trockenvermahlungsverfahren hergestellten Glukose bei der fermentativen Herstellung von Feinchemikalien berichtet (siehe WO 2005/116228 und WO 2007/028804). Das in diesen Anmeldungen beschriebene Verfahren der Trockenvermahlung mit anschließender Verflüssigung/Verzuckerung erlaubt die Herstellung einer wässrigen Glukose mit einer erhöhten Zuckerkonzentration, ohne dass es einer Abtrennung der in der Stärkequelle enthaltenen nicht-löslichen Feststoffe bedarf. Der Einsatz einer so hergestellten Glukose führt jedoch in einigen Fällen zu einer Hemmung oder Verzögerung der Vermehrung der Mikroorganismen.

Wie bereits oben erläutert, enthält die durch einen Trockenvermahlungsprozess mit anschließender Verflüssigung/Verzuckerung herstellte wässrige Glukose neben den fermentierbaren Zuckerbestandteilen große Mengen an nicht-löslichen Feststoffen, die nicht fermentierbar sind. Bei Einsatz einer solchen wässrigen Glukose in einer Fermentation, sei es zur Herstellung von Bioethanol oder zur Herstellung von Feinchemikalien, werden diese Feststoffe durch das Fermentationsverfahren geschleust und erhöhen somit den Volumenstrom. Nach Abtrennung des Fermentationsproduktes verbleiben sie als Feststoff, der entsorgt werden muss, oder allenfalls als Tierfutter verwendet werden kann. Da die nicht-fermentierbaren Bestandteile jedoch ihrerseits zum Teil Wertstoffe darstellen, wurde verschiedentlich berichtet, einen Teil oder alle diese Bestandteile vor der Fermentation abzutrennen.

So beschreiben im Rahmen der Bioethanolherstellung beispielsweise die US 2005/0233030 und US 2005/0239181 sowie N. Jakel in Biofuels Journal (http://www.renessen.com/news_release/Renessen_ethanol_art.pdf) eine Trockenvermahlung von Mais, bei der man das Mahlgut in eine stärkereiche Endosperm-Fraktion und eine stärkearme Keimling/Faser-Fraktion auftrennt und im Wesentlichen nur die Endosperm-Fraktion einer Verflüssigung/Verzuckerung zuführt. Auf diese Weise lässt sich die Menge des bei der fermentativen Herstellung von Ethanol anfallenden Koppelprodukts verringern. Zudem kann die Keimling/Faser-Fraktion zur Herstellung von Pflanzenöl genutzt werden.

In der US 4,287,304 wird ein Verfahren zur Herstellung einer wässrigen Glukoselösung aus trocken vermahlenem Mais beschrieben. Bei diesem Verfahren wird zunächst bei der Trockenvermahlung in eine Keimling/Faser-Fraktion und eine stärkereiche Endosperm-Fraktion, welche neben der Stärke noch Proteinbestandteile (Gluten) und Teile des in den Körnern enthaltenen Öls enthält, aufgetrennt. Die Endosperm-Fraktion wird anschließend einer Verflüssigung zugeführt. Aus dem dabei erhaltenen wässrigen Stärke-Partialhydrolysat werden die nicht-löslichen Bestandteile, d. h. Protein und Ölbestandteile, abgetrennt. Anschließend wird die verflüssigte Stärke, also das wässrige Stärke-Partialhydrolysat einer Verzuckerung zugeführt. Eigene Untersuchungen der Anmelderin haben gezeigt, dass die Abtrennung der nicht-löslichen Bestandteile auf der Stufe der verflüssigten Stärke problematisch und aufwändig ist und mit Glukoseverlusten verbunden ist. Zudem wird auf diese Weise eine wässrige Glukose mit einer vergleichsweise geringen Glukosekonzentration erhalten.

In der CN 1173541 wird ein Verfahren zur Herstellung von Milchsäure durch Fermentation beschrieben, bei dem man eine aus Mais oder Reis gewonnene Glukose als Zuckerquelle einsetzt. Bei dem dort beschriebenen Verfahren wird Mais bzw. Reis trocken vermahlen und das erhaltene Mahlgut wird zunächst einer Verflüssigung zugeführt. Die dabei erhaltene Maische wird in eine flüssige Phase, welche die partiell hydrolysierten Stärkebestandteile enthält, und eine Feststoffphase, welche die nicht-löslichen, nicht-fermentierbaren Feststoffbestandteile des Mahlguts enthält, aufgetrennt. Die flüssige Phase wird anschließend einer Verzuckerung zugeführt. Dieses Verfahren hat ähnliche Nachteile wie das in US 4,287,304 beschriebene Verfahren. Die Abtrennung der nicht-fermentierbaren Feststoffbestandteile vor der Fermentation dient ihrer Nutzbarmachung als Futtermittel.

Die Patentschriften WO 2006/004748 A (GRAINVALUE, LLC, 12. Januar 2006) und US 4 448 881 A (MULLER, W.C. & MILLER, F.D., 15. Mai 1984) offenbaren Verfahren zur Herstellung von wässrigen Glukoselösungen aus Mais, umfassend:

a) fraktionierendes, trockenes Vermahlen von Maiskörnern, wobei man die Maiskörner in eine Maisstärke enthaltende Endosperm-Fraktion und eine ölreiche Keimling-Fraktion und gegebenenfalls eine Kleie-Fraktion trennt,

b) enzymatisches Verflüssigen und Verzuckern eines Teils der Maisstärke in einer wässrigen Suspension der Endosperm-Fraktion, wobei man eine Maisgluten enthaltende, wässrige Glukoselösung erhält,

c) Abreichern des Maisglutens und gegebenenfalls vorhandener Kleie aus der wässrigen Glukoselösung, wobei die Glukoselösungen für die Ethanolfermentation bestimmt sind.

Die Patentschrift WO 2005/116228 A (BASF AKTIENGESELLSCHAFT, 8. Dezember 2005) offenbart ein Verfahren zur Herstellung einer konzentrierten wässrigen Glukoselösung mit einem Monosaccharidgehalt von mehr als 20 Gew.-% aus Mais, umfassend:

a) trockenes Vermahlen von Maiskörnern

b) enzymatisches Verflüssigen und Verzuckern der Maisstärke in einer wässrigen Suspension, wobei man eine Maisgluten enthaltende, wässrige Glukoselösung erhält, wobei die Glukoselösung für die fermentative Herstellung von Feinchemikalien bestimmt ist.

Der vorliegenden Erfindung liegt somit die Aufgabe zugrunde, ein Verfahren zur Herstellung einer wässrigen Glukose lösung aus Mais bereitzustellen, welches die Nachteile des Standes der Technik nicht aufweist. Insbesondere sollte die dabei erhaltene Glukose nicht nur zur Herstellung von Bioethanol, sondern auch zur Herstellung davon verschiedener Feinchemikalien geeignet sein.

Diese Aufgabe wird durch das im Folgenden beschriebene Verfahren gelöst. Die vorliegende Erfindung betrifft somit ein Verfahren zur Herstellung einer wässrigen Glukoselösung aus Mais, umfassend

a) fraktionierendes, trockenes Vermahlen von Maiskörnern, wobei man die Maiskörner in eine Maisstärke enthaltende Endosperm-Fraktion und eine ölreiche Keimling-Fraktion und gegebenenfalls eine Kleie-Fraktion auftrennt;

b) Enzymatisches Verflüssigen und Verzuckern der Maisstärke in einer wässrigen Suspension der Endosperm-Fraktion, wobei man eine Maisgluten enthaltende, wässrige Glukoselösung erhält; und

c) Abreichern des Maisglutens und gegebenenfalls vorhandener Kleie aus der wässrigen Glukoselösung.

wobei man in Schritt b) eine wässrige Suspension des in Schritt a) erhaltenen Maismehls einsetzt, welches die Endosperm-Fraktion und gegebenenfalls die Kleiefraktion enthält, wobei die Menge des Maismehls so gewählt ist, dass die wässrige Suspension 30 bis 45 Gew.-%, bezogen auf das Gesamtgewicht der Suspension, Stärke enthält.

Das erfindungsgemäße Verfahren ist mit einer Reihe von Vorteilen verbunden. Zum einen ist der apparative Aufwand, aber auch der Energieaufwand zur Herstellung einer wässrigen Glukoselösung nach dem erfindungsgemäßen Verfahren sehr viel geringer als nach dem konventionellen Verfahren der Nassvermahlung. Zudem ist die nach dem erfindungsgemäßen Verfahren erhältliche Glukose in besonderer Weise als Kohlenstoffquelle für fermentative Verfahren zur Herstellung von Chemikalien geeignet. Ihre Eignung ist nicht nur deutlich besser als die einer Glukoselösung, die durch Verflüssigung/Verzuckerung eines durch Trockenvermahlung hergestellten Maismehls erhältlich ist, sondern führt im Vergleich mit reiner Glukose bzw. einer in einem Nassvermahlungsverfahren erhaltenen Glukose bei einer Reihe von Mikroorganismen zu einem besseren Wachstum der zur Fermentation eingesetzten Mikroorganismen und/oder zu höheren Ausbeuten, bezogen auf die eingesetzte Glukose. Zudem können durch das erfindungsgemäße Verfahren Glukoselösungen mit hoher Glukosekonzentration hergestellt werden. Die Viskositätseigenschaften einer erfindungsgemäß erhältlichen Glukose sind denjenigen einer Glukose, die durch Verflüssigung/Verzuckerung eines durch konventionelle Trockenvermahlung ohne Fraktionierung hergestellten Maismehls hergestellt wurde, deutlich überlegen.

Unter den Begriffen "Kleie" oder "Schale" ist die harte äußere Hülle des Maiskorns zu verstehen, das Perikarp (in der Regel < 2 Gew.-% des Maiskorns). Bei "Kleiebestandteilen" bzw. "Schalenbestandteilen" handelt es sich um Bruchstücke bzw. Teile davon. Die "Kleie-Fraktion" bzw. "Schalenfraktion" besteht zu wesentlichen Anteilen aus der Kleie bzw. der Schale, kann aber auch andere Bestandteile des Maiskorns, insbesondere Teile des Endosperms enthalten.

Unter dem Begriff "Keimling" oder "Keim" ist der Embryo des Maiskorns zu verstehen (in der Regel 8 bis 10 Gew.-% des Maiskorns). Bei "Keimlingbestandteilen" bzw. "Keimbestandteilen" handelt es sich um Bruchstücke bzw. Teile davon. Die "Keimling-Fraktion" bzw. "Keim-Fraktion" besteht zu wesentlichen Anteilen aus dem Keimling bzw. dem Keim, kann aber auch andere Bestandteile des Maiskorns, z. B. Teile des Endosperms oder der Kleie, enthalten.

Unter dem Begriff "Endosperm" ist der primär stärkehaltige Teil des Maiskorns zu verstehen (in der Regel 80 bis 85 Gew.-% des Maiskorns). Die "Endosperm-Fraktion" besteht zu wesentlichen Teilen aus dem Endosperm, kann aber auch andere Bestandteile, z. B. Teile des Keimlings oder der Kleie, enthalten.

Die nach dem erfindungsgemäßen Verfahren hergestellten Glukoselösungen weisen eine charakteristische Zusammensetzung auf, die Glukoselösungen, welche auf anderem Wege hergestellt wurden, nicht aufweisen.

Außerdem zeichnet sich der in Schritt c) des erfindungsgemäßen Verfahrens anfallende Proteinbestandteil Maisgluten durch eine besondere Qualität aus, die ihn von den in anderen Verfahren der Maisaufbereitung anfallenden Glutenbestandteilen unterscheidet und ihn für viele Anwendungen geeignet macht.

<u>Schritt a):</u>

In Schritt a) des erfindungsgemäßen Verfahrens werden Maiskörner einer fraktionierenden, trockenen Vermahlung unterworfen. Die fraktionierende Vermahlung dient der Zerkleinerung der Maiskörner und der Auftrennung des Maiskorns in seine Bestandteile, nämlich Keimling, Endosperm und Schalenbestandteile (im Folgenden auch als Kleiebestandteile bezeichnet).

Erfindungsgemäß wird auf dieser Stufe die Hauptmenge, d. h. wenigstens 70 Gew.-%, insbesondere wenigstens 80 Gew.-% der in den Maiskörnern enthaltenen Keimlinge beziehungsweise Keimlingbestandteile als ölreiche Keimling-Fraktion von den übrigen Bestandteilen des Maiskorns, d. h. Endosperm und Schalenbestandteile, abgetrennt. In der Regel erfolgt bei der fraktionierenden trockenen Vermahlung auch eine Auftrennung in eine Endosperm-Fraktion, welche im Wesentlichen die Stärke- und Proteinbestandteile der Maiskörner enthält, sowie in eine Kleie-Fraktion, welche im Wesentlichen, d. h. wenigstens 60 Gew.-%, insbesondere wenigstens 80 Gew.-%, der in den Maiskörnern enthaltenen Schalenbestandteile enthält.

Zur Vermeidung von Stärkeverlusten kann jedoch ein Teil oder die Gesamtmenge, z. B. 10 bis 100 Gew.-%, der Kleie-Fraktion zusammen mit der Endosperm-Fraktion der Verflüssigung und Verzuckerung in Schritt b) zugeführt werden. Alternativ ist es möglich, die Kleie-Fraktion einer anderen Verwendung zuzuführen und der Verflüssigung/ Verzuckerung in Schritt b) nur die Endosperm-Fraktion und gegebenenfalls geringe Mengen an Kleie, d. h. weniger als 20 Gew.-%, bezogen auf die in den Maiskörnern enthaltenen Kleiebestandteile, zuzuführen.

Zur fraktionierenden, trockenen Vermahlung der Maiskörner können die Maiskörner, so wie sie angeliefert werden, eingesetzt werden. Vorzugsweise setzt man jedoch gereinigte Maiskörner ein. Bei der Reinigung werden sowohl grobteilige Verunreinigungen, beispielsweise Holzstücke, Pflanzenbestandteile wie Stängel oder Blätter, Steine, Glasscherben, Schrauben etc., als auch feinteilige Verunreinigung wie gebrochene Maiskörner, Fremdsamen, kleine Steine, Sand von den Maiskörnern abgetrennt. Die Abtrennung kann in an sich bekannter Weise, z. B. durch Sieben, Sichten oder Kombinationen dieser Maßnahmen erfolgen. In der Regel wird man hierbei so vorgehen, dass man zunächst grobe Partikel von den Maiskörnern und den feinteiligen Verunreinigungen abtrennt und dann eine Abtrennung der feinteiligen Partikel von den Maiskörnern vornimmt. Als grobteilige Partikel werden solche angesehen, deren Partikelgröße zumindest oberhalb einer Grenze von 15 bis 20 mm liegt. Als feinteilige Partikel werden solche Partikel angesehen, deren maximale Partikelgröße einen Wert von 5 bis 6,5 mm nicht überschreitet.

Da die feinteiligen Verunreinigungen neben Sand und Staubbestandteilen auch gebrochene Maiskörner enthalten, ist es von Vorteil, wenn man die feinteiligen Verunreinigungen erneut einer Fraktionierung unterwirft. Hierzu trennt man die feinteiligen Verunreinigungen in eine erste Fraktion mit einer Maximalpartikelgröße von 3,5 bis 4,5 mm, die im Wesentlichen Sand und sonstiges staubiges Material enthält, und eine etwas grobteiligere Fraktion mit Partikelgrößen von mindestens 3,5 bis 4,5 mm, welche im Wesentlichen kleine oder gebrochene Maiskörner enthält, auf. Die letztere Fraktion kann dem gereinigten Mais zur Reduzierung von Stärkeverlusten wieder zugeführt werden. Die erste Fraktion kann der Kleie-Fraktion zugegeben werden, die sich bei der Fraktionierung ergibt.

Der so gereinigte Mais wird anschließend der fraktionierenden, trockenen Vermahlung unterworfen. Die fraktionierende Vermahlung erfolgt in an sich bekannter Weise. In der Regel gliedert sich die trockene Vermahlung in eine erste Mahlstufe, bei der der Keimling entfernt wird bzw. eine Auftrennung in eine Endosperm-Fraktion, eine Keimling-Fraktion und eine Kleie-Fraktion vorgenommen wird, und eine zweite Mahlstufe, bei der die Endosperm-Fraktion auf die gewünschte Partikelgröße vermahlen wird. Es versteht sich für den Fachmann, dass die Auftrennung in der Regel nicht vollständig ist sondern nur bis zur gewünschten Reinheit der Fraktionen durchgeführt wird, d. h. die Endosperm-Fraktion enthält nach der Abtrennung des Keimlings in der Regel noch bis zu 30 Gew.-%, vorzugsweise nicht mehr als 20 Gew.-%, der im Maiskorn enthaltenen Keimlingbestandteile und nach Abtrennung der Kleiebestandteile bis zu 40 Gew.-%, vorzugsweise nicht mehr als 20 Gew.-%, der im Maiskorn enthaltenden Schalenbestandteile.

In der ersten Stufe, die häufig auch als Maisentkeimung bezeichnet wird, werden die Maiskörner zerkleinert, z. B. durch Walzenstühle, wie sie beispielsweise von den Firmen Bühler AG oder Ocrim spa erhältlich sind, spezielle Degerminatoren, beispielsweise Vorrichtungen mit einem oder mehreren Walzenrotoren, die von einem strukturierten Siebmantel umgeben sind, oder auch durch Kombinationen dieser Apparate. Das Verfahren kann einstufig durchgeführt werden und wird vorzugsweise in mehreren Mahlschritten durchgeführt. Im Anschluss an einen Mahlgang wird dann das Mahlgut in an sich bekannter Weise in eine Endosperm-Fraktion, eine Keimling-Fraktion und eine Kleie-Fraktion aufgetrennt. Hierbei wird man in der Regel so vorgehen, dass man zunächst eine Auftrennung in eine Endosperm-Fraktion und in eine Kleie- und Keimling-Fraktion vornimmt und die abgetrennte Kleie- und Keimling-Fraktion in einem zweiten Schritt in ihre Bestandteile auftrennt. Da die Endosperm-Bestandteile des Mahlguts in der Regel kleinere Partikelgrößen aufweisen als die Partikel der Keimling- und Kleie-Fraktion des Mahlguts, kann die erste Auftrennung in einfacher Weise durch ein Sieb-Verfahren erfolgen. Die Auftrennung der Keimling- und Kleie-Fraktion des Mahlguts kann beispielsweise durch Sichten erfolgen. Selbstverständlich können die einzelnen Trennschritte Kombinationen dieser Maßnahmen umfassen.

In einer mehrstufigen Maisentkeimung wird die Endosperm-Fraktion einer vorangehenden Stufe in einer nachgeschalteten Stufe weiter zerkleinert und analog der oben beschriebenen Vorgehensweise aufgearbeitet. Typisch sind 2 bis 4 stufige Verfahren. Die Mehrstufigkeit führt zu höheren Reinheiten der einzelnen Fraktionen und zu einer höheren Stärkeausbeute der Endosperm-Fraktion.

Bei der Verwendung von Degerminatoren können in der ersten Stufe besonders kleine Partikel erzeugt werden, die durch Sieben oder Sichten nicht mehr in die drei gewünschten Fraktionen Endosperm, Keimling und Kleie getrennt

werden können. In der Regel werden diese Partikel der Kleie-Fraktion zugegeben, wobei es zur Erzielung einer hohen Stärkeausbeute vorteilhaft sein kann, diese Partikel vor oder während der Feinvermahlung der Endosperm-Fraktion zuzugeben.

Für die Maisentkeimung hat es sich als vorteilhaft erwiesen, wenn der Mais eine gewisse Feuchte aufweist, die im Bereich von 5 bis 30 Gew.-% und insbesondere im Bereich von 12 bis 20 Gew.-% liegt. Dementsprechend wird man Mais, der nicht die gewünschte Feuchtigkeit aufweist, vor oder während der Maisentkeimung mit einer geringen Wassermenge versetzen. Nach der Zugabe von Wasser wird der Mais vor der Weiterverarbeitung vorzugsweise über einen Zeitraum von 0,5 bis 24 h gelagert, wodurch die auf der Oberfläche anhaftende Feuchtigkeit in das Innere des Maiskorns, speziell zum Maiskeim, diffundieren kann. In der Regel führt man daher das Vermahlen in Schritt a) in Gegenwart von 5 bis 30 Gew.-% Wasser, bezogen auf die Masse der eingesetzten Maiskörner, durch. Vorzugsweise beträgt die Menge an Wasser 10 bis 25 Gew.-% und insbesondere 12 bis 20 Gew.-%. Das Wasser wird vorzugsweise vor der Maisentkeimung zugesetzt, kann aber auch während der Maisentkeimung zugesetzt werden. Bei einer mehrstufigen Entkeimung kann zwischen den jeweiligen Entkeimungsschritten noch einmal der Wassergehalt eingestellt werden. Das Wasser kann auch gegebenenfalls dampfförmig zugegeben werden. Durch Analyse der eingesetzten Maiskörner, aber auch des auf der jeweiligen Stufe enthaltenen Mahlguts kann der Fachmann den Wassergehalt leicht bestimmen und erforderliche Mengen an zusätzlichem Wasser leicht ermitteln.

In der Regel erfolgt dann wenigstens eine weitere Vermahlung der Endosperm-Fraktion, die ebenfalls aus einem oder mehreren Mahlschritten bestehen kann. Hierbei wird die Endosperm-Fraktion auf die für die Verflüssigung/Verzuckerung günstigste Teilchengröße eingestellt. Dieser Schritt wird häufig auch als Feinvermahlung bezeichnet. Bei der Feinvermahlung wird die Endosperm-Fraktion in der Regel auf einen mittleren Teilchendurchmesser im Bereich von 0,05 bis 1,5 mm und vorzugsweise auf eine Teilchengröße im Bereich von 0,1 bis 1 mm und speziell im Bereich von 0,25 bis 0,8 mm vermahlen. Der mittlere Teilchendurchmesser ist massenbezogen und wird in einer dem Fachmann bekannten Weise vorzugsweise mittels Siebanalyse ermittelt. Insbesondere hat es sich als vorteilhaft erwiesen, wenn wenigstens 80 Gew.-%, insbesondere wenigstens 90 Gew.-% und speziell wenigstens 95 Gew.-% der Partikel einen Durchmesser von nicht mehr als 0,4 mm aufweisen. Bei einer mehrstufigen Durchführung der Feinvermahlung erfolgt vorzugsweise nach jedem Mahlvorgang eine Auftrennung in Partikel, deren Größe oberhalb der gewünschten Maximalgröße liegt, und Partikel, deren Größe die gewünschte Obergrenze nicht überschreitet. Nur die zu großen Partikel werden dann einem weiteren Mahlvorgang zugeführt.

Wie bereits oben erläutert, kann zur Vermeidung von Stärkeverlusten ein Teil oder die gesamte Menge der Kleie-Fraktion in die Endosperm-Fraktion zurückgeführt werden. Die Rückführung erfolgt vorzugweise vor oder während der Feinvermahlung. Vorzugsweise erfolgt jedoch keine Rückführung der Kleie-Fraktion in die Endosperm-Fraktion.

Die so aufgetrennten Fraktionen weisen typischerweise die folgenden Zusammensetzungen auf:

Der Kleiebestandteil weist typischerweise die folgenden Bestandteile in den folgenden Mengen (bezogen auf die Gesamttrockenmasse) auf:

| | |
|---|---|
| Rohprotein: | 1 bis 20 Gew.-%, bevorzugt 5 bis 15 Gew.-% |
| Stärke: | 1 bis 30 Gew.-%, bevorzugt 5 bis 20 Gew.-% |
| Rohfasern: | 1 bis 40 Gew.-%, bevorzugt 5 bis 20 Gew.-% |
| Rohfett: | 0 bis 20 Gew.-%, bevorzugt 0,5 bis 15 Gew.-% |
| Rohasche: | 0 bis 10 Gew.-%, bevorzugt 0,1 bis 5 Gew.-% |

Die Feuchte der Kleie liegt typischer Weise zwischen 8 und 20 Gew.-%, bevorzugt zwischen 10 und 17 Gew.-%.

Die Maiskleie ist ein spelzenähnliches Material, bestehend aus vorwiegend flachen Partikeln. Der mittlere Durchmesser dieser flachen Partikel liegt zwischen 0,5 mm und 8 mm, bevorzugt zwischen 0,6 mm und 5 mm. Die mittlere Höhe der flachen Partikel liegt zwischen 0,01 mm und 4 mm, bevorzugt zwischen 0,05 mm und 2 mm.

Die Keimling-Fraktion weist typischerweise die folgenden Bestandteile in den folgenden Mengen (bezogen auf die Gesamttrockenmasse) auf:

| | |
|---|---|
| Rohprotein: | 1 bis 30 Gew.-%, bevorzugt 5 bis 20 Gew.-% |
| Stärke: | 1 bis 60 Gew.-%, bevorzugt 5 bis 50 Gew.-% |
| Rohfasem: | 1 bis 20 Gew.-%, bevorzugt 2 bis 12 Gew.-% |
| Rohfett: | 8 bis 40 Gew.-%, bevorzugt 10 bis 35 Gew.-% |
| Rohasche: | 0 bis 15 Gew.-%, bevorzugt 0,1 bis 10 Gew.-% |

Die Feuchte des Maiskeimlings liegt typischer Weise zwischen 8 und 20 Gew.-%, bevorzugt zwischen 10 und 15 Gew.-%.

Der Maiskeimling hat eine Art tropfenförmige Form. Der mittlere Durchmesser dieser Partikel liegt zwischen 0,1 mm und 5 mm, bevorzugt zwischen 1 mm und 4 mm. Die mittlere Höhe der Partikel liegt zwischen 2 mm und 10 mm, bevorzugt zwischen 3 mm und 8 mm.

Die Endosperm-Fraktion weist typischerweise die folgenden Bestandteile in den folgenden Mengen (bezogen auf die Gesamttrockenmasse) auf:

| | |
|---|---|
| Rohprotein: | 1 bis 30 Gew.-%, bevorzugt 5 bis 15 Gew.-% |
| Stärke: | 40 bis 95 Gew.-%, bevorzugt 60 bis 90 Gew.-% |
| Rohfasern: | 0 bis 20 Gew.-%, bevorzugt 0,2 bis 12 Gew.-% |
| Rohfett: | 0,2 bis 10 Gew.-%, bevorzugt 0,5 bis 5 Gew.-% |
| Rohasche: | 0 bis 15 Gew.-%, bevorzugt 0,1 bis 3 Gew.-% |

Die Feuchte des Endosperms liegt typischer Weise zwischen 8 und 20 Gew.-%, bevorzugt zwischen 8 und 15 Gew.-%.

Für den Keimling, die Kleie und die Endosperm-Fraktion sind nur die für Futtermittel relevanten Bestandteile angegeben, wie sie sich aus einer hierfür typischen Analytik ergeben. Dabei umfasst der für Rohprotein angegebene Wert den Gesamtstickstoff nach Kjeldahl multipliziert mit dem Faktor 6,25, also neben Proteinen z. B. auch weitere freie Aminosäuren, Nucleinsäuren wie auch anorganischen Stickstoff. Der für Rohfasern angegebene Wert umfasst als Hauptbestandteil die Cellulose und Hemicellulosen, es werden aber auch inkrustierende Substanzen wie Lignin erfasst. Von dem für Rohfett werden alle Stoffe erfasst, die sich wie z. B. Triglyceride, freie Fettsäuren und Phospolipide in Fettlösungsmittein wie z. B. Petrolether oder Hexan lösen. Die Rohasche umfasst alle nicht-organischen Bestandteile, die nach Erhitzen auf 550 °C über einen längeren Zeitraum verbleiben. Im Wesentlichen handelt es sich dabei um Mineralstoffe in Form von Oxiden und Salzen. Neben der separat analysierten Stärke werden Nicht-Stärke-Polysaccharide wie z. B. Pentosane von der gewählten Analytik nicht oder nur ungenau erfasst.

Die hier verwendeten Bezeichnungen Rohprotein, Rohfaserbestandteile, Rohfett und Rohasche sind dem Fachmann geläufig und beispielsweise in Naumann, C., Bassler, R., 1976. VDLUFA-Methodenbuch, Band 3, Die chemische Untersuchung von Futtermitteln (Loseblattsammlung mit Ergänzungen von 1983, 1988, 1993, 1997 und 2004), VDLUFA-Verlag, Darmstadt, Germany [Zusammenstellung aller in Deutschland zur Beurteilung von Futtermitteln relevanten Parameter/Methoden], definiert.

Schritt b)

Das so erhaltene Maismehl, welches im Wesentlichen die Endosperm-Fraktion und gegebenenfalls die Kleie-Fraktion enthält, wird dann einer enzymatischen Verflüssigung und Verzuckerung unterworfen, wobei die Stärke-Bestandteile der Endosperm-Fraktion zu Glukose hydrolysiert werden. In einem ersten Schritt b.1) führt man eine Verflüssigung des in Schritt a) erhaltenen Maismehls durch, wobei die Stärkebestandteile des Maismehls typischerweise zu Zuckerketten mit 4 bis 20 und insbesondere 8 bis 12 Glukoseeinheiten aufgeschlossen bzw. hydrolysiert wird. Dieser Schritt wird im Folgenden auch als Verflüssigung bezeichnet.

Die Verflüssigung kann in üblicher Weise durch Zusatz von Enzymen erfolgen. Verfahren hierzu sind aus dem eingangs zitierten Stand der Technik bekannt, z. B. aus dem eingangs zitierten "The Alcohol Textbook - A reference for the beverage, fuel and industrial alcohol industries", Kapitel 2, S. 7 bis 23.

Hierzu wird man zunächst das in Schritt a) erhaltene Maismehl mit einer wässrigen Flüssigkeit, z. B. Frischwasser, rückgeführtem Prozesswasser, z. B. aus einer nachfolgenden Fermentation oder Eindampfung, oder mit einer Mischung dieser Flüssigkeiten vermischen, wobei man eine wässrige Suspension erhält. Dieser Vorgang wird häufig auch als Anmaischen bezeichnet.

Die Menge an Mehl wird so gewählt, dass die Suspension 30 bis 45 Gew.-% und bevorzugt 32 bis 38 Gew.-% Stärke, bezogen auf das Gesamtgewicht der Suspension (Maische), enthält. Da 1 kg Stärke in einer Verflüssigung/ Verzuckerung in der Regel 1,0 bis 1,1 kg Mono-, Di- und Oligosaccharide liefert, liegt dementsprechend die Gesamtkonzentration an Mono- Di-, und/oder Oligosacchariden in der erhaltenen Glukoselösung nach der Verzuckerung im Bereich von bis 495 g/kg und insbesondere im Bereich von 320 bis 410 g/kg. Hierbei macht Glukose in der Regel wenigstens 80 Gew.-%, insbesondere wenigstens 90 Gew.-%, bezogen auf die Gesamtmenge an Mono-, Di- und/oder Oligosacchariden, aus.

Die Temperatur des eingesetzten Wassers wird in der Regel so gewählt, dass die Suspension eine Temperatur im Bereich von 30 bis 60 °C, bevorzugt 40 bis 58 °C und ganz besonders bevorzugt 50 bis 55 °C aufweist. Eine Temperatur von 60 °C sollte vorzugsweise nicht überschritten werden, um ein ungewünschtes Verkleistern der Stärke zu verhindern.

Zur Verflüssigung des Stärkeanteils im Maismehl können grundsätzlich alle Stärke verflüssigenden Enzyme, insbesondere α-Amylasen (Enzymklasse EC 3.2.1.1), eingesetzt werden, beispielsweise α-Amylasen, die aus Bacillus

lichenformis oder Bacillus staerothermophilus erhältlich sind, unter anderem solche, die zum Verflüssigen von durch Dry-Milling-Verfahren gewonnenen Materialien im Rahmen der Herstellung von Bioethanol verwendet werden. Die zum Verflüssigen geeigneten $\alpha$-Amylasen sind auch kommerziell erhältlich, beispielsweise von Novozymes unter der Bezeichnung Termamyl 120 L, Typ L; oder von Genencor unter der Bezeichnung Spezyme. Es kann auch eine Kombination verschiedener $\alpha$-Amylasen zur Verflüssigung eingesetzt werden. Die Konzentration des Enzyms in der Maische, bezogen auf den Stärkegehalt, beträgt in der Regel 0,01 bis 0,2 Gew.-%, besonders bevorzugt 0,02 bis 0,1 Gew.-% und ganz besonders bevorzugt 0,04 bis 0,08 Gew.-%.

Vorteilhafterweise wird man die Mengen an Stärke verflüssigendem Enzym und Maismehl so wählen, dass die Viskosität während des Gelierungsvorgangs ausreichend reduziert ist, um eine effektive Durchmischung der Suspension, z. B. mittels Rühren, zu ermöglichen. Bevorzugt beträgt die Viskosität der Reaktionsmischung während des Gelierens maximal 20 Pas, besonders bevorzugt maximal 15 Pas und ganz besonders bevorzugt maximal 8 Pas. Die Messung der Viskosität erfolgt in der Regel mit einem Haake Viskosimeter, Type Roto Visko RV20 mit Messsystem M5 und Messeinrichtung MVDIN bei einer Temperatur von 50 °C und einer Schergeschwindigkeit von 200 $s^{-1}$.

Häufig führt man die Verflüssigung in Gegenwart wenigstens eines Calciumsalzes durch. Die Calciumkonzentration in der Maische wird dann durch Zugabe eines Calciumsalzes auf in der Regel 10 bis 200 ppm, bevorzugt 15 bis 100 ppm und ganz besonders bevorzugt auf 20 bis 60 ppm eingestellt. Die Anwesenheit von Calciumionen ist jedoch nicht zwingend, und es sind eine Reihe verflüssigender Enzyme für die Verflüssigung und Verzuckerung bekannt, die auch in Abwesenheit von Calcium gute Umsätze und Ausbeuten liefern, so dass in diesen Fällen auf den Zusatz von Calciumsalzen verzichtet werden kann.

Für eine optimale Wirkung des Stärke verflüssigenden Enzyms wird die Verflüssigung vorzugsweise zumindest zeitweise im pH-Optimum des verflüssigenden Enzyms, häufig bei einem pH-Wert im schwach sauren Bereich, in der Regel im Bereich von 4,0 bis 7,0, vorzugsweise im Bereich von 5,0 bis 6,5, besonders bevorzugt im Bereich von 5,3 bis 6,0 durchgeführt. Üblicherweise wird vor oder zu Beginn der Verflüssigung eine pH-Einstellung vorgenommen; dieser pH-Wert wird in der Regel während der Verflüssigung kontrolliert und gegebenenfalls nachgestellt. Die Einstellung des pH-Werts erfolgt vorzugsweise mit verdünnten Mineralsäuren wie HCl, $HNO_3$, $H_2SO_4$ oder $H_3PO_4$, mit organischen Säuren wie Essigsäure, mit Alkalimetallhydroxide wie NaOH oder KOH, oder Erdalkalimetallhydroxide wie Magnesiumhydroxid oder Calciumhydroxid. Vorzugsweise erfolgt die pH-Stellung mit Calciumhydroxid und/oder Schwefelsäure.

Die Herstellung der Maismehlsuspension kann diskontinuierlich oder kontinuierlich erfolgen, wobei man etwaige Mittel zur Einstellung des pH-Wertes wie Calciumhydroxid und/oder Schwefelsäure und das verflüssigende Enzym vorher mit dem Wasser vermischt oder auch einzeln zur Maismehl/Wasser-Mischung gegeben werden können. Die Reihenfolge der Zugabe ist dabei beliebig. Bei der diskontinuierlichen Herstellung der Maismehlsuspension können alle Arten durchmischter Reaktoren eingesetzt werden. Bei der kontinuierlichen Herstellung werden in der Regel langsam oder schnelllaufende kontinuierliche Mischer eingesetzt.

Die so angesetzte Suspension (Maische) wird dann vorzugsweise auf eine Temperatur oberhalb der Gelierungstemperatur der verwendeten Stärke erhitzt. In der Regel wird eine Temperatur im Bereich von 80 bis 120 °C, bevorzugt von 90 bis 115 °C und besonders bevorzugt im Bereich von 95 bis 110 °C gewählt, wobei die Temperatur vorzugsweise mindestens 5 K, insbesondere 10 K und besonders bevorzugt mindestens 20 K, z. B. 10 bis 80 K, insbesondere 20 bis 60 K, oberhalb der Gelierungstemperatur (Verkleisterungstemperatur) liegt. Die Verflüssigung kann auch unterhalb der Verkleisterungstemperatur durchgeführt werden, z. B. unter Verwendung von den in WO 2004/113551 beschriebenen Enzyme bzw. Enzymkombinationen.

In einer bevorzugten Ausführungsform zur Verflüssigung des Stärkeanteils wird die Maische zunächst durch Einbringen von Direktdampf auf eine Temperatur oberhalb der Verkleisterungstemperatur der Stärke erhitzen. Typischerweise wird man auf eine Temperatur erhitzen, die wenigstens 10 K und insbesondere wenigstens 20 K, z. B. 10 bis 80 K, insbesondere 20 bis 60 K oberhalb der jeweiligen Verkleisterungstemperatur liegt. Vorzugsweise erhitzt man die Suspension auf Temperaturen im Bereich von 80 bis 120 °C, insbesondere im Bereich von 90 bis 115 °C und speziell im Bereich von 95 bis 110 °C.

Bei dem zum Erhitzen eingesetzten Direktdampf handelt es sich typischerweise um überhitzen Wasserdampf, der eine Temperatur von wenigstens 105 °C, insbesondere wenigstens 110 °C, z. B. im Bereich von 110 bis 210 °C, aufweist. Der Einsatz von Sattdampf ist aber ebenfalls möglich. Vorzugsweise wird der Dampf mit Überdruck in die Suspension eingebracht. Dementsprechend weist der Dampf vorzugsweise einen Druck von wenigstens 1,5 bar, z. B. 1,5 bis 16 bar, insbesondere 2 bis 12 bar auf.

Das Einbringen von Direktdampf in die Suspension erfolgt in der Regel so, dass man den Dampf mit Überdruck, vorzugsweise einem Überdruck von 1 bis 10 oder 11 bar, insbesondere 1,5 bis 5 bar und vorzugsweise mit hoher Geschwindigkeit in die Suspension einträgt. Durch das Eintragen des Dampfes erhitzt sich die Suspension augenblicklich auf Temperaturen oberhalb 90 °C, also auf Temperaturen oberhalb der Verkleisterungstemperatur.

Vorzugsweise erfolgt das Erhitzen mit Direktdampf in einer kontinuierlich arbeitenden Vorrichtung, in welche man die Maische kontinuierlich mit einem bestimmten Förderdruck, der sich aus der Viskosität der Suspension, der Fördergeschwindigkeit und der Geometrie der Vorrichtung ergibt, einspeist und in die man im Bereich des Einspeisens

der Suspension den heißen Dampf mit Überdruck, bezogen auf den Förderdruck, über eine regulierbare Düse einspeist. Durch das Einspeisen des Dampfs mit Überdruck wird die Suspension nicht nur erhitzt, sondern es wird auch mechanische Energie in das System eingetragen, welche eine weitere Durchmischung der Maismehlpartikel fördert, einen besonders gleichmäßigen Energieeintrag bewirkt und somit eine besonders gleichmäßige Verkleisterung der granulären Stärkepartikel im Maismehl zur Folge hat. Typischerweise haben diese Vorrichtungen eine röhrenförmige Geometrie. Vorzugsweise erfolgt das Eintragen des Dampfes in Richtung der Längsachse der röhrenförmigen Vorrichtung. Die Suspension wird in der Regel in einem flachen Winkel zum Dampfstrom, der in der Regel 50° nicht überschreitet, zugeführt. Die regulierbare Düse weist typischerweise eine konische Geometrie auf, die sich in Fließrichtung des Dampfs verjüngt. In dieser Düse ist eine Nadel oder ein auf einer in Längsrichtung verschiebbaren Stange angeordneter Kegel angeordnet. Die Nadel bzw. der Kegel bildet mit dem Konus der Düse einen Spalt. Durch Verschieben der Nadel bzw. der Stange in Längsrichtung lässt sich die Größe des Spalts und damit die Querschnittsfläche der Düsenöffnung in einfacher Weise einstellen, wodurch man in einfacher Weise die Geschwindigkeit des Dampfeintrags regulieren kann.

Typischerweise weisen diese Vorrichtungen außerdem ein Vermischungsrohr auf, in welches die Suspension nach dem Dampfeintrag transportiert und aus der Vorrichtung ausgetragen wird. Dieses Vermischungsrohr ist üblicherweise in Richtung des Dampfeintrags angeordnet. Das Vermischungsrohr bildet typischerweise mit der Düse einen Spalt, durch den die Suspension transportiert wird. Durch diesen Spalt wirken beim Transport zusätzliche Scherkräfte auf die Suspension und erhöhen somit den mechanischen Energieeintrag in die Suspension. Das Vermischungsrohr kann in Längsrichtung verschiebbar angeordnet sein. Durch Verschieben des Vermischungsrohrs lässt sich in einfacher Weise die Größe der Spaltöffnung und damit das Druckgefälle in der Vorrichtung einstellen.

Derartige Vorrichtungen sind unter der Bezeichnung Jet-Kocher aus dem Stand der Technik bekannt, beispielsweise die in "The Alcohol Textbook", Kapitel 2, loc. cit., Figur 13 dargestellte Vorrichtung und kommerziell erhältlich, beispielsweise unter der Bezeichnung HYDROHEATER® oder JetCooker® der Fa. Hydro Thermal Corp. Waukesha WI, USA.

Die mit Direktdampf erhitzte Maische wird in der Regel im Anschluss daran in eine Nachreaktionszone überführt, um das Gelieren der Stärkebestandteile fortzusetzen. Gleichzeitig beginnt das verflüssigende Enzym die Stärke zu hydrolisieren. In der Nachreaktionszone herrscht typischerweise Überdruck, typischerweise ein Absolutdruck im Bereich von 2 bis 8 bar. Die Temperaturen in der Nachreaktionszone liegen typischerweise im Bereich von 80 bis 120 °C, insbesondere im Bereich von 90 bis 115 °C. Die Verweilzeit in dieser Nachreaktionszone kann in Abhängigkeit von der Temperatur der Suspension im Bereich von 1 bis 30 min, häufig 2 bis 20 min, und insbesondere 5 bis 10 min, betragen. Die Nachreaktionszonen weisen typischerweise eine röhrenförmige oder säulenförmige Geometrie auf. In einer Ausführungsform weist die Nachreaktionszone die Geometrie einer senkrecht angeordneten Säule auf. Die Suspension wird hierbei nach Verlassen der Vorrichtung zur Dampfbehandlung im oberen Bereich der Säule aufgebracht und im unteren Bereich entnommen. In einer anderen Ausführungsform der Erfindung weist die Nachreaktionszone eine röhrenförmige Geometrie auf.

Nach Verlassen der Nachreaktionszone wird die Suspension in der Regel abgekühlt und man führt dann eine zweite Verflüssigung durch. Diese Abkühlung kann durch Entspannung der unter Druck stehenden Lösung erfolgen. Vorzugsweise führt man die Entspannung als Flash-Verdampfung durch, um die Suspension abzukühlen, vorzugsweise auf Temperaturen unterhalb 110 °C, insbesondere unterhalb 105 °C, z. B. im Bereich von 80 bis 110 °C, bevorzugt 90 bis 105 °C und ganz besonders bevorzugt 95 bis 100 °C. In der Regel erfolgt dann eine Verflüssigung der so aufgeschlossenen Stärke in einem separaten Reaktionsgefäß. Gegebenenfalls kann es sinnvoll sein, statt Zugabe der gesamten Menge des verflüssigenden Enzyms vor oder während des Erhitzens eine Teilmenge davon nach Einstellung der Temperatur zur zweiten Verflüssigung zuzugeben. Diese Teilmenge kann 0 bis 80 %, bevorzugt 10 bis 60 % und ganz besonders bevorzugt 15 bis 40 % der Gesamtmenge an verflüssigendem Enzym betragen. Die zweite Verflüssigung kann über einen Zeitraum von 30 bis 240 min, bevorzugt 45 bis 180 min und ganz besonders bevorzugt 60 bis 120 min, erfolgen. Die zweite Verflüssigung kann einem kontinuierlichen Strömungsrohr, kontinuierlich in einer Rührkesselkaskade oder in diskontinuierlichen Rührkesseln erfolgen. Bei Verwendung von Rührkesseln ist es vorteilhaft, eine ausreichende Anzahl von Rührkessein vorzusehen, die es erlaubt, einzelne Rührkessel parallel zum laufenden Betrieb zu reinigen, ohne Kapazität zu verlieren.

Zum vollständigen Abbau der Stärke zu Dextrinen wird das Reaktionsgemisch so lange bei der eingestellten Temperatur gehalten oder gegebenenfalls weiter erhitzt, bis der Stärkenachweis mit Jod oder gegebenenfalls ein anderer Test zum Nachweis von Stärke negativ oder mindestens im Wesentlichen negativ ausfällt. Gegebenenfalls können hierbei noch eine oder mehrere weitere Teilmengen $\alpha$-Amylase, z. B. im Bereich von 0,001 bis 0,5 Gew.-% und bevorzugt 0,002 bis 0,2 Gew.-%, bezogen auf die Gesamtmenge der eingesetzten Stärkequelle, zu der Reaktionsmischung gegeben werden.

Anstelle des Erhitzens der Maische durch Direktdampf, kann man diese auch indirekt mit einem Heizmedium, z. B. Dampf, in so genannten "Wide Gap"-Wärmetauschern auf die gewünschte Temperatur erhitzen, wodurch eine Verdünnung der Maismehlsuspension durch den eingetragenen Dampf vermieden wird. Auch hier werden in der Regel eine Nachreaktion und eine zweite Verflüssigung durchgeführt, wie für das Erhitzen mit Direktdampf beschrieben. Bezüglich der dabei ergriffenen Maßnahmen gilt das zuvor Gesagte in analoger Weise.

Auf diese Weise erhält man ein wässriges Stärkepartialhydrolysat, welches den verflüssigten Stärkeanteil aus dem Maismehl, typischerweise Dextrine und gegebenenfalls weitere Oligosaccharide und Mono- oder Disaccharide, sowie die Protein- und gegebenenfalls Kleiebestandteile des Maismehls enthält.

Nach abgeschlossener Verflüssigung der Stärke erfolgt eine Verzuckerung der in dem wässrigen Stärkepartialhydrolysat enthaltenen Dextrine, d. h. deren Abbau zu Glukose bzw. Saccharose. Die Verzuckerung kann kontinuierlich oder diskontinuierlich in an sich bekannter Weise durchgeführt werden.

Die Verzuckerung der Dextrine (d. h. Oligosaccharide) in der verflüssigten Stärkelösung erfolgt in der Regel auf enzymatischem Wege, d. h. mit Hilfe wenigstens eines die Dextrine verzuckernden Enzyms. Hierzu können grundsätzlich alle Glucoamylasen (Enzymklasse EC 3.2.1.3) eingesetzt werden, insbesondere Glucoamylasen, die aus Aspergillus gewonnen wurden und speziell solche, die zum Verzuckern von durch Dry-Milling-Verfahren gewonnenen Materialien im Rahmen der Herstellung von Bioethanol verwendet werden. Die zum Verzuckern geeigneten Glucoamylasen sind auch kommerziell erhältlich, beispielsweise von Novozymes unter der Bezeichnung Dextrozyme GA; oder von Genencor unter der Bezeichnung Optidex. Es kann auch eine Kombination verschiedener Glucoamylasen verwendet werden.

Das wenigstens eine verzuckernde Enzym, insbesondere wenigstens eine Glucoamylase, wird dem nach der Verflüssigung erhaltenen dextrinhaltigen Flüssigmedium üblicherweise in einer Menge von 0,001 bis 5,0 Gew.-%, bevorzugt von 0,005 bis 3,0 Gew.-% und besonders bevorzugt von 0,01 bis 1,0 Gew.-%, bezogen auf die Gesamtmenge der eingesetzten Stärkequelle, zugesetzt.

In der Regel wird die verflüssigte Stärkelösung üblicherweise auf das Temperaturoptimum des verzuckernden Enzyms oder leicht darunter, z. B. auf 40 bis 70 °C, bevorzugt 50 bis 65 °C und insbesondere 60 bis 63 °C abgekühlt bzw. temperiert und anschließend mit dem verzuckernden Enzym versetzt. Vorzugsweise wird das wässrige Stärkepartialhydrolysat unmittelbar im Anschluss an die Verflüssigung einer Verzuckerung unterworfen. Das heiße wässrige Stärkepartialhydrolysat wird dann auf die oben genannten Temperaturen abgekühlt, bevor das verzuckernde Enzym zugegeben wird. Diese Abkühlung erfolgt vorteilhaft in einem Wärmetauscher, wobei die freiwerdende Energie zur Vorwärmung anderer Prozessströme genutzt werden kann.

Vorteilhafterweise erfolgt die Verzuckerung bei einem pH-Wert im optimalen Wirkungsbereich des eingesetzten Enzyms, vorzugsweise bei einem pH-Wert im Bereich von 3,0 bis 5,5, insbesondere im Bereich von 4,0 bis 5,0 und besonders bevorzugt im Bereich von 4,2 bis 4,8. Vorzugsweise wird der pH-Wert vor Zugabe des verzuckernden Enzyms, insbesondere der Glucoamylase, auf den gewünschten Wert eingestellt.

Die Verzuckerung kann diskontinuierlich in Rührreaktoren oder kontinuierlich in einem Strömungsrohr oder besonders bevorzugt in einer Rührkesselkaskade erfolgen. Bei Verwendung von Rührkesseln ist es vorteilhaft, eine ausreichende Anzahl von Rührkesseln vorzusehen, die es erlaubt, einzelne Rührkessel parallel zum laufenden Betrieb zu reinigen, ohne Kapazität zu verlieren.

Nach Zugabe des verzuckernden Enzyms wird die dextrinhaltige Suspension vorzugsweise für einen Zeitraum von z. B. 8 bis 72 h oder länger, sofern erforderlich, häufig 12 bis 60 h, bevorzugt 24 bis 54 h und besonders bevorzugt 36 bis 48 h bei der eingestellten Temperatur gehalten, wobei die Dextrine zu Mono- und Disacchariden verzuckert werden. Der Fortschritt der Verzuckerung kann mit dem Fachmann bekannten Methoden, z. B. HPLC, Enzymtests oder Glucose-Teststäbchen, verfolgt werden. Die Verzuckerung ist abgeschlossen, wenn die Konzentration der Monosaccharide nicht mehr wesentlich ansteigt oder wieder fällt.

<u>Schritt c):</u>

Durch die Verzuckerung erhält man eine wässrige Glukoselösung, die neben Glukose noch die nicht hydrolysierten Bestandteile des Maismehls als Feststoffe in suspendierter Form enthält. Bei diesen Feststoffen handelt es sich in erster Linie um einen proteinreichen Feststoff, der hier und im Folgenden als Maisgluten bezeichnet wird, und, sofern eine Kleie-Rückführung bei der Vermahlung durchgeführt wurde, Kleiebestandteile enthält. Diese Bestandteile werden in Schritt c) des erfindungsgemäßen Verfahrens aus der Glukoselösung abgereichert. Hierbei kann man so vorgehen, dass man die Gesamtmenge der in Schritt b) hergestellten, Maisgluten enthaltenden Glukoselösung einer Feststoffabtrennung unterwirft. Man kann jedoch auch nur einen Teilstrom der in Schritt b) hergestellten, Maisgluten enthaltenden Glukoselösung einer Feststoffabtrennung unterwerfen und die verbleibende, Maisgluten enthaltende Glukose einer anderen Verwendung zuführen, beispielsweise einer Bioethanol-Herstellung.

In der Regel erfolgt eine Abreicherung in dem Maße, dass wenigstens 80 Gew.-%, vorzugsweise wenigstens 90 Gew.-% und insbesondere wenigstens 95 Gew.-% der in der Glukoselösung enthaltenen Glutenbestandteile bzw. Kleiebestandteile abgetrennt werden.

Die Abtrennung des Maisglutens und der gegebenenfalls vorhandenen Kleie kann auf jede bekannte Fest-/Flüssig-Trennung erfolgen, wobei mechanische Verfahren wie Zentrifugation, Dekantierung und Filtration, einschließlich Kombinationen dieser Maßnahmen, bevorzugt sind.

Für die Abtrennung der Feststoffe aus der Glukoselösung hat es sich als vorteilhaft erwiesen, wenn die der Abtrennung zugeführte Glukoselösung eine Temperatur im Bereich von 60 bis 100 °C, insbesondere im Bereich von 70 bis 90 °C und besonders bevorzugt im Bereich von 75 bis 85 °C aufweist. Hierzu wird man in der Regel die in Schritt b) erhaltene Glukoselösung vor der Abreicherung der Feststoffbestandteile Gluten und Kleie auf die gewünschte Temperatur erwärmen. Das Erwärmen erfolgt vorteilhaft in einem Wärmetauscher, wobei die benötigte Energie zur Abkühlung anderer Prozessströme genutzt werden kann.

Weiterhin hat es sich als vorteilhaft erwiesen, wenn der pH-Wert der Glukoselösung vor dem Abreichern der Feststoffe auf einen Wert im Bereich von 4,0 bis 6,5, insbesondere im Bereich von 4,5 bis 6,0 und besonders bevorzugt im Bereich von 5,0 bis 5,5 eingestellt wird. Zur Einstellung des pH-Wertes kann jede beliebige Base, vorzugsweise jedoch ein Alkalimetallhydroxid, z. B. Natronlauge, oder Ammoniak eingesetzt werden.

Bei der Abreicherung erhält man eine feststoffarme Glukoselösung und eine feststoffreiche Fraktion, welche Maisgluten und gegebenenfalls Kleiebestandteile enthält und die einen geringeren Glukoseanteil als die feststoffarme Glukoselösung aufweist.

Die feststoffarme Glukoselösung kann noch geringe Mengen an ungelösten Feststoff enthalten, wobei die Menge in der Regel 15 Vol.-%, insbesondere 10 Vol.-% und speziell 5 Vol.-%, bezogen auf das Gesamtvolumen der wässrigen Glukoselösung, nicht überschreitet und häufig im Bereich von 0,001 bis 15 Vol.-%, insbesondere im Bereich von 0,01 bis 10 Vol.-% und besonders bevorzugt im Bereich von 0,02 bis 5 Vol.-%, bezogen auf das Gesamtvolumen der wässrigen Glukoselösung, liegt. Die Bestimmung des ungelösten Feststoffes erfolgt durch Zentrifugation der Glukoselösung in graduierten Zentrifugenröhrchen bei 1650 g über 15 min und anschließendes Ablesen der Menge an ungelöstem Feststoff.

Zur Erzielung einer hohen Glukoseausbeute ist es von Vorteil, wenn man die bei der Fest-/Flüssig-Trennung erhaltene feststoffreiche Fraktion in Wasser resuspendiert und anschließend einer erneuten Fest-/Flüssig-Trennung unterzieht. Die Menge an Wasser liegt typischerweise im Bereich von 3 bis 15 l/kg suspendierter Feststoff, gerechnet als Trockensubstanz oder im Bereich von 3 bis 20 l pro l feuchtem, abgetrenntem Feststoff. Durch diese zweite Fest-/Flüssig-Trennung erhält man eine flüssige Phase, welche Teile der in der Feststoffphase der ersten Fest-/Flüssig-Trennung enthaltenen Glukose gelöst enthält. Die flüssige Phase wird dann der flüssigen Phase der ersten Fest-/Flüssig-Trennung zugeschlagen. Zur weiteren Erhöhung der Glukose-Ausbeute kann dieser Vorgang, d. h. das Resuspendieren des erhaltenen Feststoffs in Wasser und anschließende Fest-/Flüssig-Trennung ein- oder mehrfach wiederholt werden, wobei jeweils die erhaltenen wässrigen Glukoselösungen der in der ersten Fest-/Flüssig-Trennung erhaltenen Glukoselösung zugeschlagen werden.

Die Temperatur, bei der die zweite und die gegebenenfalls weiteren Fest-/Flüssig-Trennung(en) durchgeführt werden, liegt typischerweise im Bereich von 60 bis 100 °C, vorzugsweise im Bereich von 70 bis 90 °C und besonders bevorzugt im Bereich von 75 bis 85 °C. Bezüglich des pH-Wertes gilt das zuvor für die erste Fest-/Flüssig-Trennung Gesagte.

Das Wasser, welches zum Resuspendieren der feststoffreichen Fraktion der ersten und der weiteren Fest-/Flüssig-Trennungen verwendet wird, kann Frischwasser sein. Häufig wird man jedoch zum Resuspendieren die wässrige Glukoselösung einer Fest-/Flüssig-Trennung einsetzen, um zum einen die Verdünnung der zusammengeführten feststoffarmen Glukoselösungen der einzelnen Fest-/Flüssig-Trennungsstufen durch Frischwasser zu reduzieren und zum anderen den Frischwasserbedarf insgesamt zu verringern. Beispielsweise wird man bei drei sukzessiven Fest-/Flüssig-Trennungen die Flüssigphase der dritten Fest-/Flüssig-Trennung zum Resuspendieren der Feststoffphase der zweiten Fest-/Flüssig-Trennung verwenden und die flüssige Phase der zweiten Fest-/Flüssig-Trennung zum Resuspendieren der feststoffreichen Phase der ersten Fest-/Flüssig-Trennung verwenden. Neben dem Frischwasser kann aber auch Prozesswasser eingesetzt werden, das z. B. bei der späteren Eindampfung der Glucose-Lösung als Kondensat anfällt, oder dass bei der Trocknung der Nebenprodukte (z. B. Maisgluten oder Biomasse) anfällt.

Zur weiteren Reduzierung der Feststoffe in den so erhaltenen wässrigen Glukoselösungen kann es von Vorteil sein, diese einer so genannten Polierstufe zu unterwerfen, um weitere, darin enthaltene Feststoffe abzureichern. Die weitere Abreicherung kann auf jede bekannte Art der Fest-/Flüssig-Trennung erfolgen, wie beispielsweise Membranfiltration, einschließlich Mikrofiltration und Ultrafiltration, konventionelle Filtration, Flotation, Zentrifugieren, Dekantieren oder Separieren. Auch mehrstufige Ausführungsformen, die sich aus beliebiger Verschaltung der hier genannten Verfahren ergeben, sind denkbar.

Die feststoffarme Glukoselösung, welche nach Abreichern des Maisglutens und gegebenenfalls vorhandener Kleie aus der in Schritt b) erhaltenen wässrigen Glukose erhältlich ist, ist neu und eignet sich in besonderer Weise zur Herstellung von Chemikalien.

Unter dem Trockenmasseanteil versteht man die Gesamtmenge an gelösten und nichtgelösten Feststoffen in der wässrigen Glukoselösung. Diese lassen sich durch Eindampfen der Glukoselösung in an sich bekannter Weise ermitteln. Hierzu wird eine bestimmte Menge der jeweiligen Glukoselösung im Trockenschrank bei 80 °C zur Trockene eingedampft. Auswiegen des Trockenrückstandes liefert den Trockenmassegehalt. Alternativ können Trockenwaagen eingesetzt werden, wie sie beispielsweise von der Fa. PCE Deutschland, Meschede, zu diesem Zweck vertrieben werden.

Bezogen auf die in der wässrigen Glukoselösung enthaltenen Feststoffe weist die wässrige Glukoselösung die folgenden charakteristischen Bestandteile auf:

a) 80 bis 98 Gew.-%, vorzugsweise 93 bis 97 Gew.-% Zucker in Form von Glukose und gegebenenfalls Disacchariden wie Saccharose, Maltose und Isomaltose,
b) 1 bis 7 Gew.-%, häufig 2 bis 7 Gew.-%, vorzugsweise 2,5 bis 5 Gew.-% Rohprotein,
c) 0,001 bis 0,1 Gew.-%, häufig 0,01 bis 0,1 Gew.-% Rohfasern,
d) 200 bis 1500 mg/kg, vorzugsweise 600 bis 1200 mg/kg freie Aminosäuren und
e) 0,01 bis 1 Gew.-% Rohaschebestandteile.

Daneben kann die Glukoselösung noch geringe Mengen an Öl/Fett aus der Keimlingfraktion enthalten. Die Hauptmenge etwaiger Öl/Fettbestandteile wird jedoch in der Regel zusammen mit dem Gluten in Schritt c) abgetrennt. Gleiches gilt für etwaige Kleiebestandteile, die vor der Verzuckerung nicht abgetrennt wurden.

Im erfindungsgemäßen Verfahren fällt in einer Menge von 4 bis 40 Gew.-%, insbesondere 8 bis 30 Gew.-%, bezogen auf die Trockenmasse des eingesetzten Mais, an. Das Maisgluten weist in der Regel die folgende Bruttozusammensetzung auf, wobei die Angaben jeweils auf die Gesamttrockenmasse des Maisglutens bezogen sind.

a) 10 bis 60 Gew.-%, insbesondere 20 bis 55 Gew.-% Rohprotein;
b) 1 bis 60 Gew.-%, insbesondere 2 bis 45 Gew.-% Zuckerbestandteile;
c) bis zu 20 Gew.-%, häufig 0,5 bis 10 Gew.-% Rohfett, Pflanzenfette und/oder Pflanzenöle;
d) bis zu 20 Gew.-%, insbesondere 1 bis 12 Gew.-% Rohfaserbestandteile; und
e) bis zu 15 Gew.-%, z. B. 0,1 bis 10 Gew.-%, davon verschiedene, feste Bestandteile, auch als Rohasche bezeichnet.

Bei dem in Schritt c) abgetrennten Maisgluten handelt es sich um einen feinteiligen Feststoff, der nach Abtrennung in der Regel eine Feuchte im Bereich von 50 bis 85 Gew.-% und insbesondere im Bereich von 55 bis 75 Gew.-%, bezogen auf die Gesamtmasse des abgetrennten Maisglutens, aufweist. Das Maisgluten kann in an sich bekannter Weise zu einem feinteiligen, nicht bis leicht staubenden und nicht klebenden Pulver getrocknet werden. Die Feuchte liegt dann typischerweise unterhalb 50 Gew.-%, in der Regel unterhalb 30 Gew.-% und speziell unter 15 Gew.-%. Ein feuchtes Maisgluten mit meinem Trockenmasseanteil von 35 Gew.-%, bzw. einem Wassergehalt von 185 %, bezogen auf den trockenen Maisgluten, verhält sich wie ein Feststoff.

Die mittlere Teilchengröße der Maisglutenpartikel (Gewichtsmittel, bestimmt durch Lichtstreuung oder durch Siebanalyse), liegt typischerweise im Bereich von 50 bis 600 μm und insbesondere im Bereich von 100 bis 500 μm.

Das Maisgluten hat ein hohes Wasseraufnahmevermögen und ist in der Lage, bis zu 185 Gew.-% Wasser, bezogen auf sein Trockengewicht, zu absorbieren, ohne dabei klebrig zu werden. Es eignet sich daher in besonderer Weise als Formulierungshilfsmittel, insbesondere zur Herstellung von festen Formulierungen feuchter oder pastöser Stoffe, die ihrerseits zum Verkleben neigen. Insbesondere eignet sich das Maisgluten zur Formulierung einer Biomasse, wie sie bei einer Fermentation anfällt. Auf diese Weise erhält man ein nicht-klebriges, Biomasse und Maisgluten enthaltendes Produkt, das beispielsweise als Futtermittel oder Futtermittelbestandteil eingesetzt werden kann.

Das Maisgluten zeichnet sich zudem durch ein hohes Absorptionsvermögen für Öle und ölartige Substanzen, insbesondere für Pflanzenöle auf. Er eignet sich daher in besonderem Maße für die Herstellung fester Formulierungen hochwertiger Pflanzenöle oder Pflanzeölbestandteile oder Substanzen mit vergleichbaren Eigenschaften wie Tocopherole.

Die nach der/den Fest-/Flüssig-Trennung(en) erhaltene wässrige Glukose kann gegebenenfalls in einer ein- oder mehrstufigen Eindampfung auf die gewünschte Glukosekonzentration aufkonzentriert werden. Hierfür wird man die wässrige Glukoselösung bei Temperaturen im Bereich von 50 bis 100 °C, vorzugsweise im Bereich von 70 bis 95 °C und besonders bevorzugt im Bereich von 80 bis 90 °C, vorzugsweise unter Anlegen von vermindertem Druck, einengen. Vorzugsweise wird man das Einengen solange betreiben, bis eine Glukosekonzentration von wenigstens 40 Gew.-%, insbesondere wenigstens 50 Gew.-% und besonders bevorzugt wenigstens 55 Gew.-%, zum Beispiel im Bereich von 40 bis 80 Gew.-%, bevorzugt im Bereich von 50 bis 70 Gew.-% und ganz besonders bevorzugt im Bereich von 55 bis 65 Gew.-%, erreicht wird.

Verwendung der Glukose zur Herstellung von organischen Substanzen

Die so erhaltene Glukoselösung kann anschließend als Kohlenstoffquelle zur Herstellung von organischen Substanzen, d. h. Chemikalien, verwendet werden.

Der Begriff Chemikalien ist weit auszulegen und umfasst alle organischen Substanzen, d. h. definierte Verbindungen aber auch Oligomere, Polymere, einschließlich Enzyme, aber auch Biomasse wie z. B. Hefen oder Single Cell Protein, die ausgehend von Glukose, hergestellt werden oder werden können. Die Herstellung der organischen Substanz

kann sowohl durch Fermentation als auch auf nicht-fermentativem Wege erfolgen. Das Verfahren bietet insbesondere Vorteile bei der Herstellung von Chemikalien, die von Ethanol verschieden sind, da hier in der Regel höhere Anforderungen an die Glukosequalität bestehen.

Beispiele für organische Substanzen die auf nicht-fermentativem Wege aus Glukose herstellbar sind, umfassen 5-Hydroxymethylfurfural, Levulinsäure, Glukonsäure, Glukuronsäure, 2-Keto-Glukonsäure, Glutarsäure, Sorbitol, Isosorbid und Alkylpolyglukoside, Polyole wie Ethylenglycol, Propylenglycol und HFCS (High-Fructose-Corn Syrup).

Beispiele für organische Substanzen, die auf fermentativem Wege aus Glukose herstellbar sind:

- gegebenenfalls Hydroxylgruppen tragende Mono-, Di- und Tricarbonsäuren mit 2 bis 10 C-Atomen, z. B. Weinsäure, Itaconsäure, Bernsteinsäure, Essigsäure, Propionsäure, Milchsäure, 3-Hydroxypropionsäure, Fumarsäure, Maleinsäure, 2,5-Furandicarbonsäure, Glutarsäure, Lävulinsäure, Gluconsäure, Aconitsäure, Diaminopimelinsäure und Zitronensäure;
- proteinogene und nicht-proteinogene Aminosäuren, z. B. Lysin, Glutamat, Methionin, Phenylalanin, Asparaginsäure, Tryptophan und Threonin;
- Purinbasen und Pyrimidinbasen;
- Nukleoside und Nukleotide, z. B. Nikotinamidadenindinukleotid (NAD) und Adenosin-5’-monophosphat (AMP);
- Lipide,
- gesättigte und ungesättigte Fettsäuren mit vorzugsweise 10 bis 22 C-Atomen, z. B. γ-Linolensäure, Dihomo-γ-Linolensäure, Arachidonsäure, Eicosapentaensäure und Docosahexaensäure;
- Diole mit 3 bis 10 C-Atomen, z. B. Propandiol und Butandiol;
- mehrwertige Alkohole mit 3 oder mehr Hydroxylgruppen, z. B. mit 3, 4, 5 oder 6 OH-Gruppen, z. B. Glycerin, Sorbitol, Manitol, Xylitol und Arabinitol;
- langkettige Alkohole mit wenigstens 4 C-Atomen, z. B. mit 4 bis 22 C-Atomen, z. B. Butanol; Kohlenhydrate, z. B. Hyaluronsäure und Trehalose;
- Kohlenhydrate;
- aliphatische Amine, insbesondere aliphatische Diamine mit 3 bis 10 C-Atomen wie 1,5-Pentandiamin;
- aromatische Verbindungen, z. B. aromatische Amine, Vanillin und Indigo;
- Vitamine und Provitamine, z. B. Ascorbinsäure, Vitamin $B_6$, Vitamin $B_{12}$ und Riboflavin;
- Cofaktoren und Nutrazeutika;
- Proteine, z. B. Enzyme wie Amylasen, Pektinasen, saure, hybride oder neutrale Zellulasen, Esterasen wie Lipasen, Pankreasen, Proteasen, Xylanasen und Oxidoreduktasen wie Laccase, Katalase und Peroxidase, Glucanasen und Phytasen;
- Hefen, z. B. Backhefen oder Brauereihefen;
- Carotenoide, z. B. Lycopin, ß-Carotin, Astaxanthin, Zeaxanthin und Canthaxanthin;
- Ketone mit 3 bis 10 C-Atomen, z. B. Aceton und Acetoin;
- Lactone, z. B. γ-Butyrolacton;
- Polyhydroxyalkanoate, z. B. Polyhydroxyacetat;
- Polylactide;
- Polysaccharide, z. B. Glucan, Mannan, Galactan;
- Polyisoprenoide;
- Polyamide und
- Cyclodextrine.

Der Begriff "Cofaktor" umfasst nicht-proteinartige Verbindungen, die für das Auftreten einer normalen Enzymaktivität nötig sind. Diese Verbindungen können organisch oder anorganisch sein; die Cofaktor-Moleküle sind vorzugsweise organisch. Beispiele solcher Moleküle sind NAD und Nikotinamidadenindinukleotidphosphat (NADP); die Vorstufe dieser Cofaktoren ist Niacin.

Der Begriff "Nutrazeutikum" umfasst Nahrungsmittelzusätze, die bei Pflanzen und Tieren, insbesondere dem Menschen, gesundheitsfördernd sind. Beispiele solcher Moleküle sind Vitamine, Antioxidantien und bestimmte Lipide, z. B. mehrfach ungesättigte Fettsäuren.

<u>Verwendung der Glukose in einer Fermentation</u>

Weiterhin offenbart ist die Verwendung der erfindungsgemäß erhältlichen Glukoselösung als Glukoseque le für die fermentative Herstellung einer organischen Substanz, wie oben definiert.

Offenbart ist, ein Verfahren zur Herstellung einer organischen Substanz durch Fermentation, umfassend die folgenden Schritte:

i. Bereitstellung einer wässrigen Glukoselösung, z. B. durch Herstellung der Glukoselösung gemäß dem erfindungsgemäßen Verfahren und

ii. Zugabe der Glukoselösung zu einem Fermentationsmedium, das einen Mikroorganismus enthält, welcher zur Überproduktion der organischen Substanz befähigt ist.

Die Durchführung der Fermentation kann in der dem Fachmann bekannten üblichen Art und Weise erfolgen. Hierzu wird in man der Regel den jeweils gewünschten Mikroorganismus unter Verwendung einer erfindungsgemäß hergestellten wässrigen Glukose kultivieren.

Das Fermentationsverfahren kann sowohl diskontinuierlich (Batchfahrweise) als auch semikontinuierlich (Fed-Batch-Fahrweise, einschließlich Fed-Batch mit Zwischenernten) betrieben werden, wobei die semikontinuierliche Fahrweise bevorzugt ist.

Beispielsweise kann man die nach dem erfindungsgemäßen Verfahren erhaltene wässrige Glukoselösung - gegebenenfalls zusammen mit einer konventionellen Zuckerquelle, d. h. verstoffwechselbare Mono-, Di- und/oder Oligosaccharide oder die Zusammensetzung, welche verstoffwechselbare Mono-, Di- und/oder Oligosaccharide in einer Konzentration von wenigstens 45 Gew.-% enthält und welche typischerweise im Wesentlichen frei ist von in Wasser unlöslichen Feststoffen, z. B. eine Low Quality Molasse mit 45 und 50 Gew.-% Zucker - gegebenenfalls nach Verdünnen mit Wasser und Zugabe üblicher Medienbestandteile wie Puffer, Nährsalze, Stickstoffquellen wie Ammoniumsulfat, Harnstoff etc., komplexe Nährmedienbestandteile, enthaltend Aminosäuren, wie Hefeextrakte, Peptone, CSL und dergleichen, mit dem gewünschten Mikroorganismus inokulieren, und diesen unter Fermentationsbedingungen vermehren, bis die Mikroorganismenkonzentration den für die Fermentation gewünschten stationären Zustand erreicht. Hierbei werden die in der Glukoselösung enthaltenen Zucker verstoffwechselt und das gewünschte Wertprodukt gebildet (so genannte Batchfahrweise oder Batchphase).

Aufgrund des großen Anteils an freien Aminosäuren in der Glukose kann überraschenderweise auf den Zusatz sonstiger komplexer Nährmedienbestandteile verzichtet bzw. ihre Menge drastisch reduziert werden, was ein weiterer Vorteil der Glukoselösung ist.

Bei der Fed-Batch-Fahrweise wird man durch Zugabe der erfindungsgemäß erhältlichen Glukose den Fermentationsprozess weiter fortführen. Hierbei reichert sich das vom Mikroorganismus überproduzierte Stoffwechselprodukt in der Fermentationsbrühe an, wobei das Stoffwechselprodukt sowohl in den Zellen des Mikroorganismus als auch in der wässrigen Phase des Fermentationsmediums vorliegen kann.

Vorzugsweise wird man die Fermentation semikontinuierlich, d. h. als Fed-Batch durchführen. Dabei wird man so vorgehen, dass man den Mikroorganismus zunächst unter Verwendung einer Glukose Lösung und/oder einer anderen Zuckerquelle vermehrt, bis die gewünschte Mikroorganismenkonzentration im Fermenter erreicht ist. Anschließend wird die wässrige Glukose dem Fermenter zugeführt. Hierdurch wird der Fermentationsprozess aufrechterhalten und das vom Mikroorganismus überproduzierte Stoffwechselprodukt reichert sich in der Fermentationsbrühe an (s. o.). Insbesondere über die Zufuhrgeschwindigkeit der wässrigen Glukose kann der Zuckergehalt in der Fermentationsbrühe reguliert werden. In der Regel wird man die Zufuhrgeschwindigkeit so einstellen, dass die Glukosekonzentration in der Fermentationsbrühe im Bereich von > 0 Gew.-% bis etwa 5 Gew.-% liegt und insbesondere einen Wert von 3 Gew.-% nicht überschreitet.

Die Glukose kann vor der Fermentation gegebenenfalls sterilisiert werden, wobei man die kontaminierenden Mikroorganismen üblicherweise durch thermische Verfahren abtötet. Hierzu heizt man das zuckerhaltige Flüssigmedium üblicherweise auf Temperaturen oberhalb 80 °C auf. Die Abtötung bzw. Lyse der Kontaminanten kann unmittelbar vor der Fermentation erfolgen. Hierzu wird das gesamte zuckerhaltige Flüssigmedium der Sterilisation zugeführt.

Offenbart ist in Verfahren zur Herstellung von organischen nicht-flüchtigen Verbindungen mit mindestens 3 C-Atomen oder mit mindestens 2 C-Atomen und mindestens 1 N-Atom. Hierbei werden unter nichtflüchtigen organischen Verbindungen solche Verbindungen verstanden, die sich auf destillativem Weg nicht unzersetzt aus der Fermentationsbrühe gewinnen lassen. Diese Verbindungen weisen in der Regel einen Siedepunkt oberhalb des Siedepunktes von Wasser, häufig oberhalb 150 °C und insbesondere oberhalb 200 °C bei Normaldruck auf. In der Regel handelt es sich um Verbindungen, die bei Normalbedingungen (298 K, 101,3 kPa) in festem Zustand vorliegen.

Es ist jedoch auch möglich, das zuckerhaltige Flüssigmedium in einer Fermentation zur Herstellung nichtflüchtiger Stoffwechselprodukte einzusetzen, die bei Normaldruck einen Schmelzpunkt unterhalb des Siedepunktes von Wasser oder/und eine ölige Konsistenz aufweisen.

Insbesondere eignet sich das Verfahren zur Herstellung von Enzymen, Aminosäuren, Vitaminen, Nukleotiden, Di-, Oligo- und Polysacchariden, aliphatischen Mono- und Dicarbonsäuren mit 3 bis 10 C-Atomen, aliphatischen Hydroxycarbonsäuren mit 3 bis 10 C-Atomen, Ketonen mit 3 bis 10 C-Atomen, Alkanolen mit 4 bis 10 C-Atomen und Alkandiolen mit 3 bis 10 und insbesondere 3 bis 8 C-Atomen und Aminen, insbesondere aliphatischen Diaminen mit 3 bis 10 C-Atomen.

Es versteht sich für den Fachmann, dass die derart auf fermentativem Weg hergestellten Verbindungen jeweils in der von den eingesetzten Mikroorganismen produzierten Enantiomerenform erhalten werden (sofern unterschiedliche

Enantiomere existieren). So erhält man z. B. von den Aminosäuren in der Regel das jeweilige L-Enantiomer.

Die in der Fermentation eingesetzten Mikroorganismen richten sich in an sich bekannter Weise nach den jeweiligen Stoffwechselprodukten, wie unten im Einzelnen erläutert. Sie können natürlichen Ursprungs oder genetisch modifiziert sein. Beispiele für geeignete Mikroorganismen und Fermentationsverfahren sind z. B. in Tabelle A angegeben.

Tabelle A:

| Stoff | **Mikroorganismus** | **Referenz** |
|---|---|---|
| Weinsäure | Lactobacilli, (z. B. Lactobacillus delbrueckii) | Rehm, H.-J.: Biotechnology, Weinheim, VCH, 1980 und 1993-1995; Gutcho, Chemicals by Fermentation, Noyes Data Corporation (1973), |
| Itaconsäure | Aspergillus terreus, Aspergillus itaconicus | Jakubowska, in Smith u. Pateman (Hrsg.), Genetics and Physiology of Aspergillus, London: Academic Press 1977; Miall, in Rose (Hrsg.), Economic Microbiology, Vol. 2, S. 47 - 119, London: Academic Press 1978; US 3044941 (1962). |
| Bernsteinsäure | Actinobacillus sp. 130Z, Anaerobiospirillum succiniproducens, Actinobacillus succinogenes, E. coli | Int. J. Syst. Bacteriol. 26, 498 - 504 (1976); EP 249773 (1987), Erf.: Lemme u. Datta; US 5504004 (1996), Erf.: Guettler, Jain u. Soni; Arch. Microbiol. 167, 332 - 342 (1997); Guettler MV, Rumler D, Jain MK., Actinobacillus succinogenes sp. nov., a novel succinic-acid-producing strain from the bovine rumen. Int J Syst Bacteriol. 1999 Jan;49 Pt 1: 207-16; US5723322, US5573931, US5521075, WO99/06532, US5869301, US 5770435 |
| Hydroxypropiosäure | Lactobacillus delbrückii, L. leichmannii od. Sporolactobacillus inulinus | RÖMPP Online Version 2.2 |
| Propionsäure | Propionibacterium, z. B. P. arabinosum, P. schermanii, P. freudenreichii, Clostridium propionicum, | Rehm, H.-J.: Biotechnology, Weinheim, VCH, 1980 und 1993-1995; Gutcho, Chemicals by Fermentation, Noyes Data Corporation (1973), |
| Diaminopimelinsäure | Corynebacterium glutamicum | Rehm, H.-J.: Biotechnology, Weinheim, VCH, 1980 und 1993-1995; Gutcho, Chemicals by Fermentation, Noyes Data Corporation (1973), |
| Zitronensäure | Aspergillus niger, Aspergillus wentii | Crit. Rev. Biotechnol. 3, 331 - 373 (1986); Food Biotechnol. 7, 221- 234 (1993); 10, 13- 27 (1996). |
| Aconitsäure | Aspergillus niger, Aspergillus wentii | Crit. Rev. Biotechnol. 3, 331 - 373 (1986); Food Biotechnol. 7, 221- 234 (1993); 10, 13- 27 (1996).; Rehm, H.-J.: Biotechnology, Weinheim, VCH, 1980 und 1993-1995; |

(fortgesetzt)

| Stoff | **Mikroorganismus** | **Referenz** |
|---|---|---|
| Äpfelsäure | Aspergilli, z. B. Aspergillus flavus, A. niger, A. oryzae, Corynebacterium | US 3063910 |
| Gluconsäure | Aspergilli, z. B. A. niger | Gutcho, Chemicals by Fermentation, Noyes Data Corporation (1973), |
| Buttersäure | Clostridium (z. B. Clostridium acetobutylicum, C. butyricum) | Rehm, H.-J.: Biotechnology, Weinheim, VCH, 1980 und 1993-1995; |
| Milchsäure | Lactobacillus, z. B. L. delbrückii, L. leichmannii, | Rehm, H.-J.: Biotechnology, Weinheim, VCH, 1980 und 1993-1995; |
| Lysin | Corynebacterium glutamicum | Ikeda, M.: Amino Acid Production Process (2003), Adv. Biochem. Engin/Biotechnol 79, 1-35. |
| Glutamat | Corynebacterium glutamicum | Ikeda, M.: Amino Acid Production Process (2003), Adv. Biochem. Engin/Biotechnol 79, 1-35. |
| Methionin | Corynebacterium glutamicum | Ikeda, M.: Amino Acid Production Process (2003), Adv. Biochem. Engin/Biotechnol 79, 1-35. |
| Phenylalanin | Corynebacterium glutamicum, E.coli | Trends Biotechnol. 3, 64 - 68 (1985); J. Ferment. Bioeng. 70, 253- 260 (1990). |
| Threonin | E. coli | Ikeda, M.: Amino Acid Production Process (2003), Adv. Biochem. Engin/Biotechnol 79, 1-35. |
| Asparaginsäure | E. coli | Ikeda, M.: Amino Acid Production Process (2003), Adv. Biochem. Engin/Biotechnol 79, 1-35+dort. zit. Lit., Gutcho, Chemicals by Fermentation, Noyes Data Corporation (1973) |
| Purin- und Pyrimidinbasen | Bacillus subtilis | Rehm, H.-J.: Biotechnology, Weinheim, VCH, 1980 und 1993-1995; Gutcho, Chemicals by Fermentation, Noyes Data Corporation (1973), |
| Nikotinamidadenindinukleotid (NAD) | Bacillus subtilis | Rehm, H.-J.: Biotechnology, Weinheim, VCH, 1980 und 1993-1995; Gutcho, Chemicals by Fermentation, Noyes Data Corporation (1973), |
| Adenosin-5' -monophosphat (AMP) | Bacillus subtilis | Rehm, H.-J.: Biotechnology, Weinheim, VCH, 1980 und 1993-1995; Gutcho, Chemicals by Fermentation, Noyes Data Corporation (1973), |

(fortgesetzt)

| Stoff | **Mikroorganismus** | **Referenz** |
|---|---|---|
| γ-Linolensäure | Mucor, Mortiella, Aspergillus spp. | Gill, I., Rao, V.: Polyunsaturated fatty acids, part 1: occurrence, biological activities and applications (1997). Trends in Biotechnology 15 (10), 401-409; Zhu, H.: Utilization of Rice Bran by Pythium irregulare for Lipid Production. Master Thesis Louisiana State University, 31.10.2002 (URN etd-1111102-205855). |
| Dihomo-γ-Linolensäure | Mortiella, Conidiobolus, Saprolegnia spp. | Gill, I., Rao, V.: Polyunsaturated fatty acids, part 1: occurrence, biological activities and applications (1997). Trends in Biotechnology 15 (10), 401-409; Zhu, H.: Utilization of Rice Bran by Pythium irregulare for Lipid Production. Master Thesis Louisiana State University, 31.10.2002 (URN etd-1111102-205855). |
| Arachidonsäure | Mortiella, Phytium spp. | Gill, I., Rao, V.: Polyunsaturated fatty acids, part 1: occurrence, biological activities and applications (1997). Trends in Biotechnology 15 (10), 401-409; Zhu, H.: Utilization of Rice Bran by Pythium irregulare for Lipid Production. Master Thesis Louisiana State University, 31.10.2002 (URN etd-1111102-205855). |
| Eicosapentaensäure | Mortiella, Phytium spp., Rhodopseudomonas, Shewanella spp. | Gill, I., Rao, V.: Polyunsaturated fatty acids, part 1: occurrence, biological activities and applications (1997). Trends in Biotechnology 15 (10), 401-409; Zhu, H.: Utilization of Rice Bran by Pythium irregulare for Lipid Production. Master Thesis Louisiana State University, 31.10.2002 (URN etd-1111102-205855). |
| Docosahexaensäure | Thraustochytrium, Entomophthora spp., Rhodopseudomonas, Shewanella spp. | Gill, I., Rao, V.: Polyunsaturated fatty acids, part 1: occurrence, biological activities and applications (1997). Trends in Biotechnology 15 (10), 401-409; Zhu, H.: Utilization of Rice Bran by Pythium irregulare for Lipid Production. Master Thesis Louisiana State University, 31.10.2002 (URN etd-1111102-205855). |
| Propandiol | E. coli | DE 3924423, US 440379, WO 9635799, US 5164309 |

(fortgesetzt)

| Stoff | **Mikroorganismus** | **Referenz** |
|---|---|---|
| Butandiol | Enterobacter aerogenes" Bacillus subtilis, Klebsiella oxytoca | Rehm, H.-J.: Biotechnology, Weinheim, VCH, 1980 und 1993-1995; Gutcho, Chemicals by Fermentation, Noyes Data Corporation (1973); H. G. SCHLEGEL and H. W. JANNASCH, 1981; Afschar et al.: Mikrobielle Produktion von 2,3-Butandiol. CIT 64 (6), 2004, 570-571 |
| Butanol | Clostridium (z. B. Clostridium acetobutylicum, C. propionicum) | Rehm, H.-J.: Biotechnology, Weinheim, VCH, 1980 und 1993-1995; Gutcho, Chemicals by Fermentation, Noyes Data Corporation (1973), |
| Glycerin | Hefe, Saccharomyces rouxii, | Gutcho, Chemicals by Fermentation, Noyes Data Corporation (1973), |
| Mannitol | Aspergillus candida, Torulopsis mannitofaciens | Gutcho, Chemicals by Fermentation, Noyes Data Corporation (1973), |
| Arabitol | Saccharomyces rouxii, S. mellis, Sclerotium glucanicum, Pichia ohmeri | Gutcho, Chemicals by Fermentation, Noyes Data Corporation (1973), |
| Xylitol | Saccharomyces cerevisiae | Gutcho, Chemicals by Fermentation, Noyes Data Corporation (1973), |
| Hyaluronsäure | Streptococcus sp. | Rehm, H.-J.: Biotechnology, Weinheim, VCH, 1980 und 1993-1995; |
| Trehalose | Brevibacterium, Corynebacterium, Microbacterium, Arthrobacter spp., Pleurotus genus , Filobasidium floriforme | JP 05099974, JP 06311891, FR 2671099, EP 0555540, JP 3053791, Miyazaki, J.-I., Miyagawa, K.-I., Sugiyama, Y.: Trehalose Accumulation by Basidiomycotinous Yeast, Filobasidium floriforme. Journal of Fermentation and Bioengineering 81, (1996) 4, 315-319. |
| Ascorbinsäure | Gluconobacter melanogenes | RÖMPP Online Version 2.2 |
| Vitamin $B_{12}$ | Propionibacterium spp., Pseudomonas denitrificans | Chem. Ber. 1994, 923 - 927; RÖMPP Online Version 2.2 |
| Riboflavin | Bacillus subtilis, Ashbya gossypii | WO 01/011052, DE 19840709, WO 98/29539, EP 1186664; Fujioka, K.: New biotechnology for riboflavin (vitamin B2) and character of this riboflavin. Fragrance Journal (2003), 31(3), 44-48. |
| Vitamin $B_6$ | Rhizobium tropici, R. meliloti | EP0765939 |

(fortgesetzt)

| Stoff | **Mikroorganismus** | **Referenz** |
|---|---|---|
| Enzyme | Apergilli (z. B. Aspergillus niger A. oryzae), Trichoderma , E.coli, Hansenula oder Pichia (z. B. Pichia pastorius), Bacillus (z. B. Bacillus licheniformis, B. subtilis) u. v. a. | Rehm, H.-J.: Biotechnology, Weinheim, VCH, 1980 und 1993-1995; Gutcho, Chemicals by Fermentation, Noyes Data Corporation (1973), |
| Zeaxanthin | Dunaliella salina | Jin et al (2003) Biotech.Bioeng. 81: 115- 124 |
| Canthaxanthin | Brevibacterium | Nelis et al (1991) J Appl Bacteriol 70: 181- 191 |
| Lycopin | Blakeslea trispora, Candida utilis | WO 03/056028, EP 01/201762, WO 01/12832, WO 00/77234, Miura et al (1998) Appl Environ Microbiol 64: 1226-1229 |
| β-Carotin | Blakeslea trispora, Candida utilis | Kim S., Seo W., Park Y., Enhanced production of beta-carotene from Blakeslea trispora with Span 20, Biotechnology Letters, Vol 19, No 6, 1997, 561-562; Mantouridou F., Roukas T.: Effect of the aeration rate and agitation speed on beta-carotene production and morphology of Blakeslea trispora in a stirred tank reactor: mathematical modelling, Biochemical Engineering Journal 10 (2002), 123-135; WO 93/20183; WO 98/03480, Miura et al (1998) Appl Environ Microbiol 64:1226- 1229 |
| Astaxanthin | Phaffia rhodozyma; Candida utilis | US 5,599,711; WO 91/02060, Miura et al (1998) Appl Environ Microbiol 64:1226-1229 |
| Polyhydroxyalkanoate, Polyester | Escherchia coli, Alcaligenes latus, u.v.a. | S. Y. Lee, Plastic Bacteria, Progress and Prospects for polyhydroxyalkanoate production in bacteria, Tibtech, Bd. 14, (1996), S. 431-438., Steinbüchel, 2003; Steinbüchel (Hg.), Biopolymers, 1. Aufl., 2003, Wiley-VCH, Weinheim und dort zitierte Literatur |
| Polysaccharide | Leuconostoc mesenteroides, L. dextranicum, Xanthomonas campestris, u. v. a. | Rehm, H.-J.: Biotechnology, Weinheim, VCH, 1980 und 1993-1995; Gutcho, Chemicals by Fermentation, Noyes Data Corporation (1973), |
| Polyisoprenoide | *Lactarius sp., Hygrophorus sp., Russula sp.* | Steinbüchel (Hg.), Biopolymers, 1. Aufl., 2003, Wiley-VCH,Weinheim und dort zitierte Literatur |

(fortgesetzt)

| Stoff | **Mikroorganismus** | **Referenz** |
|---|---|---|
| Aceton | Clostridium (z. B. Clostridium acetobutylicum, C. propionicum) | Rehm, H.-J.: Biotechnology, Weinheim, VCH, 1980 und 1993-1995; Gutcho, Chemicals by Fermentation, Noyes Data Corporation (1973) |
| Acetoin | Enterobacter aerogenes, Clostridium acetobutylicum, Lactococcus lactis | Lengeler, J.W., Drews, G., Schlegel, H.G.: Hrsg., Biology of the Procaryotes, Thieme, Stuttgart (1999), S.307; RÖMPP Online-Edition |
| Vanillin | Pseudomonas putida, Amycolatopsis sp. | Priefert, H., Rabenhorst, J., Seinbüchel, A. Biotechnological production of vanillin. Appl. Microbiol. Biotechnol. 56, 296-314 (2001) |
| Thurigensin | *Bacillus thuringiensis* | Jian-Zhong Jong et al.: Fed-batch culture of Bacillus thuringiensis for thuringensin production in a tower type bioreactor. Biotechnology and Bioengineering 48 (3) (2004), 207-213. |
| Polyketide | *Streptomyces fradiae, Sorangium cellulosum* | Kirst: Fermentation-derived compounds as a source for new products. Pure & Appl. Chem. 70 (2), (1998), 335-338; Zirkle et al.: Heterologous production of the antifungal polyketide antibiotic soraphen A of Sorangium cellulosum So ce26 in Streptomyces lividans. Microbiology 150 (8), (2004), 2761-74. |
| Gibberellinsäure | *Gibberella fujikuroi* | Hollmann et al.: Extraktiv-Fermentation von Gibberellinsäure mit Gibberella fujikuroi. CIT 7 (1995), 892-895. |
| Indigo | Escherichia coli JB 102 | Berry, A., Dodge, T.C., Pepsin, M., Weyler, W.: Application of metabolic engineering to improve both the production and use of biotech indigo. Journal of Industrial Microbiology & Biotechnology 28 (2002), 127-133. |

Die hergestellte organische Verbindung ist unter gegebenenfalls Hydroxylgruppen tragenden Mono-, Di- und Tricarbonsäuren mit 3 bis 10 C-Atomen, proteinogenen und nicht-proteinogenen Aminosäuren, Purinbasen, Pyrimidinbasen; Nukleosiden, Nukleotiden, Lipiden; gesättigten und ungesättigten Fettsäuren; Diolen mit 4 bis 10 C-Atomen, mehrwertigen Alkoholen mit 3 oder mehr Hydroxylgruppen, langkettigen Alkoholen mit wenigstens 4 C-Atomen, Kohlenhydraten, insbesondere Di, Oligo- und Polysacchariden, aromatischen Verbindungen, Vitaminen, Provitaminen, Cofaktoren, Nutrazeutika, Proteinen, Carotenoiden, Ketonen mit 3 bis 10 C-Atomen, Lactonen, Aminen, Biopolymeren und Cyclodextrinen ausgewählt.

Offenbart ist der Einsatz des zuckerhaltigen Flüssigmediums in einer fermentativen Herstellung von Enzymen wie Phytasen, Xylanasen oder Glucanasen.

Offenbart ist der Einsatz des zuckerhaltigen Flüssigmediums in einer fermentativen Herstellung von Aminosäuren wie Lysin, Methionin, Threonin oder Glutamat.

Offenbart ist der Einsatz des zuckerhaltigen Flüssigmediums in einer fermentativen Herstellung von Vitaminen wie Pantothensäure und Riboflavin, Vorläufern und Folgeprodukten davon.

Offenbart ist der Einsatz des zuckerhaltigen Flüssigmediums in einer fermentativen Herstellung von

- Mono-, Di- und Tricarbonsäuren, insbesondere aliphatischen Mono- und Dicarbonsäuren mit 2 bis 10 C-Atomen wie Essigsäure, Propionsäure, Fumarsäure und Bernsteinsäure;
- aliphatischen Hydroxycarbonsäuren mit 3 bis 10 C-Atomen wie Milchsäure;
- langkettigen Alkanolen wie zuvor genannt, insbesondere Alkanolen mit 4 bis 10 C-Atomen wie Butanol;
- Diolen wie zuvor genannt, insbesondere Alkandiolen mit 3 bis 10 und insbesondere 3 bis 8 C-Atomen wie Propandiol;
- Ketonen wie zuvor genannt, insbesondere Ketonen mit 3 bis 10 C-Atomen wie Aceton;
- Aminen, insbesondere aliphatischen Diaminen mit 3 bis 10 C-Atomen wie 1,5-Diaminopentan;
- Nukleotiden wie 5'-IMP und 5'-GMP, und
- Kohlehydraten wie zuvor genannt, insbesondere Disacchariden wie Trehalose, Oligosacchariden und Polysacchariden wie Glucan.

Es handelt sich bei dem in der Fermentation von den Mikroorganismen produzierten Stoffwechselprodukt um Polyhydroxyalkanoate wie Poly-3-hydroxybutyrat und Copolyester mit anderen organischen Hydroxycarbonsäuren wie 3-Hydroxyvaleriansäure, 4-Hydroxybuttersäure und anderen, die in Steinbüchel (a. a. O.) beschrieben sind, z. B. auch langkettige (auch bezeichnet als längerkettige) Hydroxycarbonsäuren wie 3-Hydroxyoctansäure, 3-Hydroxydecansäure und 3-Hydroxytetradecansäure, sowie Mischungen davon. Zur Durchführung der Fermentation können hier analoge Bedingungen und Vorgehensweisen angewendet werden, wie sie für andere Kohlenstoffquellen beschrieben wurden, z. B. in S. Y. Lee, Plastic Bacteria Progress and prospects for polyhydroxyalkanoate production in bacteria, Tibtech, Bd. 14, (1996), S. 431-438.

Die in der Fermentation eingesetzten Mikroorganismen sind daher ausgewählt unter natürlichen oder rekombinanten Mikroorganismen, die wenigstens eines der folgenden Stoffwechselprodukte überproduzieren:

- Enzyme wie Phytase, Xylanase oder Glucanase;
- Aminosäuren wie Lysin, Threonin, Glutamat oder Methionin;
- Vitamine wie Pantothensäure und Riboflavin; Vorläufer und/oder Folgeprodukte davon;
- Disaccharide wie Trehalose;
- Polysaccharide wie Glucan;
- aliphatische Mono- und Dicarbonsäuren mit 3 bis 10 C-Atomen wie Propionsäure, Fumarsäure und Bernsteinsäure;
- aliphatische Hydroxycarbonsäuren mit 3 bis 10 C-Atomen wie Milchsäure;
- Polyhydroxyalkanoate wie Poly-3-hydroxybutyrat und Copolyester der 3-Hydroxybuttersäure;
- Ketone mit 3 bis 10 C-Atomen wie Aceton;
- Aminen, insbesondere aliphatischen Diaminen mit 3 bis 10 C-Atomen wie 1,5-Diaminopentan;
- Alkanole mit 4 bis 10 C-Atomen wie Butanol; und Alkandiole mit 3 bis 8 C-Atomen wie Propandiol.

Geeignete Mikroorganismen sind üblicherweise ausgewählt unter den Gattungen Corynebacterium, Brevibacterium, Bacillus, Ashbya, Escherichia, Aspergillus, Alcaligenes, Actinobacillus, Anaerobiospirillum, Lactobacillus, Propionibacterium, Rhizopus, Clostridium, Schizophyllum und Sclerotium, insbesondere unter Stämmen von Corynebacterium glutamicum, Corynebacterium sp AJ-1526, Brevibacterium ammoniagenes, Bacillus subtilis, Bacillus megaterium, Ashbya gossypii, Escherichia coli, Aspergillus niger, Aspergillus terreus, Aspergillus itaconicus, Alcaligenes latus, Anaerobiospirillum succiniproducens, Actinobacillus succinogenes, Lactobacillus delbrückii, Lactobacillus leichmannii, Propionibacterium arabinosum, Propionibacterium schermanii, Propionibacterium freudenreichii, Clostridium propionicum, Clostridium formicoaceticum, Clostridium acetobutylicum, Rhizopus arrhizus, Rhizopus oryzae, Schizophyllum commune und Sclerotium rolflsii.

Es handelt sich bei dem in der Fermentation eingesetzten Mikroorganismus um einen Stamm aus der Gattung Corynebacterium, insbesondere um einen Stamm des Corynebacterium glutamicum. Insbesondere handelt es sich um einen Stamm der Gattung Corynebacterium, speziell des Corynebacterium glutamicum, welcher eine Aminosäure, speziell Lysin, Methionin oder Glutamat überproduziert.

Es handelt sich bei dem in der Fermentation eingesetzten Mikroorganismus um einen Stamm aus der Gattung Escherichia, insbesondere um einen Stamm des Escherichia coli. Insbesondere handelt es sich um einen Stamm der Gattung Escherichia, speziell des Escherichia coli, welcher eine Aminosäure, speziell Lysin, Methionin oder Threonin überproduziert.

Es handelt sich bei dem in der Fermentation von den Mikroorganismen produzierten Stoffwechselprodukt um Lysin. Zur Durchführung der Fermentation können hier analoge Bedingungen und Vorgehensweisen angewendet werden, wie sie für andere Kohlenstoffquellen beschrieben wurden, z. B. in Pfefferle et al., a. a. O., und US 3,708,395.

Prinzipiell kommen sowohl eine kontinuierliche als auch eine diskontinuierliche (Batch oder Fed-Batch) Betriebsweise in Betracht, bevorzugt ist die Fed-Batch-Betriebsweise.

Es handelt sich bei dem in der Fermentation von den Mikroorganismen produzierten Stoffwechselprodukt um Methionin. Zur Durchführung der Fermentation können hier analoge Bedingungen und Vorgehensweisen angewendet werden, wie sie für andere Kohlenstoffquellen beschrieben wurden, z. B. in WO 03/087386 und WO 03/100072. Im Falle der Lysinherstellung wird hierzu aus der in Schritt c) erhaltenen Glukoselösung zusammen mit Nährsalzen und komplexen Nährmedienbestandteilen, z. B. Melasse, ein Medium für die Fermentation von Lysin hergestellt. Dieses Medium kann durch Dampf direkt oder indirekt sterilisiert werden. Nach Sterilisation wird das Medium in einer Fermentation zur Herstellung von Lysin mit üblichen Mikroorganismen, z. B. Corynebacterium glutamicum, eingesetzt. Nach Abschluss der Fermentation enthält die Fermentationsbrühe neben Lysin auch den Mikroorganismus (Biomasse), gelöste Bestandteile des Nährmediums und ggf. auch nicht-stärkehaltige feste Bestandteile der Stärkequelle, die durch die Fest-/Flüssig-Trennung (s. Kapitel 2.2.3) nicht komplett abgetrennt werden konnten. Die Gewinnung von Lysin kann in üblicher Weise erfolgen und ist weiter unten näher erläutert.

Es handelt sich bei dem in der Fermentation von den Mikroorganismen produzierten Stoffwechselprodukt um Pantothensäure. Zur Durchführung der Fermentation können hier analoge Bedingungen und Vorgehensweisen angewendet werden, wie sie für andere Kohlenstoffquellen beschrieben wurden, z. B. in der WO 01/021772.

Es handelt sich bei dem in der Fermentation von den Mikroorganismen produzierten Stoffwechselprodukt um Riboflavin. Zur Durchführung der Fermentation können hier analoge Bedingungen und Vorgehensweisen angewendet werden, wie sie für andere Kohlenstoffquellen beschrieben wurden, z. B. in WO 01/011052, DE 19840709, WO 98/29539, EP 1186664 und Fujioka, K.: New biotechnology for riboflavin (vitamin B2) and character of this riboflavin. Fragrance Journal (2003), 31 (3), 44-48.

Es handelt sich bei dem in der Fermentation von den Mikroorganismen produzierten Stoffwechselprodukt um Fumarsäure. Zur Durchführung der Fermentation können hier analoge Bedingungen und Vorgehensweisen angewendet werden, wie sie für andere Kohlenstoffquellen beschrieben wurden, z. B. in Rhodes et al., Production of Fumaric Acid in 20-L Fermentors, Applied Microbiology, 1962, 10 (1), 9-15.

Es handelt sich bei dem in der Fermentation von den Mikroorganismen produzierten Stoffwechselprodukt um Milchsäure. Zur Durchführung der Fermentation können hier analoge Bedingungen und Vorgehensweisen angewendet werden, wie sie für andere Kohlenstoffquellen beschrieben wurden, z. B. in Narayanan et al., Electronic J. Biotechnol. 2004, 7, http://www.ejbiotechnology.info/content/vol7/issue2/full/7/pdf.

Es handelt sich bei dem in der Fermentation von den Mikroorganismen produzierten Stoffwechselprodukt um Bernsteinsäure. Zur Durchführung der Fermentation können hier analoge Bedingungen und Vorgehensweisen angewendet werden, wie sie für andere Kohlenstoffquellen beschrieben wurden, z. B. in Int. J. Syst. Bacteriol. 26, 498 - 504 (1976); EP 249773 (1987), Erf.: Lemme u. Datta; US 5,504,004 (1996), Erf.: Guettler, Jain u. Soni; Arch. Microbiol. 167, 332 - 342 (1997); Guettler MV, Rumler D, Jain MK., Actinobacillus succinogenes sp. nov., a novel succinic-acid-producing strain from the bovine rumen. Int J Syst Bacteriol. 1999 Jan; 49 Pt 1:207-16; US 5,723,322, US 5,573,931, US 5,521,075, WO99/06532, US 5,869,301 oder US 5,770,435.

Es handelt sich bei dem in der Fermentation von den Mikroorganismen produzierten Stoffwechselprodukt um Itaconsäure. Zur Durchführung der Fermentation können hier analoge Bedingungen und Vorgehensweisen angewendet werden, wie sie für andere Kohlenstoffquellen beschrieben wurden, z. B. in Kautola, H., Appl. Microb. Biotechnol., 1990, 33,7 und Willke et al., Appl. Microbiol. Biotechnol., 2001, 56, 289.

Es handelt sich bei dem in der Fermentation von den Mikroorganismen produzierten Stoffwechselprodukt um eine Phytase. Zur Durchführung der Fermentation können hier analoge Bedingungen und Vorgehensweisen angewendet werden, wie sie für andere Kohlenstoffquellen beschrieben wurden, z. B. in der WO 98/55599.

Es handelt sich bei dem in der Fermentation von den Mikroorganismen produzierten Stoffwechselprodukt um Glucan. Zur Durchführung der Fermentation können hier analoge Bedingungen und Vorgehensweisen angewendet werden, wie sie für andere Kohlenstoffquellen beschrieben wurden, z. B. in Schilling et al.: Repression of oxalic acid biosynthesis in the unsterile scleroglucan productino process with Sclerotium rolfsii ATCC 15205, Bioprocess Engineering 22 (2000), 51-55 oder Rau et al.: Oxygen controlled batch cultivations of Schizophyllum commune for enhanced production of branched ß-1,3-glucans, Bioprocess Engineering 11 (1994), 161-165.

Es handelt sich bei dem in der Fermentation von den Mikroorganismen produzierten Stoffwechselprodukte um Nukleotide wie 5'-IMP und 5'-GMP. Zur Durchführung der Fermentationen können hier analoge Bedingungen und Vorgehensweisen angewendet werden, wie sie für andere Kohlenstoffquellen beschrieben wurden, z. B. in Sato et al., Accumulation of Guanosine Polyphosphates by Brevibacterium ammoniagenes: Isolation and Identification of the Products. Agr. Biol. Chem. 40 (3), 1976, 465-474; Mori et al: A novel process of inosine 5'-monophosphate production using overexpressed guanosine/inosine kinase. Appl. Microbiol. Biotechnol. (1997) 48: 693-698, oder GB 01188885.

Es handelt sich bei dem in der Fermentation von den Mikroorganismen produzierten Stoffwechselprodukt um Glutamat. Zur Durchführung der Fermentationen können hier analoge Bedingungen und Vorgehensweisen angewendet werden, wie sie für andere Kohlenstoffquellen beschrieben wurden, z. B. in E. Kimura, L-Glutamate Production, in:

Handbook of Corynebacterium glutamicum, CRC press, Boca Raton, Fl, 439-464.

Es handelt sich bei dem in der Fermentation von den Mikroorganismen produzierten Stoffwechselprodukt um 1,5-Diaminopentan. Zur Durchführung der Fermentationen können hier analoge Bedingungen und Vorgehensweisen angewendet werden, wie sie für andere Kohlenstoffquellen beschrieben wurden, z. B. in JP 2004222569.

Es handelt sich bei dem in der Fermentation von den Mikroorganismen produzierten Stoffwechselprodukt um 5-Keto-Glukonsäure. Zur Durchführung der Fermentation können hier analoge Bedingungen und Vorgehensweisen angewendet werden, wie sie für andere Kohlenstoffquellen beschrieben wurden, z. B. in Elfari, M. et al., Appl. Microbiol. Biotechnol. 2005, 66,668, und Herrmann U., et al., Appl. Microbial. Bioetchnol. 2004, 64, 86.

Es handelt sich bei dem in der Fermentation von den Mikroorganismen produzierten Stoffwechselprodukt um 2,5-Diketo-Glukonsäure. Zur Durchführung der Reaktion können hier analoge Bedingungen und Vorgehensweisen angewendet werden, wie sie für andere Kohlenstoffquellen beschrieben wurden, z. B. in Roper, H., Starch-Starke 1990, 42, 342 oder Zelic, B. et al., Chem. Biochem. Eng. Q. 2002, 16,7.

<u>Aufarbeitung der Fermentation</u>

Bei dem Verfahren zur Herstellung einer organischen Substanz durch Fermentation resultiert eine Fermentationsbrühe, die neben dem gewünschten Stoffwechselprodukt im Wesentlichen die während der Fermentation erzeugte Biomasse und nicht verwertete Zucker, sowie nicht verwertete Puffer- und Nährsalze enthält. In der Regel schließt sich daher der Fermentation eine Weiterverarbeitung der Fermentationsbrühe an, um die das Wertprodukt, d.h. die durch das Fermentationsverfahren hergestellte organische Substanz zu gewinnen und in eine handhabbare bzw. handelbare Form zu überführen sowie um die bei der Fermentation anfallenden Nebenprodukte wie Biomasse und die wässrigen Bestandteile zu Entsorgen oder einer weiteren Verwertung zuzuführen.

Die Art der Aufarbeitung und die hierfür erforderlichen Schritte richten sich in an sich bekannter Weise nach den Stoffeigenschaften der Fermentationsbrühe und insbesondere nach der Art des hergestellten Stoffwechselproduktes.

Typischerweise weisen Aufarbeitungsverfahren einen oder mehrere der folgenden Schritte auf, die in beliebiger Reihenfolge und Ausprägung verschaltet werden können:

- Deaktivierung des Mikroorganismus, z. B. durch Sterilisation in der oben beschriebenen Weise;
- Abtrennung der Biomasse von der Fermentationsbrühe;
- Isolation des nichtflüchtigen Stoffwechselproduktes aus der Fermentationsbrühe, die noch Biomasse enthält oder von der die Biomasse schon abgetrennt wurde;
- Aufreinigung des gewünschten Stoffwechselproduktes;
- Aufkonzentrierung des Stoffwechselproduktes;
- Aufkonzentrierung der Biomasse.

Die Schritte müssen dabei nicht alle zwingender Bestandteil des Aufarbeitungsverfahrens sein. Beispielsweise kann auf eine zusätzliche Aufreinigung des oder der Stoffwechselprodukte verzichtet werden, wenn keine hohen Anforderungen an die Reinheit des Produktes gestellt werden.

Die Abtrennung der Biomasse aus der Fermentationsbrühe erfolgt nach üblichen Verfahren der Fest-Flüssig-Phasenseparation (z. B. beschrieben in Belter, P. A, Bioseparations: Downstream Processing for Biotechnology, John Wiley & Sons (1988), und Ullmann's Encyclopedia of Industrial Chemistry, 5. Aufl. auf CD-ROM, Wiley-VCH) und erfolgt in der Regel durch mechanische Verfahren, wie Dekantierung, Separation, Flotation, Zentrifugation, Sedimentation, Filtration oder Membranverfahren. Dabei sind auch mehrstufige Verschaltungen eines Verfahrens oder Kombinationen unterschiedlicher Verfahren denkbar, wie z. B. Dekantierung und Separation. Des Weiteren kann auch Waschwasser eingesetzt werden, um die Ausbeute des nichtflüchtigen Stoffwechselproduktes bei der Biomassenabtrennung zu erhöhen. Bevorzugt werden die oben genannten Verfahren eingesetzt, wenn es sich bei dem Stoffwechselprodukt um einen Stoff handelt, der in Lösung in der Fermentationsbrühe vorliegt. Bei öligen oder festen Stoffwechselprodukten, ist eine mechanische Abtrennung mittels Dekantierung, Separation, Flotation, Zentrifugation, Sedimentation in der Regel dann sinnvoll, wenn es Dichteunterschiede zwischen der Biomasse und dem Stoffwechselprodukt gibt. Ansonsten kommen dann auch insbesondere chromatographische Verfahren, Destillationsverfahren oder Extraktionsverfahren in Betracht.

Die Isolierung oder Abreicherung des Wertprodukts aus der Fermentationsbrühe erfolgt in der Regel derart, dass man wenigstens ein Wertprodukt so aus der Fermentationsbrühe abreichert oder isoliert, dass der Gehalt dieses Wertprodukts in der verbleibenden Fermentationsbrühe höchstens 20 Gew.-%, insbesondere höchstens 10 Gew.-%, speziell höchstens 5 Gew.-% und ganz speziell höchstens 2,5 Gew.-%, jeweils bezogen auf das Gesamtgewicht der verbleibenden Fermentationsbrühe, beträgt. Die Isolierung oder Abreicherung des Wertprodukts aus der Fermentationsbrühe kann in einem oder mehreren Schritten erfolgen.

Zur Isolierung eines in der Fermentationsbrühe gelösten Wertprodukts wird man vorteilhafterweise so vorgehen, dass man zunächst die Biomasse und sonstige ungelöste Bestandteile aus der Fermentationsbrühe entfernt, z. B. mittels Zentrifugation oder Filtration, und anschließend das Wertprodukt aus der flüssigen Phase isoliert, z. B. durch Kristallisation, Fällung, Adsorption, Destillation, Chromatographie, Extraktion, Ionenaustausch, Membranverfahren (bevorzugt Diffusionsdialyse, Elektrodialyse, Nanofiltration). Alternativ kann das Wertprodukt auch direkt aus der Fermentationsbrühe isoliert werden, z. B. durch Einsatz chromatographischer Verfahren, Extraktionsverfahren, Membranverfahren, Adsorptionsverfahren und Destillation. Als chromatographisches Verfahren ist insbesondere die Ionenaustauschchromatographie zu nennen, bei der das Wertprodukt selektiv auf der Chromatographiesäule isoliert werden kann.

Zur Abtrennung des Wertproduktes kann es sinnvoll sein, das Wertprodukt in einem ersten Schritt in der Fermentationsbrühe chemisch zu modifizieren, z. B. durch Veresterung oder Salzbildung, um dadurch die Abtrennbarkeit zu verbessern.

Die Kristallisation ist ein Verfahren, das sowohl eine Abtrennung des Wertproduktes aus der Fermentationsbrühe ermöglicht, als auch eine weitere Reinigung des Wertproduktes erlaubt. Es wird dann bevorzugt in Kombination mit einer mechanischen Abtrennung, wie bereits oben genannt, angewendet, in der die Kristalle von der Mutterlauge abgetrennt werden können.

Im Falle flüchtiger oder öliger Verbindungen ist in der Regel eine Kontrolle der maximalen Temperaturen während der Aufarbeitung, insbesondere während der Trocknung erforderlich. Vorteilhaft können diese Verbindungen auch dadurch isoliert werden, dass man sie in quasi fester Form (pseudofester Form) auf Adsorbentien formuliert. Zu diesem Zweck geeignete Adsorbentien sind z. B. in der WO 2005/116228 der Anmelderin angegeben, beispielsweise Aktivkohlen, Aluminiumoxide, Kieselgele, Kieselsäure, Ton, Ruße, Zeolithe, anorganische Alkali- und Erdalkalimetallsalze wie Natrium-, Kalium-, Magnesium- und Calciumhydroxide, -carbonate, -silikate, -sulfate, -phosphate, insbesondere Magnesium- und Calciumsalze, z. B. $Mg(OH)_2$, $MgCO_3$, $MgSiO_4$, $CaSO_4$, Ca-$CO_3$, Erdalkalimetalloxide, z. B. MgO und CaO, andere anorganische Phosphate und Sulfate, z. B. $ZnSO_4$, Salze organischer Säuren, insbesondere deren Alkali- und Erdalkalimetallsalze und speziell deren Natrium- und Kaliumsalze, z. B. Natrium- und Kaliumacetat, -formiat, -hydrogenformiate und -citrat, höhermolekulare organische Träger wie gegebenenfalls modifizierte Stärken, Cellulose, Lignin, die weiter unten im Zusammenhang mit der Produktformulierung genannten Trägermaterialien sowie der Maisgluten. Beispiele für Wertprodukte, die vorteilhaft auf diese Weise isoliert werden können, sind γ-Linolensäure, Dihomo-γ-Linolensäure, Arachidonsäure, Eicosapentaensäure und Docosahexaensäure, weiterhin Propionsäure, Milchsäure, Propandiol, Butanol und Aceton. Auch diese Verbindungen in pseudofester Formulierung werden als nichtflüchtige Stoffwechselprodukte bzw. Wertprodukte in fester Form verstanden.

Die oben genannten Verfahrensschritte der Aufarbeitung können teilweise den Einsatz von Zusatzstoffen erfordern (z. B. für die Regenerierung des Ionenaustauschers, das Lösungsmittel für die Extraktion usw.) und/oder es kann teilweise ein Nebenstoffstrom anfallen (z. B. Mutterlauge der Kristallisation, Eluat des Ionenaustauschers). Diese Nebenstoffströme, die teilweise noch das Wertprodukt, die Biomasse, nicht-stärkehaltige feste Bestandteile des als Stärkequelle eingesetzten Mais und Anteile der Zusatzstoffe enthalten können, können entweder weiter aufgearbeitet, teilweise zu irgendeinem Verfahrensschritt in dem Gesamtprozess zurückgeführt, entsorgt oder weiterverwertet werden.

Sämtliche oben genannten Ströme, bevorzugt die Biomassehaltigen Ströme, die Wertprodukthaltigen Ströme sowie die Produktströme enthalten unter Umständen hohe Wasserkonzentrationen (bedingt durch Fermentation oder Waschwasser in der Aufarbeitung) und können aufkonzentriert werden (Reduktion des Wassergehaltes). Dies kann thermisch, z. B. mittels Eindampfung, Trocknung oder mechanisch mittels Membranverfahren, Filtration usw. geschehen. Das Wasser kann entsorgt oder als Prozesswasser zurückgeführt werden und z. B. zum Anmaischen der Endosperm-Fraktion oder zum Anmaischen des abgetrennten Festsstoffs bei der mehrstufigen Maisglutenabtrennung eingesetzt werden.

Offenbart ist ein Verfahren, bei dem man die flüchtigen Bestandteile der Fermentationsbrühe ohne vorherige Isolierung oder Abreicherung des Wertprodukts, und gegebenenfalls ohne vorherige Abtrennung der Biomasse weitgehend oder vollständig entfernt, wobei man eine feste Formulierung des Wertprodukts erhält. Eine genauere Beschreibung zur Durchführung eines solchen Verfahrens findet sich in der WO 2007/028804 der Anmelderin, auf die hiermit Bezug genommen wird.

Durch Zugabe von Formulierungshilfsmitteln wie Träger- und Coatingmaterialien, Bindemitteln sowie anderer Additive können die Eigenschaften des getrockneten und zusammen mit den festen Bestandteilen der Fermentation vorliegenden Wertprodukts gezielt hinsichtlich verschiedener Parameter wie Wirkstoffgehalt, Korngröße, Partikelform, Neigung zum Stauben, Hygroskopizität, Stabilität, insbesondere Lagerstabilität, Farbe, Geruch, Fließverhalten, Agglomerationsneigung, elektrostatischer Aufladung, Licht- und Temperaturempfindlichkeit, mechanischer Stabilität und Redispergierbarkeit in an sich bekannter Weise konfektioniert werden.

Zu den üblicherweise verwendeten Formulierungshilfsmitteln gehören z. B. Bindemittel, Trägermaterialien, Puderungs-/Fließhilfsmittel, ferner Farbpigmente, Biozide, Dispergiermittel, Antischaummittel, viskositätsregulierende Mittel, Säuren, Laugen, Antioxidantien, Enzymstabilisatoren, Enzyminhibitoren, Adsorbate, Fette, Fettsäuren, Öle oder Mischungen hieraus. Derartige Formulierungshilfsmittel werden vorteilhaft insbesondere bei Anwendung von Formulierungs- und Trocknungsverfahren wie Sprüh-, Wirbelschicht- und Gefriertrocknung als Trocknungshilfsmittel eingesetzt. Wegen weiterer Details wird auf die WO 2007/028804 verwiesen

Der Anteil an den vorgenannten Zusatzstoffen und gegebenenfalls weiteren Zusatzstoffen wie Coatingmaterialien kann je nach den speziellen Erfordernissen des jeweiligen Wertprodukts sowie in Abhängigkeit von den Eigenschaften der eingesetzten Zusatzstoffe stark variieren und z. B. im Bereich von 0,1 bis 80 Gew.-% und insbesondere im Bereich von 1 bis 30 Gew.-%, jeweils bezogen auf das Gesamtgewicht des fertig formulierten Produkts bzw. Stoffgemischs, liegen.

Die Zugabe von Formulierungshilfsmitteln kann vor, während oder nach der Aufarbeitung der Fermentationsbrühe (auch als Produktformulierung oder Feststoffdesign bezeichnet) und insbesondere während der Trocknung erfolgen. Eine Zugabe von Formulierungshilfsmitteln vor Aufarbeitung der Fermentationsbrühe bzw. des Wertprodukts kann insbesondere vorteilhaft sein, um die Prozessierbarkeit der aufzuarbeitenden Stoffe bzw. Produkte zu verbessern. Die Formulierungshilfsmittel können sowohl dem in fester Form erhaltenen Wertprodukt als auch einer dieses enthaltenden Lösung oder Suspension zugegeben werden, z. B. nach abgeschlossener Fermentation direkt zu der Fermentationsbrühe oder zu einer im Verlauf der Aufarbeitung erhaltenen Lösung oder Suspension vor dem abschließenden Trocknungsschritt.

So können die Hilfsstoffe z. B. in eine Suspension des Wertprodukts eingemischt werden; eine solche Suspension kann auch auf ein Trägermaterial gegeben werden, z. B. durch Aufsprühen oder Untermischen. Die Zugabe von Formulierungshilfsstoffen während der Trocknung kann z. B. eine Rolle spielen, wenn eine das Wertprodukt enthaltende Lösung oder Suspension versprüht wird. Insbesondere nach der Trocknung erfolgt eine Zugabe von Formulierungshilfsmitteln z. B. beim Aufbringen von Überzügen bzw. Coatings/Coatingschichten auf getrocknete Partikel. Sowohl nach dem Trocknen als auch nach einem eventuellen Coatingschritt können dem Produkt weitere Hilfsmittel zugegeben werden.

Das Entfernen der flüchtigen Bestandteile aus der Fermentationsbrühe erfolgt in an sich bekannter Weise durch übliche Verfahren zur Abtrennung fester Phasen von flüssigen Phasen, einschließlich Filtrationsverfahren und Verfahren zum Verdampfen flüchtiger Bestandteile der flüssigen Phasen. Derartige Verfahren, die auch Schritte zur Grobreinigung der Wertstoffe sowie Schritte zur Konfektionierung umfassen können, werden z. B. in Belter, P. A, Bioseparations: Downstream Processing for Biotechnology, John Wiley & Sons (1988), und Ullmann's Encyclopedia of Industrial Chemistry, 5. Aufl. auf CD-ROM, Wiley-VCH, beschrieben. Im Rahmen der Produktformulierung bzw. der Aufarbeitung nach abgeschlossener Fermentation anwendbare, dem Fachmann bekannte Verfahren, Apparaturen, Hilfsstoffe bzw. allgemeine und spezielle Beispiele sind ferner in EP 1038 527, EP 0648 076, EP 835613, EP 0219 276, EP 0394 022, EP 0547 422, EP 1088 486, WO 98/55599, EP 0758 018 und WO 92/12645 beschrieben.

In einer ersten Variante wird man das nichtflüchtige Wertprodukt, sofern es in der flüssigen Phase gelöst vorliegt, aus der flüssigen Phase in die feste Phase überführen, z. B. durch Kristallisation oder Fällung. Anschließend erfolgt eine Abtrennung der nichtflüchtigen festen Bestandteile einschließlich des Wertprodukts, z. B. mittels Zentrifugation, Dekantieren oder Filtration. In ähnlicher Weise kann man auch ölige Wertprodukte abtrennen, wobei man die jeweiligen öligen Fermentationsprodukte durch Zusatz von Adsorbentien, z. B. Kieselsäure, Kieselgele, Lehm, Ton und Aktivkohle, in eine feste Form überführt.

In einer zweiten Variante werden die flüchtigen Bestandteile durch Verdampfen entfernt. Das Verdampfen kann in an sich bekannter Weise erfolgen. Beispiele für geeignete Verfahren zum Verdampfen flüchtiger Bestandteile sind die Sprühtrocknung, Wirbelschichttrocknung bzw. -agglomeration, Gefriertrocknung, Trocknen in Strom- und Kontakttrocknern sowie Extrusionstrocknung. Auch eine Kombination der vorgenannten Verfahren mit formgebenden Verfahren wie Extrusion, Pelletierung oder Prilling kann vorgenommen werden. Bei diesen letztgenannten Verfahren werden vorzugsweise teilweise oder weitgehend vorgetrocknete wertprodukthaltige Stoffgemische eingesetzt.

Das Entfernen der flüchtigen Bestandteile der Fermentationsbruhe umfasst ein Verfahren zur Sprühtrocknung oder ein Verfahren der Wirbelschichttrocknung, einschließlich der Wirbelschichtgranulierung. Hierzu wird die Fermentationsbrühe, gegebenenfalls nach einer Vorabtrennung zur Entfernung grober Feststoffpartikel, die keine oder nur geringe Anteile an nichtflüchtigem Wertprodukt enthalten, einer oder mehreren Sprüh- oder Wirbelschichttrocknungsapparaturen zugeführt. Der Transport bzw. die Zuführung der feststoffbeladenen Fermentationsbrühe erfolgt zweckmäßigerweise mittels üblicher Transportvorrichtungen für feststoffhaltige Flüssigkeiten, z. B. Pumpen wie Exzenterschneckenpumpen (z. B. der Fa. Delasco PCM) oder Hochdruckpumpen (z. B. der Fa. LEWA Herbert Ott GmbH).

Im speziellen Fall der Herstellung von Lysin umfasst das Aufarbeitungsverfahren eine Abtrennung der Biomasse durch Separatoren. Die biomassehaltige Fraktion wird dann in Trommel- bzw. Röhrenbündeltrockner getrocknet. Gegebenenfalls wird vor der Trocknung ein Fermentationsrückstand der Vitamin $B_2$-Fermentation, das so genannte "BFR" (Vitamin $B_2$ Fermentation Residues) zur biomassehaltigen Fraktion zugemischt. Die feststoffarme Fraktion wird angesäuert und über einen Ionentauscher gefahren. Auf diesem Ionentauscher wird das Lysin gebunden. Die Lysin-abgereicherte Fermentationsbrühe, die den Ionentauscher verlässt, wird durch das Abdampfen von Wasser aufkonzentriert; dabei auskristallisierte Feststoffe werden abgetrennt und getrocknet. Das dabei entstehende Produkt wird als "Fertilizer" bezeichnet und kann in den Prozess zurückgeführt oder als Düngemittel und/oder für weitere Anwendungen eingesetzt

werden. Die Mutterlauge der Kristallisation wird als sogenanntes "CMS" (Condensed Molasses Solubles) einer Weiterverarbeitung zugeführt. Das an den Ionentauscher gebundene Lysin wird mit Ammoniakwasser eluiert und durch Abdampfen von Wasser aufkonzentriert. Aus dieser aufkonzentrierten Brühe kann Lysin als freie Base in Form einer flüssigen Formulierung entnommen werden. Im nächsten Prozessschritt wird das Lysin durch Zugabe von Salzsäure als Lysin-Hydrochlorid auskristallisiert. Die Kristalle werden durch Zentrifugation abgetrennt und getrocknet. Die Mutterlauge der Kristallisation wird entweder zum Eluat des Ionentauschers zurückgeführt oder kann als Lysin in flüssiger Formulierung entnommen werden.

Als Alternative zur beschriebenen Aufarbeitung wird nach der Fermentation die Lysinhaltige Fermentationsbrühe direkt sprühgetrocknet. Optional kann der Fermentationsrückstand der Vitamin $B_2$-Produktion zugegeben werden. Eine mögliche ein- oder mehrstufige Voreindampfung der Fermentationsbrühe kann zur Reduzierung von Energiekosten und Investition führen.

Verwendung der Glukose in einer nicht-fermentativen Umsetzung

Offenbart ist die Verwendung der erfindungsgemäß erhältlichen Glukoselösung als Glukosequelle für die nicht-fermentative Herstellung einer organischen Substanz, wie oben definiert.

Offenbart ist ein Verfahren zur Herstellung einer organischen Substanz durch nicht-fermentative Umsetzung, umfassend die folgenden Schritte:

i. Bereitstellung einer wässrigen Glukoselösung, z. B. durch Herstellung der Glukoselösung gemäß dem erfindungsgemäßen Verfahren und

ii. Verwendung der Glukoselösung oder einer durch Einengen der Lösung erhaltenen, im Wesentlichen wasserfreien Glukose (Wassergehalt < 10 Gew.-%) in einer nicht-fermentative Umsetzung zur Herstellung der gewünschten organischen Substanz.

Die Durchführung der nicht-fermentativen Umsetzung kann in der dem Fachmann bekannten üblichen Art und Weise erfolgen. Hierzu wird in man der Regel die erfindungsgemäß hergestellte wässrige Glukose gegebenenfalls unter Einsatz eines Katalysators umsetzen.

Es nandet sich bei der organischen Substanz, die auf nicht-fermentativem Weg aus Glukose herstellbar ist, um 5-Hydroxymethylfurfural. Zur Durchführung der Reaktion können hier analoge Bedingungen und Vorgehensweisen angewendet werden, wie sie für andere Kohlenstoffquellen beschrieben wurden, z. B. in Cottier et al., Trends Heterocycl. Chem. 1991, 2, 233; Lewkowski, J., Arkivoc 2001, 2, 17; Kuster, B.F.M. et al., Carbohydr. Res. 1977, 54, 159, EP 0230250, FR 2464260 oder DE 3601281.

Es nandet sich bei der organischen Substanz, die auf nicht-fermentativem Weg aus Glukose herstellbar ist, um Levulinsäure. Zur Durchführung der Reaktion können hier analoge Bedingungen und Vorgehensweisen angewendet werden, wie sie für andere Kohlenstoffquellen beschrieben wurden, z. B. in Horvat et al, Tetrahedron Lett. 1985, 26, 2111 oder US 3258481.

Es nandelt sich bei der organischen Substanz, die auf nicht-fermentativem Weg aus Glukose herstellbar ist, um Glukonsäure. Zur Durchführung der Reaktion können hier analoge Bedingungen und Vorgehensweisen angewendet werden, wie sie für andere Kohlenstoffquellen beschrieben wurden, z. B. in Lichtenthaler, F.W., Acc. Chem. Res. 2002, 35, 728, Besson, M. et al., J. Catal. 1995, 152, 116 oder EP 233816.

Es handelt sich bei der organischen Substanz, die auf nicht-fermentativem Weg aus Glukose herstellbar ist, um Glukuronsäure. Zur Durchführung der Reaktion können hier analoge Bedingungen und Vorgehensweisen angewendet werden, wie sie für andere Kohlenstoffquellen beschrieben wurden, z. B. in Corma, A. et al., Chemical Routes for the Transformation of Biomass into Chemicals., Chem. Rev. 2007, 107, 2411-2502.

Es handelt sich bei der organischen Substanz, die auf nicht-fermentativem Weg aus Glukose herstellbar ist, um 2-Keto-Glukonsäure. Zur Durchführung der Reaktion können hier analoge Bedingungen und Vorgehensweisen angewendet werden, wie sie für andere Kohlenstoffquellen beschrieben wurden, z. B. in US 2002177198, WO 9915673 oder EP 867446.

Es handelt sich bei der organischen Substanz, die auf nicht-fermentativem Weg aus Glukose herstellbar ist, um Glutarsäure. Zur Durchführung der Reaktion können hier analoge Bedingungen und Vorgehensweisen angewendet werden, wie sie für andere Kohlenstoffquellen beschrieben wurden, z. B. in Besson, M. et al., Recl. Trav. Chim. Pys-Bas 1996,115, 217 und Dirkx, J.M.H. et al., J. Catal. 1981, 67,1.

Es handelt sich bei der organischen Substanz, die auf nicht-fermentativem Weg aus Glukose herstellbar ist, um Sorbitol. Zur Durchführung der Reaktion können hier analoge Bedingungen und Vorgehensweisen angewendet werden, wie sie für andere Kohlenstoffquellen beschrieben wurden, z. B. in Dechamp, N. et al., Catal. Today 1995, 24, 29 und Maranhao, L.C. A. et al., Ind. Eng. Chem. Res. 2005, 44, 9624, WO 02100537, WO 02100539 und WO

2004052813.

Es handelt sich bei der organischen Substanz, die auf nicht-fermentativem Weg aus Glukose herstellbar ist, um Isosorbid. Zur Durchführung der Reaktion können hier analoge Bedingungen und Vorgehensweisen angewendet werden, wie sie für andere Kohlenstoffquellen beschrieben wurden, z. B. in WO 9804540, WO 9200947 und US 4297290.

Es handelt sich bei der organischen Substanz, die auf nicht-fermentativem Weg aus Glukose herstellbar ist, um Alkylpolyglukoside. Zur Durchführung der Reaktion können hier analoge Bedingungen und Vorgehensweisen angewendet werden, wie sie für andere Kohlenstoffquellen beschrieben wurden, z. B. in US 5480979 und US 5698684.

Es handelt sich bei der organischen Substanz, die auf nicht-fermentativem Weg aus Glukose herstellbar ist, um HFCS (High-Fructose-Corn-Syrup). Zur Durchführung der Reaktion können hier analoge Bedingungen und Vorgehensweisen angewendet werden, wie sie für andere Kohlenstoffquellen beschrieben wurden, z. B. in Marshall et al., Enzymatic Conversion of d-Glucose to d-Fructose 1957, Science 125 (3249), 648 und US 4523960.

<u>Formulierung der Nebenprodukte</u>

Wie bereits oben erläutert fallen bei sowohl in den Schritten a) und c) des erfindungsgemäßen Verfahrens der Glukoseherstellung, aber auch bei der fermentativen Weiterverarbeitung der Glukose zu Wertprodukten eine Reihe von Stoffströmen als Neben- bzw. Koppelprodukte an. In der Regel handelt es sich dabei um einen oder mehrere der folgenden Stoffströme, vorzugsweise in den angegebenen Mengen:

- staubiger Feinanteil der Maisreinigung, sofern anfallend, typischerweise in einer Menge bis 5 Gew.-%, insbesondere 0,1 bis 3 Gew.-%;
- Maiskleie, sofern anfallend, typischerweise in einer Menge bis 7 Gew.-%, z. B. 1 bis 6 Gew.-%;
- Maiskeimling, typischerweise in einer Menge von 2 bis 16 Gew.-%, bevorzugt 4 bis 12 Gew.-%
- Maisgluten, typischerweise in einer Menge von 4 bis 40 Gew.-%, bevorzugt 8 bis 30 Gew.-%
- Biomasse, typischerweise in einer Menge von 1 bis 40 Gew.-%, bevorzugt 5 bis 20 Gew.-% und
- gegebenenfalls Nebenstoffströme, die in dem Aufarbeitungsverfahren des Wertproduktes anfallen können, sofern anfallend, typischerweise in einer Menge bis 100 Gew.-%, bevorzugt 0,2 bis 50 Gew.-%, besonders bevorzugt 0,3 bis 20 Gew.-%,

wobei alle Gew.-%-Angaben auf die Gesamtmasse des zur Glukoseherstellung eingesetzten Mais bezogen sind.

Diese Stoffströme können getrennt verarbeitet oder einer Entsorgung zugeführt werden. Ebenfalls ist es möglich, diese Stoffströme im Rahmen einer Weiterverarbeitung in beliebiger Weise, d. h. teilweise oder vollständig in beliebiger Kombination miteinander zu vermischen (d. h. Zusammenbringen mindestens zweier Stoffströme). In der Regel erfolgt vor der Weiterverarbeitung eine Trocknung, wobei gegebenenfalls die miteinander zu vermischenden Stoffströme vor dem Vermischen oder nach dem Vermischen getrocknet werden. Häufig wird man so vorgehen, dass man die festen Partikel der zumindest teilweise von Wasser befreiten Stoffströme agglomeriert oder gemeinsam mahlt.

Die Verfahrensschritte Trocknen, Agglomerieren und Mahlen können optional und in beliebiger Reihenfolge zum Mischen verschiedener Ströme durchgeführt und kombiniert werden. Vorzugsweise wird man so vorgehen, dass man beim Vermischen der Stoffströme ein Nebenprodukt erhält, das bevorzugt futtermitteltauglich ist und mindestens einen Anteil aus den Stoffströmen der Maisverarbeitung (bzw. Zuckerherstellung) enthält und mindestens einen Bestandteil aus der Aufarbeitung der Fermentationsbrühe (Biomasse oder Nebenstoffströme) enthält.

Gegebenenfalls kann man den so hergestellten Nebenprodukte Formulierungshilfsmittel, Wirkstoffe oder eine oder mehrere Biomassen oder einen oder mehrere Nebenstoffströme anderer Fermentationsverfahren zusetzen, wobei dieser Zusatz an jeder beliebigen Stelle des Verfahrens zerfolgen kann.

Die Restfeuchten dieser Nebenprodukte betragen im ungetrockneten Zustand 10 bis 90 Gew.-%, bevorzugt 40 bis 80 Gew.-%. Im getrockneten Zustand betragen die Restfeuchte der Nebenprodukte 1 bis 20 Gew.-% und bevorzugt 3 bis 18 Gew.-% und besonders bevorzugt 5 bis 15 Gew.-%.

Der mittlere Partikeldurchmesser des Feststoffanteils der Nebenprodukte liegt zwischen 50 μm und 8 mm, bevorzugt zwischen 100 μm und 5 mm und besonders bevorzugt zwischen 150 μm und 3 mm.

Handelt es sich bei einem Nebenprodukt aus einer Mischung verschiedener Feststofffraktionen, so wird man vor dem Vermischen die Partikelgrößenverteilungen der einzelnen Stoffströme, aus denen sich das Nebenprodukt zusammensetzt, in der Regel so wählen bzw. einstellen, dass die Entmischung der Stoffströme nicht auftritt oder zumindest gering bleibt. Dies ist in der Regel dann gewährleistet, wenn die zu vermischenden Stoffströme eine möglichst ähnliche Teilchengröße aufweisen, bzw. wenn der so genannte SPAN-Wert der Nebenproduktmischung kleiner als 4, bevorzugt kleiner als 3, besonders bevorzugt kleiner als 2 und insbesondere kleiner 1,8 ist. Hierbei ist der SPAN-Wert der Nebenproduktmischung definiert als

$$SPAN = (D_{90} - D_{10}) / D_{50}$$

Der $D_{50}$ Wert ist dabei der gewichtsmittlere Partikeldurchmesser des Nebenproduktgemisches, d. h. bezogen auf die Masse gibt $D_{50}$-Wert denjenigen Partikeldurchmesser an, den 50 Gew.-% der Partikel überschreiten bzw. 50 Gew.-% unterschreiten. Der $D_{90}$-Wert ist derjenige Durchmesser, den 90 Gew.-% der Partikel unterschreiten bzw. 10 Gew.-% überschreiten. Der $D_{10}$-Wert ist derjenige Durchmesser, den 10 Gew.-% der Partikel unterschreiten bzw. 90 Gew.-% überschreiten. Der Spanwert bzw. die Teilchendurchmesser und ihre Verteilung können in an sich bekannter Weise bestimmt werden, z. B. durch Siebanalyse oder durch Lichtstreuung.

Wird ein Nebenprodukt aus mindestens einem trockenen Stoffstrom und mindestens einem flüssigen Strom hergestellt, so können zum einen die flüssigen Stoffströme zunächst getrocknet werden und danach wie feste Stoffströme behandelt werden (s. o.). Für die Mischung dieser Stoffströme gilt das gleiche, wie für die Mischung der bereits ursprünglich trockenen Stoffströme. Zum anderen können aber auch die flüssigen und die trockenen Stoffströme vor einer Trocknung oder während der Trocknung miteinander gemischt werden. Dies hat den Vorteil, dass der in dem flüssigen oder suspensionsartigen Stoffstrom enthaltende Feststoff in die trockenen Stoffströme gut eingemischt und verteilt wird, oder der flüssige Stoffstrom als Coating auf die festen Bestandteile der trockenen Stoffströme aufgezogen wird oder die flüssigen Stoffströme genutzt werden, um die festen Partikel des trocknen Stoffstrom zu agglomerieren bzw. zu binden.

Der staubige Feinanteil wird verworfen und nicht mit in die Nebenprodukte eingemischt.

Die Maiskleie wird nicht mit in die Nebenprodukte eingemischt, sondern als eigenständiges Produkt verwendet.

Der Maiskeimling wird nicht mit in die Nebenprodukte eingemischt, sondern als eigenständiges Produkt verwendet, z. B. zur Gewinnung von Maiskeimöl.

Das Maisgluten wird nicht mit in die Nebenprodukte eingemischt, sondern als eigenständiges Produkt verwendet.

wird Die Biomasse wird nicht mit in die Nebenprodukte eingemischt, sondern als eigenständiges Produkt verwendet.

Die Nebenstoffströme werden nicht mit in die Nebenprodukte eingemischt, sondern als eigenständige Produkte verwendet oder verworfen bzw. entsorgt.

Ein Anteil oder die Gesamt- \ menge der anfallenden Maiskleie, beispielsweise 10 bis 100 Gew.-%, bezogen auf Trockenmassegehalt der insgesamt anfallenden Maiskleie wird mit mindestens einem Nebenstoffstrom, z. B. mit 10 bis 100 Gew.-%, bezogen auf den jeweiligen Nebenproduktstrom vermischt und getrocknet, um so ein maiskleiehaltiges Nebenprodukt zu erhalten. Optional kann die Maiskleie vor dem Vermischen vermahlen werden, so dass mittlere Partikelgrößen von 150 bis 1400 $\mu$m und besonders bevorzugt 200 $\mu$m bis 800 $\mu$m eingestellt werden. Eine weitere Option besteht darin, vor oder nach der Mahlung einen Teil des anfallenden staubigen Feinanteils des Mais, z. B. 10 bis 100 Gew.-%, der Maiskleie zuzugeben.

In einem Verfahren zur fermentativen Herstellung von Lysin fällt beispielsweise ein sirupartiger Nebenstoffstrom CMS an, mit einem Trockenmasseanteil von 40 bis 90 Gew.-%, der mit der Maiskleie vermischt bzw. zusammengebracht werden kann, z. B. mittels Aufsprühung und dann gemeinsam getrocknet werden kann. Nach der Trocknung kann optional eine Zerkleinerung der eventuell entstandenen Agglomerate erfolgen. Die Zusammensetzung (bezogen auf die Trockenmasse) des auf diese Weise erhaltenen Nebenproduktes ist in der Regel wie folgt:

| | |
|---|---|
| Rohprotein: | 5 bis 60 Gew.-%, bevorzugt 10 bis 50 Gew.-% |
| Stärke: | 1 bis 50 Gew.-%, bevorzugt 5 bis 40 Gew.-% |
| Rohfasern: | 1 bis 20 Gew.-%, bevorzugt 2 bis 10 Gew.-% |
| Rohfett: | 1 bis 20 Gew.-%, bevorzugt 1 bis 10 Gew% |
| Rohasche: | 0 bis 15 Gew.-%, bevorzugt 0,1 bis 7 Gew.-% |
| Lysin: | 0 bis 10 Gew.-%, bevorzugt 0 bis 5 Gew.-% |

Ein Nebenprodukt A wird hergestellt, in dem jeweils 0 bis 100 Gew.-%, vorzugsweise 30 bis Ne-100 Gew.-%, besonders bevorzugt die Gesamtmenge des anfallenden Maiskeimlings, 10 bis 100 Gew.-%, vorzugsweise 30 bis 100 Gew.-%, besonders bevorzugt die Gesamtmenge des anfallenden Maisglutens sowie 10 bis 100 Gew.-%, vorzugsweise 30 bis 100 Gew.-%, besonders bevorzugt die Gesamtmenge der anfallenden Biomasse miteinander vermischt werden. Optional kann dieses Nebenprodukt einen Anteil von 0 bis 100 % der anfallenden Maiskleie und 0 bis 100 % des Feinanteils enthalten.

Zur Herstellung dieses Nebenproduktes A sind folgende Verfahrensvarianten möglich.

In einer ersten Variante werden alle Ströme (Maiskeimling, Maisgluten, Biomasse und optional Maiskleie und

/oder Feinanteil) gemischt und getrocknet. Gegebenenfalls kann das trockene Nebenprodukt oder die trockenen Einsatzstoffe Maiskeimling und Maiskleie auch noch vermahlen werden, so dass eine mittlere Partikelgröße und eine Restfeuchte, wie oben beschrieben, eingestellt werden können. In einer zweiten Variante werden nur die feuchten Ströme des Maisglutens und der Biomasse zunächst vermischt und dann gemeinsam getrocknet. Dies hat den Vorteil, dass der bereits trockene Maiskeimling und optional auch die trockene Maiskleie nicht unnötiger Weise durch den Trockner gefahren werden müssen. Nach der Trocknung der Komponenten, können entweder alle Ströme direkt vermischt werden oder zunächst die Einzelströme gemahlen und dann gemischt werden. Nach dem Mischen kann sich wiederum eine Vermahlung anschließen. Es kann eine mittlere Partikelgröße und eine Restfeuchte, wie oben beschrieben, eingestellt werden. In einer dritten Variante werden die beiden feuchten Ströme der Biomasse und des Maisglutens zunächst getrennt getrocknet. Dies kann den Vorteil haben, dass ungewünschte Zersetzungsreaktionen, wie z. B. eine Maillardreaktion zwischen Zucker- und Proteinkomponenten, die in den Strömen enthalten sein können, vermieden oder reduziert werden. Die trockenen Ströme des Maisglutens, der Biomasse, des Maiskeimlings und optional der Maiskleie können optional gemahlen und gemischt werden, oder es kann sich optional eine Mahlung an die Mischung anschließen. Es kann eine mittlere Partikelgröße und eine Restfeuchte, wie oben beschrieben, eingestellt werden. In einer vierten Variante wird wenigstens einem zu trocknenden Strom ein Anteil von 10 bis 100 % wenigstens eines anfallenden festen Stroms während oder vor der Trocknung zugeführt. Dies hat den Vorteil, dass gewünschte Agglomerate gebildet werden können, sich das Fließverhalten des Produktes verbessert oder die Staubneigung des Produktes reduziert wird. So kann zum Beispiel das feucht anfallende Maisgluten (oder Anteile davon) mit Anteilen von Maiskleie (optional vermahlen), mit Anteilen von Maiskeimling (optional vermahlen) oder Anteilen an Feinanteil oder beliebige Kombinationen daraus vor oder während dem Trocknen vermischt werden. Ebenfalls besteht die Möglichkeit, die feucht anfallende Biomasse (oder Anteile davon) mit Anteilen von Maiskleie (optional vermahlen), mit Anteilen von Maiskeimling (optional vermahlen) oder Anteilen an Feinanteil oder beliebige Kombinationen daraus vor oder während dem Trocknen zu vermischen.

Bei der Herstellung des Nebenprodukts A wird Biomasse aus der Lysinfermentation verwendet. Die Ströme Maisgluten, Maiskeimling und Biomasse werden in einer Menge von jeweils 50 bis 100 Gew.-%, bezogen auf die Gesamtmenge des jeweils anfallenden Stroms, verwendet und zu einem Nebenprodukt nach den oben beschriebenen Verfahren verarbeitet. Die bevorzugte Zusammensetzung (bezogen auf die Trockenmasse) des Nebenproduktes ist wie folgt charakterisiert:

| | |
|---|---|
| Rohprotein: | 10 bis 60 Gew.-%, besonders bevorzugt 20 bis 50 Gew.-% |
| Gesamtzucker: | 0,1 bis 50 Gew.-%, besonders bevorzugt 5 bis 45 Gew.-% |
| Rohfasern: | 0 bis 10 Gew.-%, besonders bevorzugt 0 bis 7 Gew.-% |
| Rohfett: | 1 bis 30 Gew.-%, besonders bevorzugt 5 bis 20 Gew.-% |
| Rohasche: | 0 bis 15 Gew.-%, besonders bevorzugt 0,1 bis 7 Gew.-% |
| Lysin: | 0,1 bis 20 Gew.-%, besonders bevorzugt 0,2 bis 10 Gew.-% |

Bei der Herstellung des Nebenprodukts A werden die Biomassen unterschiedlicher Fermentationen gemischt. So können die verschiedenen Biomassen auch wieder zunächst getrennt voneinander getrocknet werden oder gemischt und dann gemeinsam getrocknet werden. Die Biomassen können in einem beliebigen Mischungsverhältnis miteinander gemischt werden. Bevorzugt werden 30 bis 100 % und stärker bevorzugt 50 bis 100 %, der anfallenden Biomasse einer jeweiligen Fermentation hier miteinander gemischt.

Es wird mindestens eine Biomasse eines weiteren Fermentationsprozesses einem beliebigen (oben beschriebenen) Nebenprodukt an beliebiger Stelle des Herstellungsverfahrens zugegeben. Es handelt um ein Nebenprodukt, das sowohl Biomasse einer Lysinfermentation (wie oben beschrieben) und Biomasse einer $B_2$-Fermentation (BFR, wie oben definiert) enthält. Bevorzugt werden 30 bis 100 % und stärker bevorzugt 50 bis 100 %, der anfallenden Biomasse einer jeweiligen Fermentation hier miteinander gemischt. Gegebenenfalls enthält das Nebenprodukt Anteile von 50 bis 100 % des anfallenden Maiskeimlings und/oder 50 bis 100 % des anfallenden Maisglutens und/oder 50 bis 100 % des anfallenden Maiskleie sowie 0 bis 100 % des anfallenden Feinanteils.

Es handelt sich um ein Nebenprodukt, das sowohl Biomasse aus einer Chemikalien-Fermentation, wie z. B. einer Lysinfermentation oder einer Glutamatfermentation, als auch Biomasse aus einer Bioethanolfermentation enthält.

Beim Vermischen der wenigstens zwei Biomassen handelt es sich um Biomassen aus Fermentationen, die jeweils mit einem aus der erfindungsgemäßen Maisstärkeverzuckerung gewonnen Glukosestrom betrieben werden. Hierbei kann man so vorgehen, dass es sich bei beiden Fermentationen um den gleichen Glukosestrom handelt. Weitu hin werden jeweils die aus erfindungsgemäßen Verfahren gewonnenen Glukoseströme eingesetzt, jedoch handelt es sich um separat hergestellte Glukoseströme mit in der Regel unterschiedliche Reinheiten der Glucose. Die mindestens 2 Glucosemedien unterscheiden sich dabei typischerweise in der Konzentration der nicht-stärkehaltigen, festen Komponenten. Bezogen auf die Trockenmasse, entstehen mindestens ein Strom mit einem hohen und ein Strom mit einem geringen Anteil an nicht-stärkehaltigen, festen Komponenten. Die unterschiedlichen Reinheiten der Glucoseströme

können mittels Verfahren wie Dekantierung, Separation, Zentrifugation, Sedimentation, Filtration oder Membranverfahren erzeugt werden. Dabei sind auch mehrstufige Verschaltungen eines Verfahrens oder Kombinationen unterschiedlicher Verfahren denkbar, wie z. B. Dekantierung und Separation.

Die wenigstens zwei Fermentationen können aber auch auf unterschiedlichen C-Quellen beruhen, wobei wenigstens eine C-Quelle eine Glukose ist, die nach dem erfindungsgemäßen Verfahren erhältlich ist.

Ein Nebenprodukt, das mindestens die Biomasse aus zwei verschiedenen Fermentationen enthält, kann auch mindestens 2 verschiedene Stoffwechselprodukte enthalten.

Analog zu dem oben beschriebenen Nebenprodukt A, enthaltend Maisgluten, Maiskeimling und Biomasse (optional Maiskleie) und dem dazugehörigen Herstellungsverfahren, können auch Nebenprodukte hergestellt werden, die als Trockenbestandteile nur Maisgluten und Biomasse (optional Maiskleie und/oder Formulierungshilfsmittel) oder nur Maiskeimling und Biomasse (optional Maiskleie und/oder Formulierungshilfsmittel) oder nur Maisgluten und Maiskeimling (optional Maiskleie und/oder Formulierungshilfsmittel) enthalten. Die möglichen Herstellungsverfahren sind analog den oben genannten.

Alle Nebenprodukte können des weiteren Formulierungshilfsstoffe, Ballaststoffe, Füllstoffe oder weitere Wirkstoffe enthalten, die zu einem beliebigen Prozessschritt der Herstellung zugegeben werden.

Durch Zugabe von Formulierungshilfsmitteln wie Träger- und Coatingmaterialien, Bindemitteln sowie anderer Additive können die Eigenschaften des Nebenproduktes gezielt hinsichtlich verschiedener Parameter wie Korngröße, Partikelform, Neigung zum Stauben, Hygroskopizität, Stabilität, insbesondere Lagerstabilität, Farbe, Geruch, Fließverhalten, Agglomerationsneigung, elektrostatischer Aufladung, Licht- und Temperaturempfindlichkeit, mechanischer Stabilität und Redispergierbarkeit in an sich bekannter Weise konfektioniert werden.

Zu den üblicherweise verwendeten Formulierungshilfsmitteln gehören z. B. Bindemittel, Trägermaterialien, Puderungs-/Fließhilfsmittel, ferner Farbpigmente, Biozide, Dispergiermittel, Antischaummittel, viskositätsregulierende Mittel, Säuren, Laugen, Antioxidantien, Enzymstabilisatoren, Enzyminhibitoren, Adsorbate, Fette, Fettsäuren, Öle oder Mischungen hieraus. Derartige Formulierungshilfsmittel werden vorteilhaft insbesondere bei Anwendung von Formulierungs- und Trocknungsverfahren wie Sprüh-, Wirbelschicht- und Gefriertrocknung als Trocknungshilfsmittel eingesetzt.

Beispiele für Bindemittel sind Kohlenhydrate, insbesondere Zucker wie Mono-, Di-, Oligo- und Polysaccharide, z. B. Dextrine, Trehalose, Glucose, Glucosesirup, Maltose, Saccharose, Fructose und Lactose; kolloidale Substanzen wie tierische Proteine, z. B. Gelatine, Casein, insbesondere Natriumcaseinat, pflanzliche Proteine, z. B. Sojaprotein, Erbsenprotein, Bohnenprotein, Lupin, Zein, Weizenprotein, Maisprotein und Reisprotein, synthetische Polymere, z. B. Polyethylenglykol, Polyvinylalkohol und insbesondere die Kollidon-Marken der Fa. BASF, gegebenenfalls modifizierte Biopolymere, z. B. Lignin, Chitin, Chitosan, Polylactid und modifizierte Stärken, z. B. Octenylsuccinatanhydrid (OSA); Gummen, z. B. Gummi acacia; Cellulosederivate, z. B. Methylcellulose, Ethylcellulose, (Hydroxyethyl)methylcellulose (HEMC), (Hydroxypropyl)methylcellulose (HPMC), Carboxymethylcellulose (CMC); Mehle, z. B. Maismehl, Weizenmehl, Roggenmehl, Gerstenmehl und Reismehl.

Beispiele für Trägermaterialien sowie Ballast- oder Füllstoffe sind Kohlenhydrate, insbesondere die als Bindemittel vorgenannten Zucker sowie Stärken, z. B. aus Mais, Reis, Kartoffel, Weizen und Cassava; modifizierte Stärken, z. B. Octenylsuccinatanhydrid; Cellulose und mikrokristalline Cellulose; anorganische Mineralien oder Lehm, z. B. Ton, Kohle, Kieselgur, Kieselsäure, Talg und Kaolin; Gries, z.B. Weizengries, Kleie, z. B. Weizenkleie, die als Bindemittel vorgenannten Mehle; Salze wie Metallsalze, insbesondere Alkali- und Erdalkalimetallsalze organischer Säuren, z. B. Mg-, Ca-, Zn-, Na-, K-citrat, -acetat, -formiat und hydrogenformiate, anorganische Salze, z. B. Mg-, Ca-, Zn-, Na-, K-sulfate, -carbonate, -silikate oder phosphate; Erdalkalimetalloxide wie CaO und MgO; anorganische Pufferungsmittel wie Alkalimetallhydrogenphosphate, insbesondere Natrium- und Kaliumhydrogenphosphate, z. B. $K_2HPO_4$, $KH_2PO_4$ und $Na_2HPO_4$; sowie allgemein die im Zusammenhang mit der Herstellung von Stoffwechselprodukten mit niedrigem Schmelzpunkt bzw. öliger Konsistenz genannten Adsorbentien. Weitere Füll- oder Ballaststoffe können auch fetthaltige Produkte sein, wie z. B. Sojamehl, Sojaschrot oder Mehle oder Schrote an Mais, Roggen, Weizen, Gerste, Erbsen...

Beispiele für Puderungsmittel bzw. Fließhilfsmittel sind Kieselgur, Kieselsäure, z. B. die Sipernat-Marken der Fa. Degussa; Ton, Tonerde, Sepiolithe, Kenite, Montmorillonite, Zeolithe, Kohle, Talg und Kaolin; die als Trägermaterialien vorgenannten Stärken, modifizierten Stärken, anorganischen Salze, Salze organischer Säuren und Pufferungsmittel; Cellulose und mikrokristalline Cellulose.

Hinsichtlich sonstiger Additive seien als Beispiele genannt Farbpigmente wie $TiO_2$; Biozide; Dispergiermittel; Antischaummittel; viskositätsregulierende Mittel; anorganische Säuren wie Phosphorsäuren, Salpetersäure, Salzsäure, Schwefelsäure; organische Säuren wie gesättigte und ungesättigte Mono- und Dicarbonsäuren, z. B. Ameisensäure, Essigsäure, Propionsäure, Buttersäure, Valeriansäure, Palmitinsäure, Stearinsäure, Oxalsäure, Malonsäure, Bernsteinsäure, Glutarsäure, Adipinsäure, Pimelinsäure, Maleinsäure und Fumarsäure; Laugen wie Alkalimetallhydroxide, z. B. NaOH und KOH; Antioxidantien; Enzymstabilisatoren; Enzyminhibitoren; Adsorbate; Fette; Fettsäuren und Öle.

Der Anteil an den vorgenannten Zusatzstoffen und gegebenenfalls weiteren Zusatzstoffen wie Coatingmaterialien kann je nach den speziellen Erfordernissen des jeweiligen Nebenprodukts sowie in Abhängigkeit von den Eigenschaften der eingesetzten Zusatzstoffe stark variieren und z. B. im Bereich von 0,1 bis 80 Gew.-% bezogen auf das

Gesamtgewicht des fertig formulierten Produkts bzw. Stoffgemischs, liegen.

Die Zugabe von Formulierungshilfsmitteln kann zu einem beliebigen Herstellungsschritt des Nebenproduktes, insbesondere während der gegebenenfalls erforderlichen Trocknung erfolgen. Die Formulierungshilfsmittel können sowohl dem in fester Form erhaltenen Nebenprodukt als auch einer dieses enthaltenden Lösung oder Suspension zugegeben werden. Insbesondere nach der Trocknung erfolgt eine Zugabe von Formulierungshilfsmitteln z. B. beim Aufbringen von Überzügen bzw. Coatings/Coatingschichten auf getrocknete Partikel. Sowohl nach dem Trocknen als auch nach einem eventuellen Coatingschritt können dem Produkt weitere Hilfsmittel zugegeben werden.

Optional können neben dem jeweiligen Stoffwechselprodukt der Fermentation den Nebenprodukten noch weitere Wirkstoffe, vorzugsweise in der Futtermittelindustrie übliche Wirkstoffe an einem beliebigen Schritt des Herstellungsverfahrens zugegeben werden. Unter Wirkstoffen werden hier alle Vitamine, (vorzugsweise A, B1, B2, B5, B6, C, D3 und E), Carotenoide, Enzyme (vorzugsweise Phytase, Xylanase, Glucanase, Amylase, Cellulase, Hemicellulase, Protease, Lipase, Pektinase, Phosphatasen), Probiotika (z. B. Enterococcus ssp., Lactobacillus ssp. Bacillus ssp., Pediococcus ssp.), Antibiotika; organische Säuren und Aminosäuren (Methionin, Lysin, etc.). Die Wirkstoffe werden vorzugsweise einen Anteil von 0,001 bis 20 Gew%, besonders bevorzugt von 0,01 bis 5 Gew% des Nebenproduktes ausmachen (bezogen auf die Trockenmasse).

Das Folgende Beispiel diemt der Erläuterung der Erfindung

Als Einsatzstoff der Versuche zur Maisvermahlung wurde ein Mais der Spezifikation US Yellow No. 2 mit einer Feuchte von 11,9 Gew.-% eingesetzt. Bezogen auf Trockenmasse enthielt dieser Mais 3,8 Gew.-% Rohfett und 75,3 Gew.-% Stärke.

**Beispiel 1: Herstellung einer Glukose-Lösung**

<u>Schritt a): Fraktioniertes Vermahlen von Mais</u>

a.1. Maisvorreinigung

In einem ersten Schritt wurden durch Sieben die Fraktionen A (> 12 mm Partikeldurchmesser; 0,0 Gew.-% des eingesetzten Mais) und Fraktion B (< 6,5 mm Partikeldurchmesser, 4,05 Gew.-% des eingesetzten Mais) von der Hauptmenge des Maisstroms (Fraktion C) abgetrennt. In einem weiteren Schritt wurden leichte Bestandteile durch Sichten entfernt (0,18 Gew.-%), die verworfen wurden. Anschließend wurde Fraktion C auf einem Steinausleser von Steinen befreit, so dass die Fraktion steinfrei war.

Zur Optimierung der Stärkeausbeute des Gesamtprozesses wurde die Fraktion B erneut durch Sieben in eine Fraktion B1 mit Partikeldurchmessern zwischen 6,5 und 4,0 mm (2,95 Gew.-% des eingesetzten Mais), eine Fraktion B2 mit Partikeldurchmessern < 4,0 mm (0,98 % des eingesetzten Mais) sowie durch Sichten in eine Fraktion leichter Bestandteile (0,13 % zum Abfall) aufgetrennt. Die zur Vermahlung gehende Fraktion B1 wurde auf einem Steinausleser von Steinen befreit, wobei ein minimaler Steinanteil abgetrennt wurde.

Basierend auf den eingesetzten Rohmais wurden damit insgesamt 98,7 % des Materials zur Vermahlung gegeben.

Zur besseren Vermahlung wurde der Mais dann durch Zugabe von Wasser auf eine Feuchte von ca. 15 Gew.-% eingestellt. Vor der weiteren Verarbeitung wurde der Mais dann ca. 8 h ruhen gelassen.

a.2. Fraktionierende Vermahlung

Variante 1: Entkeimung auf einem Maisentkeimer

In dieser Verfahrensvariante wurde die Fraktion C (> 6,5 mm) des vorgereinigten Mais auf eine Maisentkeimungsmaschine geführt. Als Maisentkeimungsmaschine diente eine Vorrichtung, die eine Einzugsschnecke und eine Bearbeitungszone, umfassend einen Walzenrotor und ein den Walzenrotor mantelförmig umgebendes strukturiertes Sieb, umfasste. Der zu verarbeitende Mais wurde mittels der Einzugsschnecke in die Bearbeitungszone geführt. Die Entkeimung wurde durch die intensive Bearbeitung zwischen Walzenrotor und Siebmantel sowie durch entsprechende Einstellung des Staudrucks am Auslauf erreicht. Hierbei wurden die Keime von der Schale und der Endospermfraktion getrennt. Nach Durchlauf durch die Maschine werden die entstandenen Fraktionen durch Sieben und Sichten getrennt. Beim Sieben wurde das sog. Schälmehl als kleinste Fraktion abgetrennt. Durch Sichten wurden die freigesetzten Schalen (Kleiefraktion) abgetrennt. Da die Trennung von Endosperm, Schale und Keim innerhalb der Maisentkeimungsmaschine unvollständig war, wurde die durch Sieben und Sichten nicht abgetrennte Fraktion in eine dreistufige Walzenstuhlpassage gegeben. Die in der Vorreinigung erhaltene Maisfraktion B1 (Partikeldurchmesser zwischen 6,5 - 4 mm) wurde ebenfalls direkt in die mehrstufige Walzenstuhlpassage gegeben. Beim Durchgang durch den Walzenstuhl wurden die zugegebenen Partikel beim Durchgang durch zwei mit unterschiedlicher Geschwindigkeit rotierenden Rollen zerkleinert. Nach

jeder Passage wurden dabei durch Sieben und Sichten die freigesetzten Schalen und Keime von einer ausreichende zerkleinerten Endospermfraktion sowie einer Endospermfraktion mit ggf. anhaftenden Keim- und Schalenbestandteilen getrennt. Zur weiteren Abtrennung von Keim- und Schalenbestandteilen wurde diese Endospermfraktion der nächsten Walzenstuhlpassage zugeführt.

Durch Zusammenfassung der Endospermfraktionen (84 Gew.-% des zur Vermahlung eingesetzten Mais) wurde ein Mehl mit einem Stärkegehalt von 84,4 Gew.-% und einem Fettgehalt von 1,28 Gew.-% erhalten. Die entstandene Keimfraktion (12,3 Gew.-% des eingesetzten Mais) hatte einen Stärkegehalt von 24,1 Gew.-% und einen Fettgehalt von 20,8 Gew.-%. Die Schalenfraktion (3,6 Gew.-% des eingesetzten Mais) hatte einen Stärkegehalt von 24,4 Gew.-% und einen Fettgehalt von 1,9 Gew.-%.

Variante 2: Entkeimung auf Walzenstühlen

In einer weiteren Verfahrensvariante werden die Fraktionen C (> 6,5 mm) und B1 (6,5 bis 4,0 mm) des vorgereinigten Mais direkt auf einen Walzenstuhl mit 2 Walzenpaaren mit je zwei gegenläufig, mit unterschiedlicher Geschwindigkeit rotierenden Walzen geführt. Auf diesem Walzenstuhl wurden die Maiskörner durch zwei unterschiedlich schnell rotierende Rollen zerkleinert und teilweise die Schalen, das Endosperm und die Maiskeime durch Scherung getrennt. Nach diesem ersten Aufschluss folgten drei weitere Walzenstuhlpassagen, wobei erneut die zugegebenen Partikel beim Durchgang durch zwei mit unterschiedlicher Geschwindigkeit rotierenden Rollen zerkleinert wurden. Nach jeder Passage wurden dabei durch Sieben und Sichten die freigesetzten Schalen und Keime von einer ausreichende zerkleinerten Endospermfraktion sowie einer Endospermfraktion mit ggf. anhaftenden Keim- und Schalenbestandteilen getrennt. Zur weiteren Abtrennung von Keim- und Schalenbestandteilen wurde diese Endospermfraktion der nächsten Walzenstuhlpassage zugeführt.

Durch Zusammenfassung der Endospermfraktionen (85,3 Gew.-% des zur Vermahlung eingesetzten Mais) wurde ein Mehl mit einem Stärkegehalt von 84,6 Gew.-% und einem Fettgehalt von 1,74 Gew.-% erhalten. Die entstandene Keimfraktion (11 Gew.-% des eingesetzten Mais) hatte einen Stärkegehalt von 25,5 Gew.-% und einen Fettgehalt von 19,0 Gew.-%. Die Schalenfraktion (= Kleiefraktion, 3,7 % des eingesetzten Mais) hatte einen Stärkegehalt von 24,3 Gew.-% und einen Fettgehalt von 2,7 Gew.-%.

a.3. Größenreduktion

Zur Größenreduktion wurden die drei entstandenen Fraktionen (Endosperm, Keim und Schale) jeweils separat vermahlen.

Die Vermahlung der Endospermfraktion wurde auf einem Walzenstuhl durchgeführt. Dabei wurde ein Weizenmehl folgender Größenverteilung erhalten.

| Partikelgröße [μm] | > 905 | > 410 | > 310 | > 200 | > 132 | < 132 | Summe |
|---|---|---|---|---|---|---|---|
| Massenanteil [%] | 0,1 | 1,9 | 11,6 | 41,3 | 23,5 | 21,6 | 100,0 |

Die Vermahlung der Keimfraktion erfolgte auf einer Hammermühle mit einem Siebdurchmesser von 3 mm. Durch die Vermahlung stellte sich folgende Größenverteilung ein:

| Partikelgröße [μm] | > 2300 | > 1610 | > 1200 | > 700 | < 700 | Summe |
|---|---|---|---|---|---|---|
| Massenanteil [%] | 0,10 | 1,20 | 4,99 | 27,42 | 66,30 | 100,00 |

Die Vermahlung der Schalenfraktion erfolgte ebenfalls auf einer Hammermühle mit einem Siebdurchmesser von 3 mm. Daraus ergab sich die folgende Größenverteilung:

| Partikelgröße [μm] | > 2300 | > 1610 | > 1200 | > 700 | < 700 | Summe |
|---|---|---|---|---|---|---|
| Massenanteil [%] | 0,00 | 0,20 | 1,80 | 24,65 | 73,35 | 100,00 |

Schritt b): Enzymatisches Verflüssigen und Verzuckern von Maismehl

Allgemeine Vorschrift b1:

Zur Durchführung der Versuche wurde eine Kombination von kontinuierlich und diskontinuierlich betriebenen Reaktoren eingesetzt. Zunächst wurde das Maismehl angemaischt. Hierzu legte man in 2 Rührkesseln zu je 250 L Wasser und Maismehl vor und erhitzte mit Direktdampf auf 60°C. Entsprechend der gewählten Menge Maismehl wurde dann $CaCl_2$ (0,006 Gew.-% bezogen auf die eingesetzte Menge Mehl (Trockensubstanz)) zugegeben. Im nächsten Schritt wurde der pH-Wert mit 10 Gew.-%iger Schwefelsäure auf pH 5,5 - 5,8 eingestellt und man gab $\alpha$-Amylase (Liquozyme Supra, Novozyme A/S, 0,04 % bezogen auf die eingesetzte Menge Mehl (TS)) zu. Die derart hergestellte Maische wurde mittels einer Exzenter-Schneckenpumpe durch einen Jet Cooker (Hydroheater M101, Hydro-Thermal Corp.) gepumpt, in dem die Maische durch Direktdampf auf 109°C aufgeheizt wurde. Hierdurch wurde die im Maismehl enthaltene Stärke verkleistert und die eingesetzte $\alpha$-Amylase führte zu einer Spaltung der Stärkemoleküle. Der den Jet Cooker verlassende Strom wurde in einen Rohrreaktor, der eine Temperatur von 109°C aufwies mit 5 min Verweilzeit geleitet. Die den Rohrreaktor verlassende Reaktionsmischung wurde in einen 30 L Behälter gegen Umgebungsdruck entspannt, wodurch sich Temperaturen von 95-99 °C einstellten. Unter diesen Bedingungen wurde die Reaktionsmischung dann in einen zweiten Rohrreaktor mit 120 min Verweilzeit gepumpt. Aus diesem zweiten Rohrreaktor wurde die verflüssigte Mischung anschließend wahlweise in einen 250 L oder einen 2000 L Rührkessel gepumpt.

In den Rührkesseln wurde jeweils diskontinuierlich eine enzymatische Spaltung der in der Verflüssigung durch Spaltung der Stärkemolekülen gebildeten Dextrine in Glukose durchgeführt. Hierzu wurde in einem ersten Schritt die Temperatur der verflüssigten Mischung auf 63°C gesenkt, der pH-Wert mit 10 %iger Schwefelsäure auf 4,3 ($\pm$ 0,1) eingestellt und dann Glukoamylase (Dextrozyme DX 1.5X, Novozyme A/S, 0,06 % bezogen auf die eingesetzte Menge Mehl (TS)) zugegeben. Nach Zugabe der Glukoamylase wurde die Reaktionsmischung dann über 48 h bei 63 - 65°C gehalten, bevor durch Erhöhen der Temperatur auf > 70 °C die Spaltung der Dextrine in Glukose durch Denaturierung der Glukoamylase gestoppt wurde.

Es wurden verschiedene analog Schritt a) erzeugte Maismehle verflüssigt und verzuckert. Diese Mehle wiesen folgende Zusammensetzungen auf:

| | Restfeuchte | Stärke* | Rohprotein* | Rohfett* | Rohasche* | Rohfaser* |
|---|---|---|---|---|---|---|
| | [%] | [%] | [%] | [%] | [%] | [%] |
| Mehl 1 | 9,32 | 83,2 | 8,3 | 1,7 | 0,7 | 1,4 |
| Mehl 2 | 9,44 | 83,1 | 7,7 | 1,5 | 0,6 | 1,3 |
| Mehl 3 | 11,46 | 85,8 | 7,6 | 1,7 | 0,3 | 0,7 |
| * Massenanteil bezogen auf Trockensubstanzgehalt | | | | | | |

Die Mehle wiesen folgende Partikelgrößenverteilung auf:

| | Partikelgröße [$\mu$m] | | | | | |
|---|---|---|---|---|---|---|
| Massenanteil* | > 850 | > 600 | > 425 | > 300 | > 250 | < 250 |
| Mehl 1 | - | - | 2 | 30 | 10 | 58 |
| Mehl 2 | - | - | 2 | 50 | 6 | 42 |
| Mehl 3 | - | - | 2 | 22 | 11 | 64 |
| * Massenanteil bezogen auf Trockensubstanzgehalt | | | | | | |

Alle Mehle wurden nach der allgemeinen Vorschrift b1) angemaischt und verflüssigt, wobei jeweils das Verhältnis von Mehl und Wasser so gewählt wurde, dass jeweils ein Stärkegehalt von 31,0 Gew.-% bei der Verflüssigung und Verzuckerung resultierte. Entsprechend der unterschiedlichen Stärkegehalte der einzelnen Mehle wurden damit Trockensubstanzgehalte von 37,3 Gew.-% (Mehl 1, Mehl 2) und 36,1 Gew.-% (Mehl 3) zur Verflüssigung und Verzukkerung eingesetzt. Nach 48 h wurde durch dieses Vorgehen eine Zuckerlösung (Rohglukose) mit Zuckern unterschiedlicher Kettenlängen gebildet. Die so erhaltenen Rohglukosen wiesen eine Glukosekonzentration (DP1) von 29,1 - 29,6 Gew.-% auf. Die Anteile der Glukose (DP1) und Oligoglukosen (DP2 bis DP4) in den erhaltenen Rohglukosen sind in der folgenden Tabelle zusammengestellt:

| Polymerisationsgrad | Mehl 1 | Mehl 2 | Mehl 3 |
|---|---|---|---|
| DP 1 [%] | 94,5 | 94,7 | 95,5 |
| DP 2 [%] | 2,9 | 2,9 | 2,6 |
| DP 3 [%] | 1,5 | 1,5 | 1,0 |
| DP 4 [%] | 0,9 | 0,7 | 0,8 |
| > DP 4 [%] | 0,3 | 0,2 | 0,2 |

In einem weiteren Versuch wurde Mehl 1 mit einem Stärkegehalt von 34,7 Gew.-% zur Verflüssigung und Verzuckerung eingesetzt. Damit ergab sich ein Trockensubstanzgehalt von 41,7 Gew.-% in der Maische. In diesem Experiment wurde die Menge Glukoamylase auf 0,06 % (bezogen auf die eingesetzte Menge Mehl (TS)) reduziert. Nach 48 h wurde bei diesem Vorgehen eine Rohglukose mit einer Glukosekonzentration von 32,7 Gew% erhalten. 94,2 % der erzeugten Zucker hatten einen Polymerisationsgrad von 1.

Allgemeine Vorschrift b2:

Als Alternative zu dem unter b1) beschriebenen diskontinuierlichen Anmaischen des Maismehls in Rührkesseln wurde das Anmaischen des Mehles in einem kontinuierlichen Mischer (CoriMix K-TT, Lödige-Drais) durchgeführt. Hierzu wurde in dem zur Verzuckerung vorgesehenen Rührkessel mit 2500 L Volumen insgesamt 693 L Wasser, auf eine Temperatur von 58,1°C erwärmt und 69 g $Ca(OH)_2$ sowie 106 g Liquozyme zugegeben. Das zugemischte Maismehl (11,4 Gew.-% Restfeuchte) hatte eine Temperatur von 31°C. In einem ersten Betriebspunkt wurden 109,2 kg/h des Wassers zu 82,8 kg/h Maismehl gefahren, wodurch insgesamt 192 kg/h einer homogenen Maismehlsuspension mit 33,9 Gew.-% Stärkegehalt und 38,2 Gew.-% Trockensubstanzgehalt erzeugt wurde. Die Temperatur der Mischung betrug 42°C. In einem zweiten Betriebspunkt wurden sowohl die zugeführten Mengen Wasser als auch Mehl erhöht. Aus 217,6 kg/h Maismehl und 258,2 kg/h Wasser wurde im Mischer eine homogene Maismehlsuspension von insgesamt 475,8 kg/h mit 35,8 Gew.-% Stärke- und 40,5 Gew.-% Trockensubstanzgehalt erzeugt. Die Temperatur am zweiten Betriebspunkt betrug ebenfalls 42°C.

Die so erhaltene Maismehlsuspension wurde analog zu der in der allgemeinen Vorschrift b1 angegebenen Weise in einer Anordnung aus Jetkocher und 2 sequentiell geschalteten Rohrreaktoren verflüssigt und anschließend diskontinuierlich verzuckert.

<u>Schritt c): Abreichern der nicht-hydrolysierten Feststoffe aus der Rohglukose (Maisgluten und ggf. Kleiebestandteile)</u>

Die Abtrennung der nicht-hydrolysierten Feststoffe aus der in Schritt b) erhaltenen Rohglukose wurde auf einem Dekanter (Typ Z23-4/401 s, Fa. Flottweg) durchgeführt. Eine Übersicht über die einzelnen Verfahrensschritte gibt das folgende Schema 1.

Schema 1:

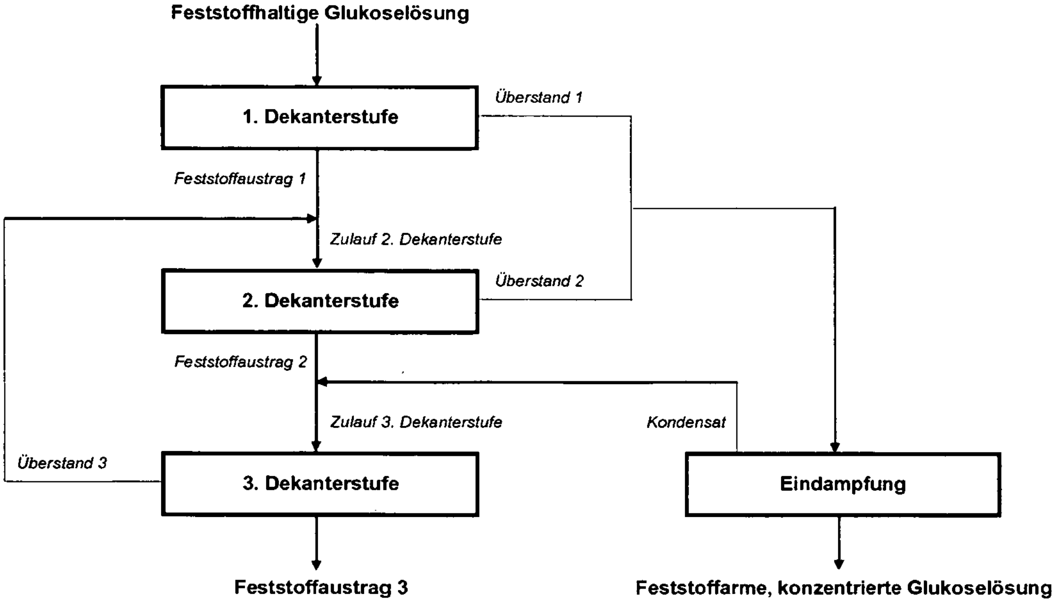


Nach dem in Schritt b) beschriebenen Verfahren wurde aus Mehl 2 eine Glukoselösung hergestellt, die bei 28,6 Gew.-% Glukosegehalt und 0,8 Gew.-% Disaccharidgehalt einen Gesamt-Trockensubstanzgehalt von 36,1 Gew.-% enthielt. Die spezifische Dichte der Glukoselösung betrug 1,15 g/cm$^3$.

Entsprechend Schema 1 wurden 440 kg dieser feststoffhaltige Glukoselösung mit einem Fluss von 440 kg/h auf die erste Dekanterstufe gegeben und in zwei Fraktionen (Überstand 1 und Feststoffaustrag 1) getrennt. Auf diese Weise erhielt man 326 kg Überstand (Überstand 1) mit einem Glukosegehalt von 30,3 Gew.-% und einen Disaccharidgehalt von 0,9 Gew.-%, einem Gesamt-Trockensubstanzgehalt von 33,1 Gew.-% und einer Dichte des Überstandes von 1,15 g/cm$^3$. Der Feststoffaustrag der ersten Dekanterstufe (Feststoffaustrag 1) von 114 kg hatte ein Glukosegehalt von 23,6 Gew.-% und einen Disaccharidgehalt von 0,6 Gew.-%. Der Gesamt-Trockensubstanzgehalt des Feststoffaustrags 1 betrug 44,6 Gew.-%.

Im nächsten Schritt wurde der Feststoffaustrag 1 mit 154 kg vom Überstand der dritten Dekanterstufe (Überstand 3) resuspendiert, wodurch man 268 kg einer feststoffhaltigen Glukoselösung mit einem Glukosegehalt von 11,9 Gew.-% und einem Disaccharidgehalt von 0,4 Gew.-% erhielt. Der Gesamt-Trockensubstanzgehalt dieser Lösung betrug 23,2 Gew.-%. Diese feststoffhaltige Glukoselösung wurde dann mit einem Fluss von 470 kg/h auf die zweite Dekanterstufe gegeben und wieder in zwei Fraktionen getrennt (Überstand 2 und Feststoffaustrag 2). Auf diese Weise erhielt man 169 kg Überstand 2 mit einem Glukosegehalt von 13,2 Gew.-%, einen Disaccharidgehalt von 0,4 Gew.-%, einem Gesamt-Trockensubstanzgehalt von 14,1 Gew.-% und einer Dichte von 1,07 g/cm$^3$. Der Feststoffaustrag 2 fiel in einer Menge von 99 kg an und hatte ein Glukosegehalt von 9,2 Gew.-% und einen Disaccharidgehalt von 0,2 Gew.-%. Der Gesamt-Trockensubstanzgehalt des Feststoffaustrags 2 betrug 38,6 Gew.-%.

Im nächsten Schritt wurde der Feststoffaustrag 2 dann mit 154 kg Kondensat der Glukoseeindampfung resuspendiert, wobei man 253 kg einer feststoffhaltigen Glukoselösung mit einem Glukosegehalt von 3,8 Gew.-% und einem Disaccharidgehalt von 0,2 Gew.-% erhielt. Der Gesamt-Trockensubstanzgehalt dieser Lösung betrug 16,1 Gew.-%. Diese feststoffhaltige Glukoselösung wurde dann mit einem Fluss von 670 kg/h auf die dritte Dekanterstufe gegeben und erneut in zwei Fraktionen getrennt (Überstand 3 und Feststoffaustrag 3). Es wurden 144 kg Überstand 3 mit einem Glukosegehalt von 4,5 Gew.-% und einen Disaccharidgehalt von 0,1 Gew.-% erhalten. Bei einem Gesamt-Trockensubstanzgehalt von 4,4 Gew.-% betrug die Dichte des Überstandes 3 1,03 g/cm$^3$. Der Feststoffaustrag 3 fiel in einer Menge von 109 kg an, und hatte ein Glukosegehalt von 3,1 Gew.-% und einen Disaccharidgehalt von 0,1 Gew.-%. Der Gesamt-Trockensubstanzgehalt dieses Feststoffaustrags 3 betrug 31,6 Gew.-%.

Die Überstand der ersten beiden Dekanterstufen (Überstand 1 und Überstand 2) wurden zusammengefasst. Auf diese Weise erhielt man 494 kg einer feststoffarmen Glukose, die einen volumetrischen Feststoffanteil von 1,0 Vol% aufwies, bestimmt durch Zentrifugation bei 1650 g. Die Mischung hatte einen Glukosegehalt von 24,4 Gew.-% und einen Disaccharidgehalt von 0,7 Gew.-%. Bei einem Gesamt-Trockensubstanzgehalt von 26,6 Gew.-% betrug die Dichte der Mischung 1,12 g/cm$^3$.

Die so erzeugte Glukoselösung wurde in einem 800 L doppelwandigen Rührbehälter eingedampft. Hierzu wurde der Rührbehälter mit 140°C heißem Dampf beaufschlagt. Die Temperatur der Glukoselösung wurde durch Einstellen eines leichten Vakuums auf 95°C gehalten.

Am Ende der Eindampfung verblieben 202 kg Glukoselösung im Rührbehälter. Diese Lösung wies einen Glukosegehalt von 60,5 Gew.-% und einen Disaccharidgehalt von 1,6 Gew.-% auf. Der Gesamt-Trockensubstanzgehalt der Lösung betrug 65,0 Gew.-%. Der Rohproteingehalt betrug 1,9 Gew.-%, der Rohfaser- und der Rohaschegehalt 0,01 Gew.-%.

Die entstandene Glukoselösung enthielt etwa 580 mg/kg Protein bzw. Aminosäuren mit folgender Aminosäureverteilung: 119 mg/kg Aspartat, 7 mg/kg Threonin, 15 mg/kg Serin, 55 mg/kg Glutamin, 16 mg/kg Glycin, 64 mg/kg Alanin, 5 mg/kg Cystein, 15 mg/kg Valin, 3 mg/kg Methionin, 11 mg/kg Isoleucin, 9 mg/kg Leucin, 33 mg/kg Tyrosin, 17 mg/kg Phenylalanin, 5 mg/kg Histidin, 10 mg/kg Lysin, 18 mg/kg Arginin und 190 mg/kg Prolin. Der pH-Wert der Lösung lag bei pH 4,4. Die Lösung enthielt 0,12 Gew.-% $SO_4^{2-}$, 19 mg/kg $Cl^-$, 0,17 Gew.-% $K^+$, 0,01 Gew.-% $Ca^{2+}$, 42 mg/kg $Na^+$ und 0,12 Gew.-% $PO_4^{3-}$. Die Viskosität der Lösung betrug bei 30°C 84 cP.

Nicht-erfindungs gemäß Beispiel 2: Herstellung eines Maisglutenpulvers durch Trocknung der in Beispiel 1, Schritt c) erhaltenen Feststofffraktion

Zur Herstellung des Maisglutenpulvers wurden der in Beispiel 1, Schritt c) abgetrennte Feststoff (Feststoffaustrag 3) in einem Multicoil-Pilottrockner (Fa. NLI) getrocknet. Dieser Trockner mit 300 L Volumen wies 3 rotierende Heizschlangen mit insgesamt 3 $m^2$ Oberfläche auf. Zum Betrieb des Trockners wurde das zu trocknende Material eingefüllt, anschließend der Druck im Trockner auf 600 mbar eingestellt und der Trockner durch 6 bar Dampf in den Heizschlangen beheizt. Dazu drehte sich der Trockner mit 13 Umdrehungen pro Minute. Zu Beginn der Versuche wurden 10 kg vorgetrocknetes Material aus einer vorangegangenen Feststoffabtrennung vorgelegt, um Anbackungen auf den Heizschlangen zu vermeiden. Nach Zugabe von 10 kg des feuchten Feststoffes mit 31,6 Gew.-% Trockensubstanzgehalt (Feststoffaustrag 3 aus Schritt c) von Beispiel 1) wurde dann für 45 min getrocknet. In weiteren Intervallen wurde dann jeweils weiterer feuchter Feststoff (Feststoffaustrag 3 aus Schritt c) von Beispiel 1) zugegeben, getrocknet und im späteren Trocknungsverlauf jeweils die Restfeuchte bestimmt.

| Zeit [min] | 0 | 45 | 70 | 105 | 120 | 145 | 165 | 200 | 220 | 250 | 290 |
|---|---|---|---|---|---|---|---|---|---|---|---|
| $Feststoff_{trocken}$ [kg] | 10 | - | - | - | - | - | - | - | - | - | - |
| $Feststoff_{feucht}$ [kg] | 10 | 10 | 20 | 6 | 18 | 16 | 18 | 22 | 40 | 40 | - |
| Restfeuchte [Gew.-%] | - | - | - | - | 24,0 | 22,8 | 25,6 | 10,4 | 15,3 | 19,2 | 10,1 |

Das auf diese Weise hergestellte Trockenprodukt hatte eine mittlere Partikelgröße von 369 μm und eine Schüttdichte von 531 g/L. Das Trockenprodukt bestand aus 36,8 Gew.-% Rohprotein, 20,1 Gew.-% Zucker, 7,0 Gew.-% Rohfett und 4,5 Gew.-% Rohfaser.

Nicht - erfindungsgemäßes Beispiel 3: Verwendung der erzeugten Glukoselösung in einer Fermentation

Eine gemäß Beispiel 1 hergestellte Glukoselösung wurde in Fermentationen mit Corynebacterium glutamicum zur Herstellung von Lysin eingesetzt.

<u>3.1 Konstruktion eines Lysin überproduzierenden C. glutamicum Stamms ATCC13032lysC$^{fbr}$</u>

3.1.1 Konstruktion des Plasmids pCIS lysC

Im ersten Schritt der Stammkonstruktion wurde ein allelischer Austausch des für das Enzym Aspartatkinase kodierenden Wildtypgens (lysC) in C. glutamicum ATCC13032 durchgeführt. Dabei wurde im lysC Gen ein Nukleotidaustausch vorgenommen, so dass im resultierenden Protein die Aminosäure Thr an der Position 311 durch ein Ile ausgetauscht wurde. Ausgehend von der chromosomalen DNA aus ATCC13032 als Template für eine PCR Reaktion wurde mit den Oligonukleotidprimern
5'-GAGAGAGAGACGCGTCCCAGTGGCTGAGACGCATC -3' (SEQ ID NO:1)
und
5'-CTCTCTCTGTCGACGAATTCAATCTTACGGCCTG-3'(SEQ ID NO:2)
lysC mit Hilfe des Pfu-Turbo PCR Systems (Stratagene, USA) nach Angaben des Herstellers amplifiziert. Chromosomale DNA aus C. glutamicum ATCC 13032 wurde nach Tauch et al. (1995) Plasmid 33:168-179 oder Eikmanns et al. (1994) Microbiology 140:1817-1828 präpariert. Das amplifizierte Fragment wird an seinem 5'-Ende von einer SalI Restriktionsschnittstelle und an seinem 3'-Ende von einer MluI Restriktionsschnittstelle flankiert. Vor der Klonierung wurde das

amplifizierte Fragment durch diese beiden Restriktionsenzyme verdaut und mit GFX™PCR, DNA and Gel Band Purification Kit (Amersham Pharmacia, Freiburg) aufgereinigt.

Das erhaltene Polynukleotid wurde über die Sall und Mlul Restriktionsschnitte in pCLIK5 MCS integrativ SacB, im folgenden pCIS genannt, (SEQ ID NO: 3) kloniert und in E.coli XL-1 blue transformiert. Eine Selektion auf Plasmid tragende Zellen wurde durch das Ausplattieren auf Kanamycin (20μg/ml) haltigem LB Agar (Lennox, 1955, Virology, 1: 190) erreicht. Das Plasmid wurde isoliert und durch Sequenzierung die erwartete Nukleotidsequenz bestätigt. Die Präparation der Plasmid DNA wurde nach Methoden und mit Materialien der Firma Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet. Das erhaltene Plasmid wurde als pCIS lysC (SEQ ID NO:4) bezeichnet. Es umfasst folgende wesentliche Teilbereiche:

| Position | Art der Sequenz | Beschreibung |
|---|---|---|
| 155-1420 | CDS | lysC |
| Complement (3935..5356) | CDS | sacB\Bacillus subtilis |
| Complement (5357..5819) | Promotor | Promotor\sacB |
| Complement (3913..3934) | C Region | sacB\Downstreambereich |
| 1974..2765 | CDS | Kanamycin-Resistenz |
| Complement (3032..3892) | CDS | Replikatiosursprung\E.coli\Plasmid pMB |

3.1.2 Mutagenese des lysC Gens aus C. glutamicum

Die gerichtete Mutagenese des lysC Gens aus C. glutamicum wurde mit dem QuickChange Kit (Stratagene, USA) nach Angaben des Herstellers durchgeführt. Die Mutagenese wurde im Plasmid pCIS lysC (SEQ ID NO:4) durchgeführt. Für den Austausch von thr 311 nach 311 jle mit Hilfe der Quickchange Methode (Stratagene) wurden folgende Oligonukleotidprimer synthetisiert:

5'-CGGCACCACCGACATCATCTTCACCTGCCCTCGTTCCG -3' (SEQ ID NO:5)

5'-CGGAACGAGGGCAGGTGAAGATGATGTCGGTGGTGCCG -3' (SEQ ID NO:6)

Der Einsatz dieser Oligonukleotidprimer in der Quickchange Reaktion führt in dem lysC Gen (SEQ ID NO:7) zu einem Austausch des Nukleotids in Position 932 (von C nach T). Der resultierende Aminosäureaustausch Thr311 Ile im lysC Gen wurde nach Transformation in E.coli XL1-blue und Plasmidpräparation durch eine Sequenzierungsreaktion bestätigt. Das Plasmid erhielt die Bezeichnung pCIS lysC thr311 ile (SEQ ID NO:8). Es umfasst folgende wesentliche Teilbereiche:

| Position | Art der Sequenz | Beschreibung |
|---|---|---|
| 155-1420 | CDS | LysC (thr311ile) |
| Complement (3935..5356) | CDS | sacB\Bacillus subtilis |
| Complement (5357..5819) | Promotor | Promotor\sacB |
| Complement (3913..3934) | C Region | sacB\Downstreambereich |
| 1974..2765 | CDS | Kanamycin-Resistenz |
| Complement (3032..3892) | CDS | Replikatiosursprung\E.coli\Plasmid pMB |

3.1.3 Transformation von pCIS lysC thr311 ile in *C. glutamicum* (Stamm ATCC13032)

Das Plasmid pCIS lysC thr311 ile wurde in *C. glutamicum ATCC13032* mittels Elektroporation, wie bei Liebl et al., FEMS Microbiology Letters 53:299-303 (1989) beschrieben, transformiert. Modifikationen des Protokolls sind in der DE 10046870 beschrieben. Die chromosomale Anordnung des lysC-Lokus einzelner Transformanten wurde mit Standardmethoden durch Southernblot und Hybridisierung, wie in Sambrook et al., Molecular Cloning. A Laboratory

Manual, Cold Spring Harbor (1989), beschrieben, überprüft. Dadurch wurde sichergestellt, dass es sich bei den Transformanten um solche handelt, die das transformierte Plasmid durch homologe Rekombination am lysC-Lokus integriert haben. Nach Wachstum solcher Kolonien über Nacht in Medien, die kein Antibiotikum enthalten, werden die Zellen auf ein Saccharose-CM-Agarmedium (10% Saccharose) ausplattiert und 24 Stunden bei 30°C inkubiert.

Da das im Vektor pCIS lysC thr311i1e sacB Gen Saccharose in ein toxisches Produkt umwandelt, können nur solche Kolonien anwachsen, die das sacB Gen durch einen zweiten homologen Rekombinationsschritt zwischen dem Wildtypgen lysC und dem mutierten Gen lysC thr311 ile deletiert haben. Während der homologen Rekombination kann entweder das Wildtypgen oder das mutierte Gen zusammen mit dem sacB Gen deletiert werden. Wenn das sacB Gen zusammen mit dem Wildtypgen entfernt wird, resultiert eine mutierte Transformante.

Anwachsende Kolonien wurden gepickt und auf einen Kanamycin-sensitiven Phänotyp hin untersucht. Klone mit deletiertem SacB Gen müssen gleichzeitg Kanamycinsensitives Wachstumsverhalten zeigen. Solche Kanamycin-sensitiven Klone wurden in einem Schüttelkolben auf ihre Lysin-Produktivität hin untersucht. Zum Vergleich wurde der nicht behandelte *C. glutamicum* ATCC13032 angezogen. Klone mit einer gegenüber der Kontrolle erhöhten Lysin-Produktion wurden selektiert, chromosomale DNA gewonnen und der entsprechende Bereich des lysC Gens durch eine PCR-Reaktion (Pfu-Turbo PCR Systems; Stratagene, USA) nach Angaben des Herstellers amplifiziert und sequenziert (nach Sanger et al., a.a.O.). Ein solcher Klon mit der Eigenschaft erhöhter Lysin-Synthese und nachgewiesener Mutation in lysC an Position 932 wurde mit ATCC13032 lysC$^{fbr}$ bezeichnet.

3.2 Herstellung der Fermentationsmedien

3.2.1 Vorkultur 1:

Die Vorkultur 1 wurde in einem 5 L Fermenter durchgeführt. Das Arbeitsvolumen im Fermenter betrug 3 L. Die Zusammensetzung des Vorkulturmediums ist in nachfolgender Tabelle dargestellt.

| Medienkomponente | Konzentration |
|---|---|
| Saccharose | 4,75 % |
| Ammoniumsulfat | 1,00 % |
| $MgSO_4$ | 0,05 % |
| $KH_2PO_4$ | 0,20 % |
| Harnstoff | 0,25 % |
| Maisquellwasser | 5,00 % |
| Hydrolysiertes Sojaprotein | 4,00 % |
| Nikotinsäure | 4,95 mg/L |
| Thiamin*HCl | 1 mg/L |
| d-Biotin | 1,5 mg/L |
| β-Alanin | 10 mg/L |
| $FeSO_4$ | 10 mg/L |
| $MnSO_4$ | 10 mg/L |
| $CUSO_4$ | 1 mg/L |
| Antischaum | 0,1 g/L |

Der Zucker wurde direkt im Fermenter in Wasser gelöst und in-situ sterilisiert. Die Stickstoffquellen wurden separat von den Zuckern sterilisiert und anschließend zugegeben. Die Vitamin- und Spurensalzlösung wurde ebenfalls separat hergestellt und durch einen 0,2 μm Sterilfilter zum Fermenter nach der Sterilisation hinzugegeben. Nach Zugabe aller Medienkomponenten wird der pH-Wert durch NaOH auf pH 7 eingestellt.

3.2.2 Vorkultur 2:

Die Vorkultur 2 wurde in einem 50 L Fermenter durchgeführt. Das Arbeitsvolumen des Fermenters betrug 30

L. Die Zusammensetzung des zweiten Vorkulturmediums ist in nachfolgender Tabelle dargestellt.

| Medienkomponente | Konzentration |
|---|---|
| Low Quality Molasses | 3,50 % |
| Saccharose | 3,50 % |
| Maisquellwasser | 3,63 % |
| Ammoniumsulfat | 0,70 % |
| Harnstoff | 0,25 % |
| $H_3PO_4$ | 0,25 % |
| Nikotinsäure | 7 mg/L |
| Thiamin*HCl | 2,5 mg/L |
| d-Biotin | 0,05 mg/L |
| ß-Alanin | 5 mg/L |
| $MnSO_4$ | 7 mg/L |
| $CuSO_4$ | 1,5 mg/L |
| Antischaum | 0,25 g/L |
| Betaine 97% | 0,07 % |

Wie beim Vorkulturmedium 1 wurden die Zuckerquellen direkt im Fermenter in Wasser gelöst und in-situ sterilisiert. Die Stickstoffquellen wurden separat von den Zuckern sterilisiert und anschließend zugegeben. Die Vitamin- und Spurensalzlösung wurde ebenfalls separat hergestellt und durch einen 0,2 μm Sterilfilter zum Fermenter nach der Sterilisation hinzugegeben. Nach Zugabe aller Medienkomponenten wird der pH-Wert durch NaOH auf pH 7 eingestellt.

3.2.3 Hauptkultur:

Die Hauptkultur wurde als Zulaufverfahren geführt, so dass neben dem Startmedium auch ein Zulaufmedium eingesetzt wurde. Es wurde ein Fermenter mit 300 L Nennvolumen eingesetzt, wobei das maximale Arbeitsvolumen 190 L betrug.

Zu Beginn jeder Hauptfermentation wurden 110 L des in nachfolgender Tabelle dargestellten Startmediums in den Fermenter gegeben. Erneut wurden die Zuckerquelle mit Wasser im Fermenter vorgelegt und in-situ sterilisiert. Die Stickstoffquellen wurden separat von den Zuckern sterilisiert. Die Vitamin- und Spurensalzlösung wurde ebenfalls separat hergestellt und durch einen 0,2 μm Sterilfilter zum Fermenter nach der Sterilisation hinzugegeben. Nach Zugabe aller Medienkomponenten wird der pH-Wert durch NaOH auf pH 7 eingestellt.

| Medienkomponente | Konzentration |
|---|---|
| Low Quality Molasses | 3,00 % |
| Maisquellwasser | 1,49 % |
| Ammoniumsulfat | 5,00 % |
| Antischaum | 0,1 g/L |
| Betain 97% | 0,07% |
| $H_3PO_4$ | 0,063 % |
| Nikotinsäure | 2,5 mg/L |
| Thiamin*HCl | 2,5 mg/L |
| d-Biotin | 0,3 mg/L |
| $MnSO_4$ | 1 mg/L |

Die Zusammensetzung des Zulaufmediums ist in der folgenden Tabelle dargestellt. Die eingesetzte Glukose wurde nach dem in Beispiel 1 dargestellten Verfahren hergestellt. Der leer sterilisierte Tank für das Zulaufmedium wurde schrittweise durch einen Spiralwärmetauscher (140°C, 90 s Verweilzeit) mit den separat angesetzten Vitamin-, Salz- und Ammoniumsulfatlösungen gefüllt. Die Zuckerlösungen wurden in einem zweiten Schritt dann ebenfalls sterilisiert über den Wärmetauscher zugefahren.

| Medienkomponente | Konzentration |
|---|---|
| Low Quality Molasses | 3,10 % |
| Glukose* | 41,90 % |
| Ammoniumsulfat | 5,50 % |
| Antischaum | 1,0 g/L |
| Betain 97% | 0,07 % |
| $H_3PO_4$ | 0,05 % |
| Nikotinsäure | 0,00045% |
| Thiamin*HCl | 0,000038% |
| d-Biotin | 0,000125% |
| *aus Glukoselösung entsprechend Beispiel 4 | |

3.3 Fermentation

Die Herstellung des Inokkulums für die Vorkultur 1 erfolgte in 2 L Schüttelkolben mit 300 mL Arbeitsvolumen (Vorkulturmedium 1). Ausgehend von Schrägagarröhrchen wurden die Schüttelkolben beimpft und bei 29°C und 120 Umdrehungen pro Minute über 19 bis 24 h zu einem Volumenanteil Biomasse von 3 Vol% geschüttelt.

Der entsprechend Abschnitt 3.2.1 vorbereitete Fermenter für die Vorkultur 1 wurde mit einem Schüttelkolben beimpft und über 24 h bei 30°C, einem spezifischen Leistungseintrag von 5 kW/$m^3$ und 1 vvm Belüftung fermentiert. Abbruchkriterium für die Fermentation war ein Biomassegehalt von 3 Vol%.

Im Anschluss daran wurde der entsprechend Abschnitt 3.2.2 vorbereitete Fermenter für die Vorkultur 2 mit der Vorkultur 1 beimpft. Es wurde eine entsprechende Menge Vorkultur 1 zugegeben, um zum Start einen Volumenanteil Biomasse von 0,5 Vol% zu erreichen. Die Fermentation wurde bei 30°C, 0,7 vvm Belüftung und einem Leistungseintrag von 2 kW/$m^3$ betrieben. Die pH-Kontrolle erfolgte durch gasförmiges Ammoniak im Bereich 6,8 bis 7,0. Die übliche Fermentationsdauer bis zum Erreichen des Abbruchkriteriums eines Volumenanteils von 10 Vol% betrug 14 bis 18 h.

Im nächsten Schritt wurde der entsprechend Abschnitt 3.2.3 mit dem Startmedium vorbereitete Hauptfermenter beimpft, indem der gesamte Inhalt der Vorkultur 2 in den Hauptfermenter gepumpt wurde. Die Hauptfermentation wurde bei 33°C, 0,5 vvm Belüftung und einem spezifischen Leistungseintrag von 0,5 kW/$m^3$ durchgeführt. Die pH-Wert wurde während der Fermentation mittels gasförmigen Ammoniaks auf einem pH-Wert von 6,8 bis 7,0 geregelt. In jeweils 2-stündigen Intervallen wurde jeweils eine Teilmenge des vorbereiteten Zulaufmediums zugegeben, wobei sich die zugegebene Menge am aktuellen Zuckerverbrauch orientierte. Um eine Akkumulation oder Verarmung an Zucker zu vermeiden, wurde jeweils soviel Zucker zugegeben, wie im kommenden Intervall erwartungsgemäß verbraucht wird. Sobald das Volumen der Inhaltstoffe im Fermenter einen Wert von 210 L überschritten hatte, wurde eine Teilmenge aus dem Fermenter entnommen, um ein Überlaufen zu vermeiden. Nach 48 h wurde die Fermentation beendet und der Fermenter entleert. Die während der Fermentation entnommenen Teilmengen wurden mit dem Fermenterinhalt zum Fermentationsende zusammengefasst und gemeinsam aufgearbeitet.

Mit dem in Abschnitt 3.1 genannten Stamm von Corynebacterium glutamicum wurden nach dem beschriebenen Vorgehen in der Hauptfermentation insgesamt 21,6 kg Lysin erzeugt. Die Lysinkonzentration am Fermentationsende betrug 98 g/L. Der Biomassegehalt in den 293 kg erzeugter Fermentationsbrühe betrug 38 g/L.

3.4 Aufarbeitung der Fermentationsbrühe durch Abtrennung und Trocknung der Biomasse

Zur Abtrennung der Biomasse wurde die Biomasse-haltige Fermentationsbrühe mit 300 L/h über einen Dekanter CA 225 (Fa. Westfalia) gefahren. Insgesamt wurden nach diesem Vorgehen 48,3 kg einer biomassehaltigen Fraktion mit 23 Gew.-% Biotrockenmasse sowie 244,7 kg eines biomassefreien Überstandes erhalten. Eine Teilmenge der

biomassehaltigen Fraktion wurde entnommen und auf Blechen in einem Trockenschrank bei 90°C getrocknet. Die Restfeuchte der getrockneten Biomasse betrug 5,2 Gew.-%. Bezogen auf Trockenmasse bestand die Biomasse aus 62,0 Gew.-% Rohprotein, 0,3 Gew.-% Rohfaser, 5,6 Gew.-% Rohfett, 5,9 Gew.-% Zucker und 3,2 Gew.-% Rohasche.

Nicht - erfindungsgemäßes Beispiel 4: Herstellung und Untersuchung einer Futtermittelzusammensetzung für Ferkel unter Verwendung von Gluten aus Beispiel 2

Es wurden Proben der in Beispiel 1 erhaltenen Keimfraktion (Probenanzahl n=1), Proben des in Beispiel 2 erhaltenen getrockneten Glutens (Probenzahl n=2) und Proben der in Beispiel 3 anfallenden Biomasse (Probenzahl n=16) hinsichtlich ihrer Zusammensetzung und ihrer Feststoffcharakteristik untersucht. Die Analytik der Proben ergab die in folgender Tabelle dargestellte Zusammensetzung. Die mittlere Partikelgröße des Glutens in den betrachteten Proben lag bei 270 μm, der Biomasse zwischen 400 und 500μm und der vermahlenden Keimfraktion zwischen 872 und 1194 μm.

| Parameter | Keimling | Gluten | Biomasse |
|---|---|---|---|
| Restfeuchte [%] | 9,65 | 5,90 | 7,24 |
| Rohprotein [%]* | 18,82 | 32,74 | 67,30 |
| Gesamtzucker [%]* | 18,09 | 27,80 | 5,62 |
| Lysin [%]* | 0,00 | 0,03 | 9,09 |
| Rohfaser [%]* | 5,90 | 6,25 | 0,11 |
| Rohfett [%]* | 19,73 | 2,60 | 6,94 |
| Rohasche [%]* | 2,35 | 0,83 | 2,75 |
| Ammonium-N [%]* | 0,18 | 0,38 | 0,53 |
| Gesamt-N [%]* | 3,01 | 5,24 | 10,77 |
| Sülfat [%]* | 0,09 | 0,13 | 4,71 |
| NDF# [%]* | 26,77 | 34,13 | 11,77 |
| * bezogen auf Trockenmasse<br># non-digestible fiber | | | |

Zur Herstellung einer Futtermittelzusammensetzung wurden die einzelnen Komponenten im Verhältnis 21% Biomasse : 22% Keimlingfraktion : 57% Gluten gemischt, um so eine Futtermittelzusämmensetzung folgender Zusammensetzung zu erhalten. Es wurden insgesamt 16 Proben hergestellt. Im Mittel hatte die so erhaltene Futtermittelzusammensetzung eine Schüttdichte zwischen 550 und 700 g/L bei einer mittleren Partikelgröße von 590 μm.

| Parameter | Futtermittelzusammensetzung |
|---|---|
| Restfeuchte [%] | 9,32 |
| Rohprotein [%]* | 36,98 |
| Gesamtzucker [%]* | 27,05 |
| Lysin [%]* | 2,65 |
| Rohfaser [%]* | 4,72 |
| Rohfett [%]* | 10,13 |
| Rohasche [%]* | 2,67 |
| Ammonium-N [%]* | 0,30 |
| Gesamt-N [%]* | 5,90 |
| Sulfat [%]* | 1,26 |

(fortgesetzt)

| Parameter | Futtermittelzusammensetzung |
|---|---|
| NDF# [%]* | 27,66 |
| * bezogen auf Trockenmasse<br># non-digestible fiber | |

Die so erhaltene Futtermittelzusammensetzung wies einen hohen Proteingehalt, speziell einen hohen Lysingehalt auf und hatte aufgrund des hohen Fett- und Zuckergehaltes einen hohen Energiegehalt.

In Fütterungsexperimenten mit Ferkeln wurden die so hergestellten Futtermittelzusammensetzungen auf ihre Eignung als Futtermittel bzw. Futtermitteladditiv geprüft. Ausgehend von einer Mais-Soja-Diät wurden 5 % der erhaltenen Futtermittelzusammensetzung zugegeben. Die zugegebene Menge wurde entsprechend der Zusammensetzung der Futtermittelmischung durch Reduktion von Sojamehl (73 %), Mais (20 %) und Sojaöl (7 %) kompensiert. Durch weitere Anpassungen in freien Aminosäuren und Mineralien wurden damit Diäten mit gleichem Energie- und Nährstoffgehalt zusammengestellt. Zum Abschluss wurden die Diäten pelletiert. Die die Futtermittelmischung enthaltende Diät wurde im Vergleich zur Mais-Soja-Diät jeweils an 12 Boxen mit 4 - 6 Wochen alten Ferkeln verfüttert. Dabei zeigten die Ferkel eine durchschnittliche Gewichtszunahme von 261 g/Tag, eine Futteraufnahmerate von 471 g/Tag und eine Futtermittel-Verwertungsrate von 1,87 kg Futtermittel pro kg Gewichtszunahme. Vergleichbare Ergebnisse erhielt man bei Fütterung mit einer konventionellen Mais-Soja-Diät, die zur Erzeugung des gewünschten Nährstoffgehalts mit Aminosäuren angereichert waren.

Die Beispiele zeigen, dass die Futtermittelzusammensetzungen anstelle oder zusammen mit konventionellen Diäten eingesetzt werden können, ohne dass hierdurch die Futterqualität leidet. Vielmehr ist kann auf den Zusatz von Aminosäuren verzichtet werden. Die Futtermittel eignen sich daher, anders als die bei der Bioethanolherstellung anfallenden Feststoffe, als hochwertiger Ersatz für Mais und Soja in Diäten für Monogastrier.

Nicht erfindungsgemäßes Beispiel 5: Herstellung von Futtermittelzusammensetzungen für Hühner-Küken unter Verwendung von Gluten erhältlich analog Beispiel 2

Proben der entsprechend der vorherigen Beispiele 1 bis 3 hergestellten Fraktionen Biomasse, Gluten und Keimling wurden hinsichtlich ihrer Zusammensetzung analysiert. Als Referenz diente Sojamehl.

Analog Beispiel 4 wurde eine Futtermittelzusammensetzung durch Vermischen der Biomasse, Gluten und Keimling im Verhältnis 26:47:27 hergestellt und in einigen Hauptbestandteilen analysiert. Die Zusammensetzung der weiteren Bestandteile wurde rechnerisch aus der Zusammensetzung der Einzelkomponenten dieser Mischung berechnet. Nach diesem Vorgehen ergab sich folgende Zusammensetzung der verschiedenen Proben:

| **Parameter** | **Keimling** | **Gluten** | **Bio-masse** | **FZ[5)]** | **Soja-mehl** |
|---|---|---|---|---|---|
| Trockensubstanzgehalt [g/kg] | 912 | 966 | 931 | 937 | 911 |
| Rohasche [g/kg] | 56 | 7 | 28 | _3 | 65 |
| Rohprotein [g/kg] | 160 | 292 | 642 | 349 | 452 |
| Sonstige Extrakte [g/kg] | 226 | 66 | 85 | 111 | 25 |
| Stärke [g/kg] | 200 | 19 | <6 | 63[4] | 53 |
| Zucker [g/kg] | 98 | 384 | 11 | 209[4] | 89 |
| Rohfaser [g/kg] | 53 | 37 | 4 | 32[4] | 69 |
| Energiegehalt ME [MJ/kg][2] | 14,8 | 12,1 | 13,0 | 13,0[4] | 9,9 |
| Ca [g/kg] | < 0,5 | < 0,5 | < 0,5 | < 0,5[4] | _3 |
| P [g/kg] | 2 | 2 | 4,4 | 2,6[4] | _3 |
| Na [g/kg] | 0,3 | 0,3 | 3,2 | 1,1[4] | _3 |
| K [g/kg] | 1,2 | 1,2 | 6,1 | 2,5[4] | _3 |

(fortgesetzt)

| Parameter | Keimling | Gluten | Bio-masse | FZ5) | Soja-mehl |
|---|---|---|---|---|---|
| Cl[g/kg] | < 0,6 | < 0,6 | 1,9 | 0,9[4] | _3 |
| [1] lt. Aminosäureanalytik<br>[2] Abschätzung durch Regressionsformel entsprechend Analyseergebnisse<br>3 nicht analysiert<br>[4] berechnet aus den Einzelkomponenten der Futtermittelformulierung<br>[5] Futtermittelzusammensetzung | | | | | |

Zur Herstellung eines Futtermittels wurde eine Basal-Diät mit der folgenden Zusammensetzung hergestellt: Die Zusammensetzung der Basal-Diät ist in nachfolgender Tabelle dargestellt:

| Komponente | [g/kg] |
|---|---|
| Mais | 677,1 |
| High Protein Sojamehl | 211,0 |
| L-Lysine HCl | 7,8 |
| D,L-Methionin | 5,4 |
| L-Threonin | 3,5 |
| L-Tryptophan | 0,9 |
| L-Arginin | 3,8 |
| L-Isoleucin | 3,1 |
| L-Leucin | 0,9 |
| L-Valin | 2,9 |
| L-Phenylalanin | 0,9 |
| L-Cystin | 1,7 |
| Sojaöl | 27,5 |
| Monokalziumphosphat | 220,6 |
| Calciumcarbonat | 19,1 |
| Natriumchlorid | 5,4 |
| Vitamin-Prämix | 5,5 |
| Cholinchlorid (50 %) | 1,4 |
| Spurenelemente-Prämix | 1,4 |

In den im Folgenden beschriebenen Fütterungsversuchen wurde die Basal-Diät als Referenz sowie 3 weitere Futtermittel eingesetzt, in denen 35 Gew.-% der Diät durch Gluten aus Beispiel 2, durch die Futtermittelzusammensetzung, bzw. durch Sojamehl (Vergleich) ersetzt wurden.

| Nr. | Zusammensetzung | |
|---|---|---|
| V1 | Basal-Diät | 100% |
| 2 | Basal-Diät + Gluten | 65%+35% |
| 3 | Basal-Diät + Futtermittelzusammensetzung | 65%+35% |

(fortgesetzt)

| Nr. | Zusammensetzung | |
|---|---|---|
| V4 | Basal-Diät + Sojamehl | 65%+35% |
| V: Vergleich | | |

Zur Sicherstellung von Homogenität wurde eine gemeinsame Grundmischung dieser Basal-Diät erzeugt. Zu dieser Grundmischung wurde dann jeweils die entsprechenden Proben zugemischt. Anschließend wurden die Mischungen durch eine 3 mm Kokille zu Pellets verpresst.

Zur Vorbereitung der Fütterungsversuche wurden männliche, ein Tag alte Hühnerküken (Ross 308) in Bodenställen mit einer handelsüblichen Starter-Diät herangezogen. Von diesen Küken wurden an Tag 8 Tiere für die Fütterungsversuche entnommen und umgesetzt.

Für Fütterungsversuche wurden pro Probe jeweils 6 Parallelexperimente mit jeweils 8 Küken in Käfigen durchgeführt. Bis zu Tag 13 wurden diese Küken mit der kommerziellen Starter-Diät gefüttert. Am Tag 13 wurden die Küken gewogen und über 9 Tage mit der experimentellen Diät gefüttert, bevor erneut das Gewicht bestimmt wurde. Bei diesem Vorgehen wurden folgende tägliche Gewichtszugaben, Futteraufnahme und Futtermittelverwertungsraten (Masse Gewichtszunahme / Masse Futteraufnahme) festgestellt:

| **Futtermittel Nr.** | **V1** | **2** | **3** | **V4** |
|---|---|---|---|---|
| Gewichtszunahme [g/Tag] | 57,5 | 52,0 | 39,9 | 61,5 |
| Futteraufnahme [g/Tag] | 85,5 | 85,1 | 76,5 | 87,8 |
| Futtermittelverwertungsrate[1)] | 1,49 | 1,64 | 1,92 | 1,43 |
| [1)] g Gewichtszunahme / g Futteraufnahme | | | | |

Die Diäten mit dem Gluten bzw. den Futtermittelzusammensetzungen führten zu einer verbesserten Futtermittelverwertung

**Patentansprüche**

**1.** Verfahren zur Herstellung einer wässrigen Glukoselösung aus Mais, umfassend:

a) fraktionierendes, trockenes Vermahlen von Maiskörnern, wobei man die Maiskörner in eine Maisstärke enthaltende Endosperm-Fraktion und eine ölreiche Keimling-Fraktion und gegebenenfalls eine Kleie-Fraktion auftrennt;
b) Enzymatisches Verflüssigen und Verzuckern der Maisstärke in einer wässrigen Suspension der Endosperm-Fraktion, wobei man eine Maisgluten enthaltende, wässrige Glukoselösung erhält; und
c) Abreichern des Maisglutens und gegebenenfalls vorhandener Kleie aus der wässrigen Glukoselösung,

wobei man in Schritt b) eine wässrige Suspension des in Schritt a) erhaltenen Maismehls einsetzt, welches die Endosperm-Fraktion und gegebenenfalls die Kleiefraktion enthält, wobei die Menge des Maismehls so gewählt ist, dass die wässrige Suspension 30 bis 45 Gew.-%, bezogen auf das Gesamtgewicht der Suspension, Stärke enthält.

**2.** Verfahren nach Anspruch 1, wobei man das Vermahlen in Schritt a) in Gegenwart von 1 bis 30 Gew.-% Wasser, bezogen auf die Masse eingesetzter Maiskörner, durchführt.

**3.** Verfahren nach Anspruch 1 oder 2, wobei man in Schritt a) im Wesentlichen nur die Keimling-Fraktion und die Kleie-Fraktion von der Endosperm-Fraktion abtrennt.

**4.** Verfahren nach Anspruch 1 oder 2, wobei man in Schritt a) die Kleie-Fraktion und die Keimling-Fraktion von der Endosperm-Fraktion abtrennt und einen Teil der Kleiefraktion in die Endosperm-Fraktion zurückführt.

**5.** Verfahren nach einem der vorhergehenden Ansprüche, wobei man das Maisgluten aus der wässrigen Glukoselösung zu wenigstens 90 %, bezogen auf die gesamten, in der Glukoselösung enthaltenen Glutenbestandteile abtrennt.

**6.** Verfahren nach einem der vorhergehenden Ansprüche, wobei man die Abreicherung des Maisglutens und gegebenenfalls vorhandener Kleiebestandteile so vornimmt, dass die nach der Abreicherung erhaltene Glukoselösung weniger als 10 Vol.-% Feststoffe enthält.

## Claims

**1.** A process for the production of an aqueous glucose solution from maize, comprising the following steps:

a) fractionating dry milling of maize kernels, where the maize kernels are separated into a maize-starch-comprising endosperm fraction and a high-oil germ fraction and optionally a bran fraction;
b) enzymatic liquefaction and saccharification of the maize starch in an aqueous suspension of the endosperm fraction, which gives an aqueous glucose solution comprising maize gluten; and
c) depletion of the maize gluten and optionally any bran present from the aqueous glucose solution,

where in step b) an aqueous suspension is used of the maize flour obtained in step a) and comprising the endosperm fraction and optionally the bran fraction, the quantity of maize flour being selected such that the aqueous suspension comprises 30 to 45% by weight of starch, based on the total weight of the suspension.

**2.** The process according to claim 1, wherein the milling in step a) is carried out in the presence of from 1 to 30% by weight of water, based on the weight of the maize kernels employed.

**3.** The process according to claim 1 or 2, wherein, in step a), essentially only the germ fraction and the bran fraction are separated from the endosperm fraction.

**4.** The process according to claim 1 or 2, wherein, in step a), the bran fraction and the germ fraction are separated from the endosperm fraction and some of the bran fraction is returned to the endosperm fraction.

**5.** The process according to any of the preceding claims, wherein at least 90% of the maize gluten, based on the total gluten constituents present in the glucose solution, are separated from the aqueous glucose solution.

**6.** The process according to any of the preceding claims, wherein the depletion of the maize gluten and optionally bran constituents present is carried out in such a way that the glucose solution comprises less than 10% by volume of solids after the depletion.

## Revendications

**1.** Procédé pour la préparation d’une solution aqueuse de glucose à partir de maïs, comprenant :

a) broyage à sec avec fractionnement de grains de maïs, dans lequel on fractionne les grains de maïs en une fraction-endosperme contenant de l’amidon de maïs et une fraction-germe riche en huile et éventuellement une fraction-son ;
b) liquéfaction et saccharification enzymatiques de l’amidon de maïs dans une suspension de la fraction-endosperme, de sorte qu’on obtient une solution aqueuse de glucose contenant du gluten de maïs ; et
c) réduction de la teneur en gluten de maïs et en son éventuellement présent de la solution aqueuse de glucose,

dans lequel on utilise dans l’étape b) une suspension aqueuse de la farine de maïs obtenue dans l’étape a), qui contient la fraction-endosperme et éventuellement la fraction-son, la quantité de la farine de maïs étant choisie de manière que la suspension aqueuse contienne de 30 à 45 % en poids d’amidon, par rapport au poids total de la suspension.

**2.** Procédé selon la revendication 1, dans lequel on effectue le broyage dans l’étape a) en présence de 1 à 30 % en poids d’eau, par rapport à la masse des grains de maïs utilisés.

**3.** Procédé selon la revendication 1 ou 2, dans lequel on ne sépare dans l’étape a) pratiquement que la fraction-germe et la fraction-son de la fraction-endosperme.

**4.** Procédé selon la revendication 1 ou 2, dans lequel on sépare dans l'étape a) la fraction-son et la fraction-germe de la fraction-endosperme et on recycle dans la fraction-endosperme une partie de la fraction-son.

**5.** Procédé selon l'une quelconque des revendications précédentes, dans lequel on sépare à raison d'environ 90 % le gluten de maïs de la solution aqueuse de glucose, par rapport à la totalité des composants de gluten contenus dans la solution de glucose.

**6.** Procédé selon l'une quelconque des revendications précédentes, dans lequel on effectue la réduction de la teneur en gluten de maïs et en composants de son éventuellement présents de manière que la solution de glucose obtenue après la réduction de la teneur contienne moins de 10 % en volume de matières solides.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005116228 A
- WO 2007028804 A
- US 20050233030 A
- US 20050239181 A
- US 4287304 A
- CN 1173541
- WO 2006004748 A, GRAINVALUE, LLC
- US 4448881 A, MULLER, W.C. & MILLER, F.D.
- WO 2004113551 A
- US 3044941 A
- EP 249773 A
- US 5504004 A
- US 5723322 A
- US 5573931 A
- US 5521075 A
- WO 9906532 A
- US 5869301 A
- US 5770435 A
- US 3063910 A
- DE 3924423
- US 440379 A
- WO 9635799 A
- US 5164309 A
- JP 05099974 B
- JP 06311891 B
- FR 2671099
- EP 0555540 A
- JP 3053791 B
- WO 01011052 A
- DE 19840709
- WO 9829539 A
- EP 1186664 A
- EP 0765939 A
- WO 03056028 A
- EP 01201762 A
- WO 0112832 A
- WO 0077234 A
- WO 9320183 A
- WO 9803480 A
- US 5599711 A
- WO 9102060 A
- US 3708395 A
- WO 03087386 A
- WO 03100072 A
- WO 01021772 A
- WO 9855599 A
- GB 01188885 A
- JP 2004222569 B
- EP 1038527 A
- EP 0648076 A
- EP 835613 A
- EP 0219276 A
- EP 0394022 A
- EP 0547422 A
- EP 1088486 A
- EP 0758018 A
- WO 9212645 A
- EP 0230250 A
- FR 2464260
- DE 3601281
- US 3258481 A
- EP 233816 A
- US 2002177198 A
- WO 9915673 A
- EP 867446 A
- WO 02100537 A
- WO 02100539 A
- WO 2004052813 A
- WO 9804540 A
- WO 9200947 A
- US 4297290 A
- US 5480979 A
- US 5698684 A
- US 4523960 A
- DE 10046870


### In der Beschreibung aufgeführte Nicht-Patentliteratur


- The Alcohol Textbook - A reference for the beverage, fuel and industrial alcohol industries. Nottingham Univ. Press, 1995, 7-23
- Determining the cost of producing ethanol from corn starch and lignocellulosic feedstocks. **MCALOON et al.** NREL/TP-580-28893. National Renewable Energy Laboratory, Oktober 2000
- **N. JAKEL.** *Biofuels Journal, http://www.renessen.com/news_release/Renessen_ethanol_art.pdf*
- The Alcohol Textbook - A reference for the beverage, fuel and industrial alcohol industries. 7-23
- **REHM, H.-J.** Biotechnology. VCH, 1980

- **GUTCHO.** Chemicals by Fermentation. Noyes Data Corporation, 1973
- Genetics and Physiology of Aspergillus. Academic Press, 1977
- Economic Microbiology. Academic Press, 1978, vol. 2, 47-119
- *Int. J. Syst. Bacteriol.,* 1976, vol. 26, 498-504
- **GUETTLER ; JAIN ; SONI.** *Arch. Microbiol.,* 1997, vol. 167, 332-342
- **GUETTLER MV ; RUMLER D ; JAIN MK.** Actinobacillus succinogenes sp. nov., a novel succinic-acid-producing strain from the bovine rumen. *Int J Syst Bacteriol.,* Januar 1999, vol. 49, 207-16
- *Crit. Rev. Biotechnol.,* 1986, vol. 3, 331-373
- *Food Biotechnol.,* 1993, vol. 7, 221-234
- *FOOD BIOTECHNOL.,* 1996, vol. 10, 13-27
- **IKEDA, M.** Amino Acid Production Process. *Adv. Biochem. Engin/Biotechnol,* 2003, vol. 79, 1-35
- *Trends Biotechnol.,* 1985, vol. 3, 64-68
- *J. Ferment. Bioeng.,* 1990, vol. 70, 253-260
- **GILL, I. ; RAO, V.** Polyunsaturated fatty acids, part 1: occurrence, biological activities and applications. *Trends in Biotechnology,* 1997, vol. 15 (10), 401-409
- **ZHU, H.** *Utilization of Rice Bran by Pythium irregulare for Lipid Production,* 31. Oktober 2002
- **ZHU, H.** Utilization of Rice Bran by Pythium irregulare for Lipid Production. *Master Thesis Louisiana State University,* 31. Oktober 2002
- **AFSCHAR et al.** *Mikrobielle Produktion von 2,3-Butandiol. CIT,* 2004, vol. 64 (6), 570-571
- **MIYAZAKI, J.-I. ; MIYAGAWA, K.-I. ; SUGIYAMA, Y.** Trehalose Accumulation by Basidiomycotinous Yeast, Filobasidium floriforme. *Journal of Fermentation and Bioengineering,* 1996, vol. 81 (4), 315-319
- *Chem. Ber.,* 1994, 923-927
- **FUJIOKA, K.** New biotechnology for riboflavin (vitamin B2) and character of this riboflavin. *Fragrance Journal,* 2003, vol. 31 (3), 44-48
- **JIN et al.** *Biotech.Bioeng.,* 2003, vol. 81, 115-124
- **NELIS et al.** *J Appl Bacteriol,* 1991, vol. 70, 181-191
- **MIURA et al.** *Appl Environ Microbiol,* 1998, vol. 64, 1226-1229
- **KIM S. ; SEO W. ; PARK Y.** Enhanced production of beta-carotene from Blakeslea trispora with Span 20. *Biotechnology Letters,* 1997, vol. 19 (6), 561-562
- **MANTOURIDOU F. ; ROUKAS T.** Effect of the aeration rate and agitation speed on beta-carotene production and morphology of Blakeslea trispora in a stirred tank reactor: mathematical modelling. *Biochemical Engineering Journal,* 2002, vol. 10, 123-135
- **MIURA et al.** *Appl Environ Microbiol,* 1998, vol. 64, 1226-1229
- **S. Y. LEE.** Plastic Bacteria, Progress and Prospects for polyhydroxyalkanoate production in bacteria. *Tibtech,* 1996, vol. 14, 431-438
- Biopolymers. Wiley-VCH, 2003
- Biology of the Procaryotes. Thieme, 1999, 307
- **PRIEFERT, H. ; RABENHORST, J. ; SEINBÜCHEL, A.** Biotechnological production of vanillin. *Appl. Microbiol. Biotechnol.,* 2001, vol. 56, 296-314
- **JIAN-ZHONG JONG et al.** Fed-batch culture of Bacillus thuringiensis for thuringensin production in a tower type bioreactor. *Biotechnology and Bioengineering,* 2004, vol. 48 (3), 207-213
- **KIRST.** Fermentation-derived compounds as a source for new products. *Pure & Appl. Chem.,* 1998, vol. 70 (2), 335-338
- **ZIRKLE et al.** Heterologous production of the antifungal polyketide antibiotic soraphen A of Sorangium cellulosum So ce26 in Streptomyces lividans. *Microbiology,* 2004, vol. 150 (8), 2761-74
- **HOLLMANN et al.** Extraktiv-Fermentation von Gibberellinsäure mit Gibberella fujikuroi. *CIT,* 1995, vol. 7, 892-895
- **BERRY, A. ; DODGE, T.C. ; PEPSIN, M. ; WEYLER, W.** Application of metabolic engineering to improve both the production and use of biotech indigo. *Journal of Industrial Microbiology & Biotechnology,* 2002, vol. 28, 127-133
- **S. Y. LEE.** Plastic Bacteria Progress and prospects for polyhydroxyalkanoate production in bacteria. *Tibtech,* 1996, vol. 14, 431-438
- **RHODES et al.** Production of Fumaric Acid in 20-L Fermentors. *Applied Microbiology,* 1962, vol. 10 (1), 9-15
- **NARAYANAN et al.** *Electronic J. Biotechnol.,* 2004, vol. 7, http://www.ejbiotechnology.info/content/vol7/issue2/full/7/pdf
- **KAUTOLA, H.** *Appl. Microb. Biotechnol.,* 1990, vol. 33, 7
- **WILLKE et al.** *Appl. Microbiol. Biotechnol.,* 2001, vol. 56, 289
- **SCHILLING et al.** Repression of oxalic acid biosynthesis in the unsterile scleroglucan productino process with Sclerotium rolfsii ATCC 15205. *Bioprocess Engineering,* 2000, vol. 22, 51-55
- **RAU et al.** Oxygen controlled batch cultivations of Schizophyllum commune for enhanced production of branched ß-1,3-glucans. *Bioprocess Engineering,* 1994, vol. 11, 161-165
- **SATO et al.** Accumulation of Guanosine Polyphosphates by Brevibacterium ammoniagenes: Isolation and Identification of the Products. *Agr. Biol. Chem.,* 1976, vol. 40 (3), 465-474

- **MORI et al.** A novel process of inosine 5'-monophosphate production using overexpressed guanosine/inosine kinase. *Appl. Microbiol. Biotechnol.,* 1997, vol. 48, 693-698
- L-Glutamate Production. **E. KIMURA.** Handbook of Corynebacterium glutamicum. CRC press, 439-464
- **ELFARI, M. et al.** *Appl. Microbiol. Biotechnol.,* 2005, vol. 66, 668
- **HERRMANN U. et al.** *Appl. Microbial. Bioetchnol.,* 2004, vol. 64, 86
- **ROPER, H.** *Starch-Starke,* 1990, vol. 42, 342
- **ZELIC, B. et al.** *Chem. Biochem. Eng. Q.,* 2002, vol. 16, 7
- **BELTER, P. A.** Bioseparations: Downstream Processing for Biotechnology. John Wiley & Sons, 1988
- Ullmann's Encyclopedia of Industrial Chemistry. Wiley-VCH
- **COTTIER et al.** *Trends Heterocycl. Chem.,* 1991, vol. 2, 233
- **LEWKOWSKI, J.** *Arkivoc,* 2001, vol. 2, 17
- **KUSTER, B.F.M. et al.** *Carbohydr. Res.,* 1977, vol. 54, 159
- **HORVAT et al.** *Tetrahedron Lett.,* 1985, vol. 26, 2111
- **LICHTENTHALER, F.W.** *Acc. Chem. Res.,* 2002, vol. 35, 728
- **BESSON, M. et al.** *J. Catal.,* 1995, vol. 152, 116
- **CORMA, A. et al.** Chemical Routes for the Transformation of Biomass into Chemicals. *Chem. Rev.,* 2007, vol. 107, 2411-2502
- **BESSON, M. et al.** *Recl. Trav. Chim. Pys-Bas,* 1996, vol. 115, 217
- **DIRKX, J.M.H. et al.** *J. Catal.,* 1981, vol. 67, 1
- **DECHAMP, N. et al.** *Catal. Today,* 1995, vol. 24, 29
- **MARANHAO, L.C. A. et al.** *Ind. Eng. Chem. Res.,* 2005, vol. 44, 9624
- **MARSHALL et al.** Enzymatic Conversion of d-Glucose to d-Fructose. *Science,* 1957, vol. 125 (3249), 648
- **TAUCH et al.** *Plasmid,* 1995, vol. 33, 168-179
- **EIKMANNS et al.** *Microbiology,* 1994, vol. 140, 1817-1828
- **LENNOX.** *Virology,* 1955, vol. 1, 190
- **SANGER et al.** *Proceedings of the National Academy of Sciences USA,* 1977, vol. 74, 5463-5467
- **LIEBL et al.** *FEMS Microbiology Letters,* 1989, vol. 53, 299-303
- **SAMBROOK et al.** Molecular Cloning. A Laboratory Manual. Cold Spring Harbor, 1989